(19) Europäisches Patentamt
European Patent Office
Office européen des brevets

(11) EP 4 172 142 B1

(12) EUROPEAN PATENT SPECIFICATION

(45) Date of publication and mention of the grant of the patent:
**04.06.2025 Bulletin 2025/23**

(21) Application number: **21830025.9**

(22) Date of filing: **25.06.2021**

(51) International Patent Classification (IPC):
***C08G 65/334*** *(2006.01)* ***C07C 323/54*** *(2006.01)*
***A61P 11/00*** *(2006.01)* ***A61P 31/16*** *(2006.01)*
***A61P 37/04*** *(2006.01)*

(52) Cooperative Patent Classification (CPC):
**A61P 11/00; A61K 47/60; A61P 31/16; A61P 37/04; C08G 65/33324; C08G 65/334**

(86) International application number:
**PCT/AU2021/050667**

(87) International publication number:
**WO 2021/258154 (30.12.2021 Gazette 2021/52)**

(54) **BRANCHED LIPID COMPOUNDS**
VERZWEIGTE LIPIDVERBINDUNGEN
COMPOSÉS LIPIDIQUES RAMIFIÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.06.2020 PCT/AU2020/050660**
**24.12.2020 AU 2020904847**

(43) Date of publication of application:
**03.05.2023 Bulletin 2023/18**

(73) Proprietor: **Ena Respiratory Pty Ltd**
**Sydney, NSW 2000 (AU)**

(72) Inventor: **MCLACHLAN, Grant**
**Melbourne, Victoria 3000 (AU)**

(74) Representative: **Weickmann & Weickmann PartmbB**
**Postfach 860 820**
**81635 München (DE)**

(56) References cited:
**WO-A1-2011/119759** **WO-A1-2011/119759**
**WO-A1-2019/119067** **WO-A1-2019/119067**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

### Field of the invention

The present invention relates to compounds and their compositions, and to such compounds and compositions for use in the prevention and/or treatment of diseases or conditions ameliorated by modulation of TLR2 function, including respiratory infections, or respiratory diseases or conditions associated with viral or bacterial infections.

### Background of the invention

Respiratory infections are among the most common causes of human disease worldwide and are commonly caused by viruses. According to the World Health Organisation (WHO), worldwide, seasonal epidemics of influenza alone are estimated to result in about 3 to 5 million cases of severe illness, and about 250,000 to 500,000 deaths per year.

Although vaccines are available for some seasonal strains, for example influenza, these have not always been shown to be adequate due to several factors, such as infection between the lag phase between inoculation and the formation of antibodies and immune cells being formed. Seasonal vaccinations often also need modification, including re-formulation and administration, and may also not provide protection for the full length of time desired. For other occurrences of influenza, such as unexpected pandemic outbreaks, a vaccine is not always known, developed or available.

Viral respiratory infections can also worsen the severity of diseases of respiratory conditions leading to exacerbations (attacks). Exacerbations can occur for conditions such as asthma and chronic obstructive pulmonary disease (COPD). Asthma and COPD exacerbations are the most clinically and economically important forms of the diseases.

The vast majority of exacerbations, particularly in asthma, continue to occur despite use of the best available current therapies. When exacerbations do occur, treatment options are limited and have developed little in recent years. Treatment involves increasing doses of inhaled bronchodilators and systemic or oral corticosteroids - which are the same drugs that failed to prevent the exacerbation occurring in the first place.

There is a need, therefore, for new or improved compounds and methods for the treatment and/or prevention for respiratory infections, or respiratory conditions associated with viral or bacterial infections.

Reference to any prior art in the specification is not an acknowledgment or suggestion that this prior art forms part of the common general knowledge in any jurisdiction or that this prior art could reasonably be expected to be understood, regarded as relevant, and/or combined with other pieces of prior art by a skilled person in the art.

### Summary of the invention

The present invention provides Toll-Like Receptor 2 protein (TLR2) agonist compounds defined in claims 1-13 and their compositions defined in claim 14. TLR2 agonists have previously been identified to show potential in treating respiratory diseases and conditions associated with infectious agents such as viruses and bacteria. Advantageously, the compounds and compositions of the present application may show activity and have use in therapeutic areas such as treating and/or preventing respiratory diseases or conditions associated with viral or bacterial infections. In addition, the compounds and compositions of the present application may demonstrate increased stability which may translate to longer clearance rates following administration. Compounds of the invention demonstrate improved solution stability compared to other related compounds.

In one aspect, the present invention provides a compound of formula (I):

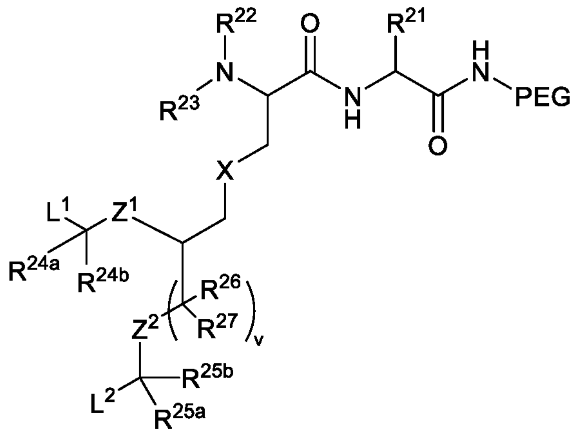

(I)

wherein:

$R^{21}$ is selected from the group consisting of H, $-CH_2OH$, $-CH_2CH_2OH$, $-CH(CH_3)OH$, $-CH_2OPO(OH)_2$, $-CH_2C(=O)NH_2$, $-CH_2CH_2C(=O)OH$ and $-CH_2CH_2C(=O)OR_8$, wherein any one of the alkyl hydrogens can be replaced with a halogen;

$R^{22}$ is H, $C_1$-$C_6$ aliphatic, an amino protecting group, $L^3$-C(=O)-, or $A^2$;

$L^1$ and $L^2$ are each independently $C_6$-$C_{21}$ aliphatic or $C_5$-$C_{20}$ heteroaliphatic;

$L^3$ is $C_1$-$C_{21}$ aliphatic or $C_2$-$C_{20}$ heteroaliphatic;

$A^2$ is an amino acid or a peptide;

$R^{23}$ is H or $C_1$-$C_6$ aliphatic;

$R^{24a}$ and $R^{25a}$ are each independently selected from $C_1$-$C_6$ aliphatic and $C_1$-$C_6$ heteroaliphatic and

$R^{24b}$ and $R^{25b}$ are each independently selected from H, $C_1$-$C_6$ aliphatic and $C_1$-$C_6$ heteroaliphatic; or

$R^{24a}$ and $R^{24b}$ together with the carbon atom to which they are attached form a $C_{3-8}$cycloalkyl or 3-8 membered heterocyclyl group, and/or

$R^{25a}$ and $R^{25b}$ together with the carbon atom to which they are attached form a $C_{3-8}$cycloalkyl or 3-8 membered heterocyclyl group;

X is selected from -S-, -S(=O)- and $-S(=O)_2-$;

v is an integer from 1-3

$R^{26}$ and $R^{27}$ are each independently selected from H and $C_1$-$C_6$ aliphatic;

$Z^1$ and $Z^2$ are independently selected from the group consisting of -C(=O)O-, -OC(=O)-, -C(=O)NR-, -NRC(=O)-, -C(=O)S-, -SC(=O)-, -OC(=O)O-, -NRC(=O)O-, -OC(=O)NR-, and -NRC(=O)NR-; wherein each R is independently H or $C_1$-$C_6$ aliphatic;

PEG is a polyethylene glycol;
wherein any aliphatic, heteroaliphatic, cycloalkyl and heterocyclyl present in any of $R^{21}$, $R^{22}$, $R^{23}$, $R^{24a}$, $R^{24b}$, $R^{25a}$, $R^{25b}$, $R^{26}$, $R^{27}$, $L^1$, $L^2$ and $L^3$ is optionally substituted.

In some embodiments, the compound of formula (I) is provided in the form of a pharmaceutically acceptable salt, solvate or prodrug thereof.

In some embodiments, the compound of formula (I) is selected from any one of compounds B1-B16.

The present invention also provides for compositions comprising, consisting essentially of, or consisting of, a compound of the invention or a pharmaceutically acceptable salt, solvate or prodrug thereof, and a pharmaceutically acceptable carrier, diluent or excipient.

The present invention also provides a compound of the invention or a pharmaceutically acceptable salt, solvate or prodrug thereof for the use specified in claim 15. Methods of medical treatment are described herein for illustrating the invention, but not to be understood as being encompassed by the invention.

In one aspect, the present invention provides a compound disclosed herein for use in raising an innate immune response in a subject.

In another aspect, the present invention provides a compound for use in treating and/or preventing a disease caused b, an infectious agent.

In another aspect, the present invention provides a compound for use in treating and/or preventing a respiratory disease or condition associated with a viral or bacterial infection.

In another aspect, the present invention provides a compound for use in treating and/or preventing a respiratory infection.

In another aspect, the present invention provides a compound for use in reducing airway inflammation.

The present invention also provides a compound for use in improving the ability of a subject to control a respiratory disease or condition during a respiratory viral infection.

The present invention also provides a compound for use in treating and/or preventing a disease or condition associated with the TLR2.

Further aspects of the present invention and further embodiments of the aspects described in the preceding paragraphs will become apparent from the following description, given by way of example and with reference to the accompanying drawings.

## Brief description of the drawings

**Figure 1.** Human TLR2 dose response (optical density at 650nm vs concentration (ng/mL) of compound B6 from the NK-κB luciferase assay described in Example 9.

**Figure 2.** Human TLR2 dose response (optical density at 650nm vs concentration (ng/mL) of compound B12 from the NK-κB luciferase assay described in Example 9.

**Figure 3.** Human TLR2 dose response (optical density at 650nm vs concentration (ng/mL) of compound B4. B6, B8, B12, B14 and B16 and heat killed *Listeria monocytogenes* (HKLC) from the assay described in Example 10.

**Figure 4.** TLR2 activity of compounds 4 of WO2020/257870 and B12 from the NK-κB luciferase assay described in Example 11.

**Figure 5.** Percentage recovery from 3 or 6 week storage of compounds 4 of WO2020/257870, B12, B14 and B16 at 40 °C described in Example 12.

## Detailed description of the embodiments

The present invention is defined in the appended claims.

Reference will now be made in detail to certain embodiments of the invention. While the invention will be described in conjunction with the embodiments, it will be understood that the intention is not to limit the invention to those embodiments. On the contrary, the invention is intended to cover all alternatives, modifications, and equivalents, which may be included within the scope of the present invention as defined by the claims.

One skilled in the art will recognize many methods and materials similar or equivalent to those described herein, which could be used in the practice of the present invention. The present invention is in no way limited to the methods and materials described. It will be understood that the invention disclosed and defined in this specification extends to all alternative combinations of two or more of the individual features mentioned or evident from the text or drawings. All of these different combinations constitute various alternative aspects of the invention.

As used herein, except where the context requires otherwise, the term "comprise" and variations of the term, such as "comprising", "comprises" and "comprised", are not intended to exclude further additives, components, integers or steps.

For purposes of interpreting this specification, terms used in the singular will also include the plural and vice versa.

The general chemical terms used in the formulae herein have their usual meaning.

The term "aliphatic" is intended to include saturated and unsaturated, nonaromatic, straight chain, branched, acyclic, and cyclic hydrocarbons. Those skilled in the art will appreciate that aliphatic groups include, for example, alkyl,

alkenyl, alkynyl, cycloalkyl, and cycloalkenyl groups, and hybrids thereof such as (cycloalkyl)alkyl, (cycloalkenyl)alkyl and (cycloalkyl)alkenyl groups. In various embodiments, aliphatic groups comprise from 1-12, 1-8, 1-6, or 1-4 carbon atoms. In some embodiments, aliphatic groups comprise 5-21, from 9-21, or from 11-21 carbon atoms, such as from 11, 13, 15, 17, or 19 carbon atoms. In some embodiments, the aliphatic group is saturated.

The term "heteroaliphatic" is intended to include aliphatic groups, wherein one or more chain and/or ring carbon atoms are independently replaced with a heteroatom, preferably a heteroatom selected from oxygen, nitrogen and sulfur. In some embodiments, the heteroaliphatic is saturated. Examples of heteroaliphatic groups include linear or branched, heteroalkyl, heteroalkenyl, and heteroalkynyl groups.

The term "alkyl" is intended to include saturated straight chain and branched chain hydrocarbon groups. In some embodiments, alkyl groups have from 1 to 12, 1 to 10, 1 to 8, 1 to 6, or from 1 to 4 carbon atoms. In some embodiments, alkyl groups have from 5-21, from 9-21, or from 11-21 carbon atoms, such as from 11, 13, 15, 17, or 19 carbon atoms. Examples of straight chain alkyl groups include, but are not limited to, methyl, ethyl, n-propyl, n-butyl, n-pentyl, n-hexyl, n-heptyl, and n-octyl. Examples of branched alkyl groups include, but are not limited to, isopropyl, iso-butyl, sec-butyl, tert-butyl, neopentyl, isopentyl, and 2,2-dimethylpropyl.

The term "alkenyl" is intended to include straight and branched chain alkyl groups having at least one double bond between two carbon atoms. In some embodiments, alkenyl groups have from 2 to 12, from 2 to 10, from 2 to 8, from 2 to 6, or from 2 to 4 carbon atoms. In some embodiments, alkenyl groups have from 5-21, from 9-21, or from 11-21 carbon atoms, such as from 11, 13, 15, 17, or 19 carbon atoms. In some embodiments, alkenyl groups have one, two, or three carbon-carbon double bonds. Examples of alkenyl groups include, but are not limited to, vinyl, allyl, $-CH=CH(CH_3)$, $-CH=C(CH_3)_2$, $-C(CH_3)=CH_2$, and - $C(CH_3)=CH(CH_3)$.

The term "alkynyl" is intended to include straight and branched chain alkyl groups having at least one triple bond between two carbon atoms. In some embodiments, the alkynyl group have from 2 to 12, from 2 to 10, from 2 to 8, from 2 to 6, or from 2 to 4 carbon atoms. In some embodiments, alkynyl groups have one, two, or three carbon-carbon triple bonds. Examples include, but are not limited to, $-C=CH$, - $C=CH_3$, $-CH_2C=CH_3$, and $-C=CH_2CH(CH_2CH_3)_2$.

The term "heteroalkyl" is intended to include alkyl groups, wherein one or more chain carbon atoms are replaced with a heteroatom, preferably a heteroatom selected from the group consisting of oxygen, nitrogen, and sulfur. In some embodiments, the heteroalkyl is saturated. Heteroalkyl groups include, for example, polyethylene glycol groups and polyethylene glycol ether groups, and the like.

The term "cycloalkyl" is intended to include mono-, bi- or tricyclic alkyl groups. In some embodiments, cycloalkyl groups have from 3 to 12, from 3 to 10, from 3 to 8, from 3 to 6, from 3 to 5 carbon atoms in the ring(s). In some embodiments, cycloalkyl groups have 5 or 6 ring carbon atoms. Examples of monocyclic cycloalkyl groups include, but are not limited to, cyclopropyl, cyclobutyl, cyclopentyl, cyclohexyl, cycloheptyl, and cyclooctyl. In some embodiments, the cycloalkyl group has from 3 to 8, from 3 to 7, from 3 to 6, from 4 to 6, from 3 to 5, or from 4 to 5 ring carbon atoms. Bi- and tricyclic ring systems include bridged, spiro, and fused cycloalkyl ring systems. Examples of bi- and tricyclic ring cycloalkyl systems include, but are not limited to, bicyclo[2.1.1]hexanyl, bicyclo[2.2.1]heptanyl, adamantyl, and decalinyl.

The term "cycloalkenyl" is intended to include non-aromatic cycloalkyl groups having at least one double bond between two carbon atoms. In some embodiments, cycloalkenyl groups have one, two orthree double bonds. In some embodiments, cycloalkenyl groups have from 4 to 14, from 5 to 14, from 5 to 10, from 5 to 8, or from 5 to 6 carbon atoms in the ring(s). In some embodiments, cycloalkenyl groups have 5, 6, 7, or 8 ring carbon atoms. Examples of cycloalkenyl groups include cyclohexenyl, cyclopentenyl, cyclohexadienyl, butadienyl, pentadienyl, and hexadienyl.

The term "aryl" is intended to include cyclic aromatic hydrocarbon groups that do not contain any ring heteroatoms. Aryl groups include monocyclic, bicyclic and tricyclic ring systems. Examples of aryl groups include, but are not limited to, phenyl, azulenyl, heptalenyl, biphenyl, fluorenyl, phenanthrenyl, anthracenyl, indenyl, indanyl, pentalenyl, and naphthyl. In some embodiments, aryl groups have from 6 to 14, from 6 to 12, or from 6 to 10 carbon atoms in the ring(s). In some embodiments, the aryl groups are phenyl or naphthyl. Aryl groups include aromatic-aliphatic fused ring systems. Examples include, but are not limited to, indanyl and tetrahydronaphthyl.

The term "heterocyclyl" is intended to include non-aromatic ring systems containing 3 or more ring atoms, of which one or more is a heteroatom. In some embodiments, the heteroatom is nitrogen, oxygen, or sulfur. In some embodiments, the heterocyclyl group contains one, two, three, or four heteroatoms. In some embodiments, heterocyclyl groups include mono-, bi- and tricyclic rings having from 3 to 16, from 3 to 14, from 3 to 12, from 3 to 10, from 3 to 8, or from 3 to 6 ring atoms. Heterocyclyl groups include partially unsaturated and saturated ring systems, for example, imidazolinyl and imidazolidinyl. Heterocyclyl groups include fused and bridged ring systems containing a heteroatom, for example, quinuclidyl. Heterocyclyl groups include, but are not limited to, aziridinyl, azetidinyl, azepanyl, diazepanyl, 1,3-dioxanyl, 1,3-dioxolanyl, isoxazolidinyl, morpholinyl, piperazinyl, piperidinyl, pyranyl, pyrazolidinyl, pyrrolinyl, pyrrolidinyl, tetrahydrofuranyl, tetrahydrothienyl, thiadiazolidinyl, and trithianyl.

The term "heteroaryl" is intended to include aromatic ring systems containing 5 or more ring atoms, of which, one or more is a heteroatom. In some embodiments, the heteroatom is nitrogen, oxygen, or sulfur. In some embodiments, heteroaryl groups include mono-, bi- and tricyclic ring systems having from 5 to 16, from 5 to 14, from 5 to 12, from 5 to 10,

from 5 to 8, or from 5 to 6 ring atoms. Heteroaryl groups include, but are not limited to, pyrrolyl, pyrazolyl, triazolyl, tetrazolyl, oxazolyl, isoxazolyl, thiazolyl, pyridinyl, pyridazinyl, pyrimidinyl, pyrazinyl, thiophenyl, benzothiophenyl, furanyl, benzofuranyl, indolyl, azaindolyl (pyrrolopyridinyl), indazolyl, benzimidazolyl, pyrazolopyridinyl, triazolopyridinyl, benzotriazolyl, benzoxazolyl, benzothiazolyl, imidazopyridinyl, isoxazolopyridinylxanthinyl, guaninyl, quinolinyl, isoquinolinyl, tetrahydroquinolinyl, quinoxalinyl, and quinazolinyl. Heteroaryl groups include fused ring systems in which all of the rings are aromatic, for example, indolyl, and fused ring systems in which only one of the rings is aromatic, for example, 2,3-dihydroindolyl.

The term "halo" or "halogen" is intended to include F, Cl, Br, and I.

The term "heteroatom" is intended to include oxygen, nitrogen, sulfur, or phosphorus. In some embodiments, the heteroatom is selected from the group consisting of oxygen, nitrogen, and sulfur.

As used herein, the term "substituted" is intended to mean that one or more hydrogen atoms in the group indicated is replaced with one or more independently selected suitable substituents, provided that the normal valency of each atom to which the substituent(s) are attached is not exceeded, and that the substitution results in a stable compound. In some embodiments, optional substituents in the compounds described herein include but are not limited to halo, CN, $NO_2$, OH, $NH_2$, $NHR_{100}$, $NR_{100}R_{200}$, $C_{1-6}$haloalkyl, $C_{1-6}$haloalkoxy, $C(O)NH_2$, $C(O)NHR_{100}$, $C(O)NR_{100}R_{200}$, $SO_2R_{100}$, $OR_{100}$, $SR_{100}$, $S(O)R_{100}$, $C(O)R_{100}$, and $C_{1-6}$aliphatic; wherein $R_{100}$ and $R_{200}$ are each independently $C_{1-6}$aliphatic, for example $C_{1-6}$alkyl.

The term "carboxyl protecting group" as used herein is intended to mean a group that is capable of being readily removed to provide the OH group of a carboxyl group and protects the carboxyl group against undesirable reaction during synthetic procedures. Such protecting groups are described in Protective Groups in Organic Synthesis edited by T. W. Greene et al. (John Wiley & Sons, 1999) and 'Amino Acid-Protecting Groups' by Fernando Albericio (with Albert Isidro-Llobet and Mercedes Alvarez) Chemical Reviews 2009 (109) 2455-2504. Examples include, but are not limited to, alkyl and silyl groups, for example methyl, ethyl, *tert*-butyl, methoxymethyl, 2,2,2-trichloroethyl, benzyl, diphenylmethyl, trimethylsilyl, and tertbutyldimethylsilyl, and the like.

The term "amine protecting group" as used herein is intended to mean a group that is capable of being readily removed to provide the $NH_2$ group of an amine group and protects the amine group against undesirable reaction during synthetic procedures. Such protecting groups are described in Protective Groups in Organic Synthesis edited by T. W. Greene et al. (John Wiley & Sons, 1999) and 'Amino Acid-Protecting Groups' by Fernando Albericio (with Albert Isidro-Llobet and Mercedes Alvarez) Chemical Reviews 2009 (109) 2455-2504. Examples include, but are not limited to, acyl and acyloxy groups, for example acetyl, chloroacetyl, trichloroacetyl, o-nitrophenylacetyl, o-nitrophenoxy-acetyl, trifluoroacetyl, acetoacetyl, 4-chlorobutyryl, isobutyryl, picolinoyl, aminocaproyl, benzoyl, methoxy-carbonyl, 9-fluorenylmethoxycarbonyl, 2,2,2-trifluoroethoxycarbonyl, 2-trimethylsilylethoxy-carbonyl, tert-butyloxycarbonyl, benzyloxycarbonyl, p-nitrobenzyloxycarbonyl, 2,4-dichloro-benzyloxycarbonyl, and the like. Further examples include Cbz (carboxybenzyl), Nosyl (o- or p-nitrophenylsulfonyl), Bpoc (2-(4-biphenyl)isopropoxycarbonyl) and Dde (1-(4,4-dimethyl-2,6-dioxohexylidene)ethyl).

The term "carboxamide protecting group" as used herein is intended to mean a group that is capable of being readily removed to provide the $NH_2$ group of a carboxamide group and protects the carboxamide group against undesirable reaction during synthetic procedures. Such protecting groups are described in Protective Groups in Organic Synthesis edited by T. W. Greene et al. (John Wiley & Sons, 1999) and 'Amino Acid-Protecting Groups' by Fernando Albericio (with Albert Isidro-Llobet and Mercedes Alvarez) Chemical Reviews 2009 (109) 2455-2504. Examples include, but are not limited to, 9-xanthenyl (Xan), trityl (Trt), methyltrityl (Mtt), cyclopropyldimethylcarbinyl (Cpd), and dimethylcyclopropylmethyl (Dmcp).

As used herein, the term "and/or" means "and", or "or", or both.

The term "(s)" following a noun contemplates the singular and plural form, or both.

The term "ester" refers to a carboxylic acid group where the hydrogen of the hydroxyl group has been replaced by a saturated, straight-chain (i.e. linear) or branched hydrocarbon group. Specific examples of alkyl groups are methyl, ethyl, propyl, *iso*-propyl, *n*-butyl, *iso*-butyl, *sec*-butyl, *tert*-butyl, *n*-pentyl, *iso*pentyl, *n*-hexyl and 2,2-dimethylbutyl. The alkyl group may be a $C_1$-$C_6$ alkyl group. As used herein a wording defining the limits of a range of length such as, for example, "from 1 to 5" means any integer from 1 to 5, i.e. 1, 2, 3, 4 and 5. In other words, any range defined by two integers explicitly mentioned is meant to comprise and disclose any integer defining said limits and any integer comprised in said range. The alkyl group may be a branched alkyl group.

It is intended that reference to a range of numbers disclosed herein (for example, 1 to 10) also incorporates reference to all rational numbers within that range (for example, 1, 1.1, 2, 3, 3.9, 4, 5, 6, 6.5, 7, 8, 9, and 10) and also any range of rational numbers within that range (for example, 2 to 8, 1.5 to 5.5, and 3.1 to 4.7) and, therefore, all sub-ranges of all ranges expressly disclosed herein are hereby expressly disclosed. These are only examples of what is specifically intended and all possible combinations of numerical values between the lowest value and the highest value enumerated are to be considered to be expressly stated in this application in a similar manner.

As discussed above, the inventors have developed and optimised compounds for the treatment and/or

prevention of respiratory diseases or conditions, particularly those associated with an infectious agent, such as bacteria or virus. Specifically, the compounds may provide significant protection against viral replication in the lung when those compounds are administered to the upper respiratory tract. These compounds may have greater efficacy than other known TLR2 agonists. The TLR2 agonist efficacy may also occur without significantly compromising TLR specificity and/or causing significant weight loss in the animal models described herein. Further, the compounds may demonstrate favourable stability, e.g. within a formulation and/or biological matrix environment, as they may be relatively resistant to hydrolytic and/or enzyme-mediated processes degradation. Compounds of the invention demonstrate improved solution stability compared to other related compounds.

In one aspect, the present invention provides a compound of formula (I):

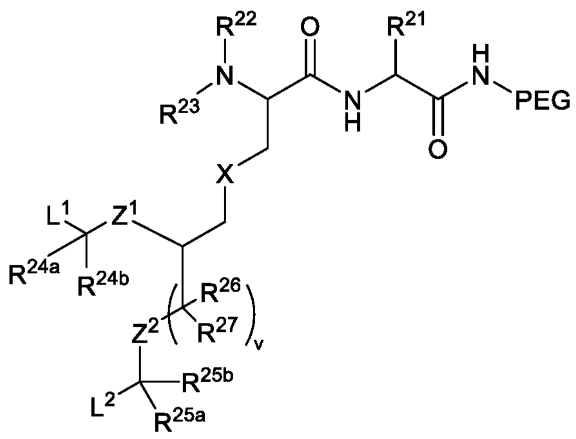

(I)

wherein:

$R^{21}$ is selected from the group consisting of H, $-CH_2OH$, $-CH_2CH_2OH$, $-CH(CH_3)OH$, $-CH_2OPO(OH)_2$, $-CH_2C(=O)NH_2$, $-CH_2CH_2C(=O)OH$ and $-CH_2CH_2C(=O)OR_8$, wherein any one of the alkyl hydrogens can be replaced with a halogen;

$R^{22}$ is H, $C_1$-$C_6$ aliphatic, an amino protecting group, $L^3$-C(=O)-, or $A^2$;

$L^1$ and $L^2$ are each independently $C_6$-$C_{21}$ aliphatic or $C_5$-$C_{20}$ heteroaliphatic;

$L^3$ is $C_1$-$C_{21}$ aliphatic or $C_2$-$C_{20}$ heteroaliphatic;

$A^2$ is an amino acid or a peptide;

$R^{23}$ is H or $C_1$-$C_6$ aliphatic;

$R^{24a}$ and $R^{25a}$ are each independently selected from $C_1$-$C_6$ aliphatic and $C_1$-$C_6$ heteroaliphatic and

$R^{24b}$ and $R^{25b}$ are each independently selected from H, $C_1$-$C_6$ aliphatic and $C_1$-$C_6$ heteroaliphatic, or

$R^{24a}$ and $R^{24b}$ together with the carbon atom to which they are attached form a $C_{3-8}$cycloalkyl or 3-8 membered heterocyclyl group, and/or

$R^{25a}$ and $R^{25b}$ together with the carbon atom to which they are attached form a $C_{3-8}$cycloalkyl or 3-8 membered heterocyclyl group;

X is selected from -S-, -S(=O)- and $-S(=O)_2$-;

v is an integer from 1-3

$R^{26}$ and $R^{27}$ are each independently selected from H and $C_1$-$C_6$ aliphatic;

$Z^1$ and $Z^2$ are independently selected from the group consisting of -C(=O)O-, -OC(=O)-, -C(=O)NR-, -NRC(=O)-,

-C(=O)S-, -SC(=O)-, -OC(=O)O-, -NRC(=O)O-, -OC(=O)NR-, and -NRC(=O)NR-; wherein each R is independently H or $C_1$-$C_6$ aliphatic;

PEG is a polyethylene glycol;

wherein any aliphatic, heteroaliphatic, cycloalkyl and heterocyclyl present in any of $R^{21}$, $R^{22}$, $R^{23}$, $R^{24a}$, $R^{24b}$, $R^{25a}$, $R^{25b}$, $R^{26}$, $R^{27}$, $L^1$, $L^2$ and $L^3$ is optionally substituted.

***$R^{24a}$, $R^{24b}$, $R^{25a}$ and $R^{26b}$***

The compounds of the invention possess branching at the carbon atom bonded to $L^1$/$L^2$ and $Z^1$/$Z^2$. Without wishing to be bound by theory, it is believed that branching at this position provides steric hindrance around the $Z^1$/$Z^2$ group making the compounds more resistant to degradation. Prior to the present invention it was not known whether branching at this position would be tolerated and surprisingly it was found that in addition to providing increased solution stability, substantial TLR2 activity may be retained compared to analogues comprising straight chain (i.e. non-branched) lipid moieties (e.g. compounds of formula (I) wherein $R^{24a}$, $R^{24b}$, $R^{25a}$ and $R^{25b}$ are each H).

In some embodiments, $R^{24a}$ and $R^{25a}$ are each independently selected from $C_1$-$C_6$ aliphatic and $C_1$-$C_6$ heteroaliphatic and $R^{24b}$ and $R^{25b}$ are each independently selected from H, $C_1$-$C_6$ aliphatic and $C_1$-$C_6$ heteroaliphatic.

In some embodiments, $R^{24a}$ and $R^{24b}$ together with the carbon atom to which they are attached form a $C_{3-8}$cycloalkyl or 3-8 membered heterocyclyl group.

In some embodiments, $R^{25a}$ and $R^{25b}$ together with the carbon atom to which they are attached form a $C_{3-8}$cycloalkyl or 3-8 membered heterocyclyl group.

In some embodiments, $R^{24a}$ is the same as $R^{25a}$.

In some embodiments, $R^{24b}$ is the same as $R^{25b}$.

In some embodiments, $R^{24b}$ and $R^{25b}$ are H.

In some embodiments, $R^{24a}$ and $R^{25a}$ are each independently $C_1$-$C_6$ aliphatic. In these embodiments, $R^{24a}$ and $R^{25a}$ each may independently be a $C_1$-$C_6$ alkyl. In some embodiments, $R^{24a}$ and $R^{25a}$ are each methyl.

In some embodiments, $R^{24a}$ and $R^{24b}$ together with the carbon atom to which they are attached form a $C_{3-8}$cycloalkyl or a 3-8 membered heterocyclyl selected from: cyclohexanyl, piperidinyl, cyclopentanyl, cycloheptanyl, epoxide, cyclopropyl, cyclobutyl, oxiranyl, aziridinyl, and pyranyl.

In some embodiments, $R^{25a}$ and $R^{25b}$ together with the carbon atom to which they are attached form a $C_{3-8}$cycloalkyl or a 3-8 membered heterocyclyl selected from: cyclohexanyl, piperidinyl, cyclopentanyl, cycloheptanyl, epoxide, cyclopropyl, cyclobutyl, oxiranyl, aziridinyl, and pyranyl.

***$R^{26}$, $R^{27}$ and v***

In some embodiments, $R^{26}$ and $R^{27}$ are the same.

In some embodiments, $R^{26}$ and $R^{27}$ are selected from H and methyl.

In some embodiments, one of $R^{26}$ and $R^{27}$ is H.

In some embodiments, $R^{26}$ is H.

In some embodiments, $R^{27}$ is H.

In some embodiments, $R^{26}$ and $R^{27}$ are each H.

In some embodiments, v is an integer selected from 1, 2 or 3. In some embodiments, v is 1 or 2. In some embodiments, v is 1. In some embodiments, v is 2.

***X***

In some embodiments, X is -S-.

In some embodiments, X is -S(O)- or -S(=O)$_2$-. In some embodiments, X is -S(O)-. In some embodiments, X is $-SO_2-$.

***$R^{22}$ and $R^{23}$***

In some embodiments, $R^{22}$ is selected from H, $C_1$-$C_6$ aliphatic, $L^3$-C(=O)-, or $A^2$.

In some embodiments, $R^{22}$ is selected from H, $C_{1-6}$ alkyl, an amino protecting group, $L^3$-C(=O)-, or $A^2$.

In some embodiments, $R^{22}$ is an amino protecting group. Preferably the amino protecting group is suitable for solid phase peptide coupling.

In some embodiments, $R^{22}$ is selected from H, $C_1$-$C_6$ aliphatic and $L^3$-C(=O)-, preferably H.

In some embodiments, $R^{22}$ is selected from H, $C_1$-$C_6$ alkyl, -C(=O)$C_1$-$C_6$alkyl or -C(=O)$C_{11}$-$C_{19}$alkyl.

In some embodiments, $R^{23}$ is H or $C_{1-6}$alkyl, preferably $R^{23}$ is H or methyl, more preferably $R^{23}$ is H.

***$R^{21}$***

In some embodiments, $R^{21}$ is $-CH_2OH$.

***$L^1$ and $L^2$***

In some embodiments, $L^1$ and $L^2$ are independently selected from $C_5$-$C_{21}$ aliphatic or $C_4$-$C_{20}$ heteroaliphatic. In some embodiments, $L^1$ and $L^2$ and independently selected from $C_{10}$-$C_{18}$ aliphatic or $C_{10}$-$C_{18}$ heteroaliphatic. In some embodiments, $L^1$ and $L^2$ are independently selected from $C_{14}$-alkyl and $C_{15}$-alkyl. Typically, $L^1$ and $L^2$ represent straight chain aliphatic groups of any of the specified lengths described herein. Accordingly, in some embodiments, the only branching in the lipid moieties is provided at $R^{24a}$, $R^{24b}$, $R^{25a}$ and $R^{25b}$.

***$Z^1$ and $Z^2$***

In some embodiments, $Z^1$ and $Z^2$ are independently selected from the group consisting of -C(=O)O-, -OC(=O)-, -C(=O)NR-, -NRC(=O)-, -C(=O)S-, -SC(=O)-, -OC(=O)O-, -NRC(=O)O-, -OC(=O)NR-, and -NRC(=O)NR-, wherein each R is independently H or $C_1$-$C_6$ aliphatic.

In some embodiments, $Z^1$ and $Z^2$ are the same.

In some embodiments, $Z^1$ and $Z^2$ are independently selected from -C(O)O- and -OC(O)-.

In some embodiments, $Z^1$ and $Z^2$ are each -C(O)O-. In these embodiments, the carbonyl carbon is directly bonded to the carbon atom bonded to $L^1$, $R^{24a}$ and $R^{24b}$ or $L^2$, $R^{25a}$ and $R^{25b}$. In these embodiments, the compound may be of formula (II):

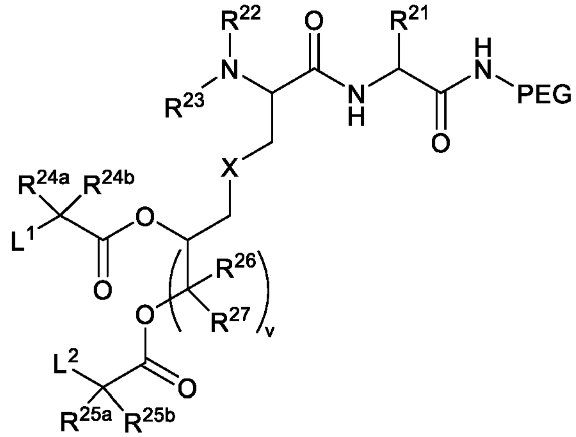

(II)

wherein each of $R^{21}$, $R^{22}$, $R^{23}$, $R^{24a}$, $R^{24b}$, $R^{25a}$, $R^{25b}$, $R^{26}$, $R^{27}$, v, X, $L^1$, $L^2$ and PEG have the meaning defined for any compound of the invention described herein, and wherein any aliphatic, heteroaliphatic, cycloalkyl and heterocyclyl present in any of $R^{21}$, $R^{22}$, $R^{23}$, $R^{24a}$, $R^{24b}$, $R^{25a}$, $R^{25b}$, $R^{26}$, $R^{27}$, $L^1$, $L^2$ and $L^3$ is optionally substituted.

***PEG***

In the compounds of the invention PEG represents a polyethylene glycol. The polyethylene glycol includes any length polymer of ethylene oxide. The polyethylene glycol may also include substituted polyethylene glycol ("substituted PEG"). In some embodiments, substituted PEG may be defined by formulas B-I or B-II as described herein.

In some embodiments, PEG is a substituted polyethylene glycol according to the following formula B-I:

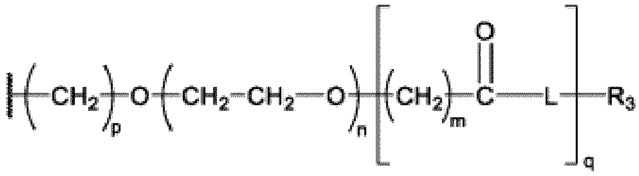

(B-I)

wherein

n is 3 to 100;

m is 1, 2, 3 or 4;

p is 2, 3 or 4;

q is null or 1;

$R_3$ is H, -$NH_2$ or -OH, wherein when q is null, $R_3$ is H and when q is 1, $R_3$ is -$NH_2$ or -OH;

L is null or consists of 1 to 10 units, wherein each unit is a natural alpha amino acid or derived from a natural alpha amino acid, and has the formula:

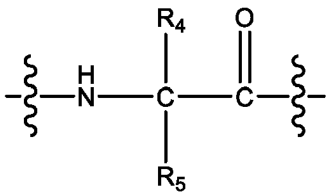

wherein $R_4$ is H; and

$R_5$ is the side chain, or second hydrogen of the amino acid.

In some embodiments, PEG is a substituted polyethylene glycol according to the following formula B-II:

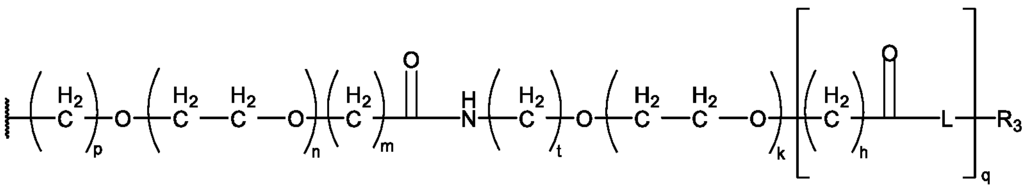

wherein

p is 2, 3 or 4;

n is 3 to 100;

m is 1, 2, 3 or 4;

t is 2, 3 or 4;

k is 3 to 100;

h is 1, 2, 3 or 4;

q is null or 1;

wherein when q is 1, $R_3$ is $-NH_2$ or -OH;

wherein when q is null, $R_3$ is H;

L is null or consists of 1 to 10 units, wherein each unit is a natural alpha amino acid or derived from a natural alpha amino acid, and has the formula:

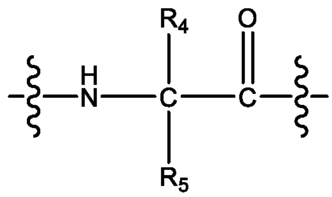

wherein $R_4$ is H; and

$R_5$ is the side chain, or second hydrogen of the amino acid.

In some embodiments of the substituted PEG of formula B-I or B-II, q is 1.

In the substituted PEG or formula B-I or B-II, n is an integer from 3 to 100. In some embodiments, n may be any sub-range within 3 to 100. In some embodiments, n has a minimum value of 4, 5, 6, 7, 8, 9, 10, 11, 15, 20, 24, 25, 26 or 27. In some embodiments, n has a maximum value of 100, 99, 98, 95, 90, 80, 70, 60, 50, 40, 30, 29, 28, 27, 25, 20, 15, 14, 13, 12 or 11. In some embodiments, n may be from any of these minimum values to any of these maximum values, for example from 4 to 100, 5 to 100, 10 to 100 or 10 to 30.

In some embodiments of the substituted PEG of formula B-I or B-II, n may be from 10 to 14, such as 11, or from 24 to 30, such as 27.

In some embodiments of the substituted PEG of formula B-I or B-II, m is from 1 to 3, such as 2.

In some embodiments of the substituted PEG of formula B-I or B-II, when q is 1, $R_3$ is $-NH_2$.

In some embodiments of the substituted PEG of formula B-I or B-II, L is a natural alpha amino acid residue.

***Further embodiments***

In some embodiments, the invention provides a compound of formula (I) wherein:

X is S;

v is an integer selected from 1 or 2;

$R^{26}$ and $R^{27}$ are H;

$Z^1$ and $Z^2$ are independently selected from the group consisting of -C(=O)O-, -OC(=O)-, -C(=O)NR-, -NRC(=O)-, -C(=O)S-, -SC(=O)-, -OC(=O)O-, -NRC(=O)O-, -OC(=O)NR-, and - NRC(=O)NR-; wherein each R is independently H or $C_1$-$C_6$ aliphatic;

$R^{22}$ is selected from the group consisting of H, $C_1$-$C_6$ alkyl, -C(=O) $C_1$-$C_6$ alkyl or -C(=O)$C_{11}$-$C_{19}$alkyl; and

$L^1$ and $L^2$ and independently selected from $C_{10}$-$C_{18}$ aliphatic or $C_{10}$-$C_{18}$ heteroaliphatic.

In some embodiments, the invention provides a compound of formula (I) wherein:

$R^{24b}$ and $R^{25b}$ are H;

$R^{24a}$ and $R^{25a}$ are selected from $C_{1-6}$alkyl and $C_{1-6}$heteroalkyl;

X is S;

v is an integer selected from 1 or 2;

$R^{26}$ and $R^{27}$ are H;

$Z^1$ and $Z^2$ are independently selected from the group consisting of -C(=O)O-, -OC(=O)-, -C(=O)NR-, -NRC(=O)-, -C(=O)S-, -SC(=O)-, -OC(=O)O-, -NRC(=O)O-, -OC(=O)NR-, and - NRC(=O)NR-; wherein each R is independently H or $C_1$-$C_6$ aliphatic;

$R^{22}$ is selected from the group consisting of H, $C_1$-$C_6$ alkyl, -C(=O) $C_1$-$C_6$ alkyl or -C(=O)$C_{11}$-$C_{19}$alkyl; and

$L^1$ and $L^2$ and independently selected from $C_{10}$-$C_{18}$ aliphatic or $C_{10}$-$C_{18}$ heteroaliphatic.

In some embodiments, the invention provides a compound of formula (I) wherein:

$R^{24b}$ and $R^{25b}$ are H;

$R^{24a}$ and $R^{25a}$ are selected from $C_{1-6}$alkyl;

X is S;

v is an integer selected from 1 or 2;

$R^{26}$ and $R^{27}$ are H;

$Z^1$ and $Z^2$ are independently selected from the group consisting of -C(=O)O-, -OC(=O)-, -C(=O)NR-, -NRC(=O)-, -C(=O)S-, -SC(=O)-, -OC(=O)O-, -NRC(=O)O-, -OC(=O)NR-, and - NRC(=O)NR-; wherein each R is independently H or $C_1$-$C_6$ aliphatic;

$R^{22}$ is selected from the group consisting of H, $C_1$-$C_6$ alkyl, -C(=O) $C_1$-$C_6$ alkyl or -C(=O)$C_{11}$-$C_{19}$alkyl; and

$L^1$ and $L^2$ and independently selected from $C_{10}$-$C_{18}$ aliphatic or $C_{10}$-$C_{18}$ heteroaliphatic.

In some embodiments, the invention provides a compound of formula (I) wherein:

$R^{24b}$ and $R^{25b}$ are H;

$R^{24a}$ and $R^{25a}$ are each methyl;

X is S;

v is an integer selected from 1 or 2;

$R^{26}$ and $R^{27}$ are H;

$Z^1$ and $Z^2$ are independently selected from the group consisting of -C(=O)O-, -OC(=O)-, -C(=O)NR-, -NRC(=O)-, -C(=O)S-, -SC(=O)-, -OC(=O)O-, -NRC(=O)O-, -OC(=O)NR-, and - NRC(=O)NR-; wherein each R is independently H or $C_1$-$C_6$ aliphatic;

$R^{22}$ is selected from the group consisting of H, $C_1$-$C_6$ alkyl, -C(=O) $C_1$-$C_6$ alkyl or -C(=O)$C_{11}$-$C_{19}$alkyl; and

$L^1$ and $L^2$ and independently selected from $C_{10}$-$C_{18}$ aliphatic or $C_{10}$-$C_{18}$ heteroaliphatic.

In some embodiments, the invention provides a compound of formula (I) wherein:

$R^{24b}$ and $R^{25b}$ are H;

$R^{24a}$ and $R^{25a}$ are each methyl;

X is S;

v is an integer selected from 1 or 2;

$R^{26}$ and $R^{27}$ are H;

$Z^1$ and $Z^2$ are independently selected from the group consisting of -C(=O)O-, -OC(=O)-, -C(=O)NR-, -NRC(=O)-, -C(=O)S-, -SC(=O)-, -OC(=O)O-, -NRC(=O)O-, -OC(=O)NR-, and - NRC(=O)NR-; wherein each R is independently H or $C_1$-$C_6$ aliphatic;

$R^{22}$ is selected from the group consisting of H, $C_1$-$C_6$ alkyl, -C(=O) $C_1$-$C_6$ alkyl or -C(=O)$C_{11}$-$C_{19}$alkyl; and

$L^1$ and $L^2$ and independently selected from $C_{10}$-$C_{18}$ alkyl.

In some embodiments, the invention provides a compound of formula (I) wherein:

$R^{24b}$ and $R^{25b}$ are H;

$R^{24a}$ and $R^{25a}$ are each methyl;

X is S;

v is an integer selected from 1 or 2;

$R^{26}$ and $R^{27}$ are H;

$Z^1$ and $Z^2$ are independently selected from the group consisting of -C(=O)O-;

$R^{22}$ and $R^{23}$ are each H; and

$L^1$ and $L^2$ and independently selected from $C_{10}$-$C_{18}$ alkyl.

In some embodiments, the invention provides a compound of formula (I) wherein:

$R^{24b}$ and $R^{25b}$ are H;

$R^{24a}$ and $R^{25a}$ are each methyl;

X is S;

v is an integer selected from 1 or 2;

$R^{26}$ and $R^{27}$ are H;

$Z^1$ and $Z^2$ are independently selected from the group consisting of -C(=O)O-;

$R^{21}$ is $-CH_2OH$;

$R^{22}$ and $R^{23}$ are each H; and

$L^1$ and $L^2$ and independently selected from $C_{10}$-$C_{18}$ alkyl.

In some embodiments, $R^{24b}$ and $R^{25b}$ are each H. In these embodiments, the compound of formula (I) may be a compound of formula (III):

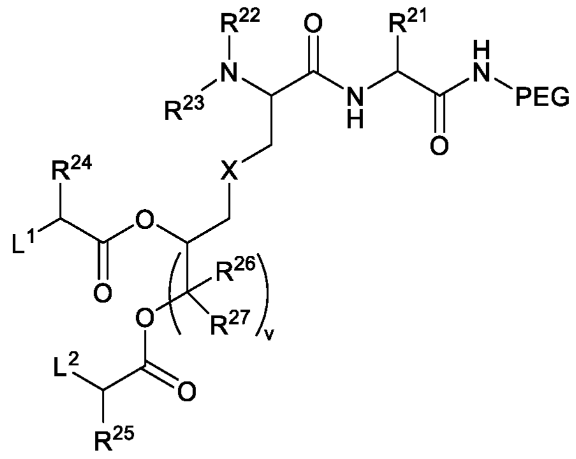

(III)

wherein each of $R^{21}$, $R^{22}$, $R^{23}$, $R^{26}$, $R^{27}$, v, X, $L^1$, $L^2$ and PEG have the meanings provided in any embodiment described herein and

$R^{24}$ and $R^{25}$ are independently selected from $C_1$-$C_6$ aliphatic and $C_1$-$C_6$ heteroaliphatic, wherein any aliphatic, heteroaliphatic, cycloalkyl and heterocyclyl present in any of $R^{21}$, $R^{22}$, $R^{23}$, $R^{24}$, $R^{25}$, $R^{26}$, $R^{27}$, $L^1$, $L^2$ and $L^3$ is optionally substituted.

$R^{24}$ and $R^{25}$ may be any group described herein for $R^{24a}$ and $R^{25a}$, respectively.

In some embodiments, $R^{24}$ and $R^{25}$ are the same.
In some embodiments, $R^{24}$ and $R^{25}$ are independently selected from $C_1$-$C_6$ alkyl and $C_1$-$C_6$ heteroaliphatic.
In some embodiments, $R^{24}$ and $R^{25}$ are independently $C_{1-6}$alkyl.
In some embodiments, $R^{24}$ and $R^{25}$ are independently $C_{1-4}$alkyl.
In some embodiments, $R^{24}$ and $R^{25}$ are each methyl.

**Formulations**

The present invention also provides for compositions containing a compound of the invention or a pharmaceutically acceptable salt, solvate or prodrug thereof, and a pharmaceutically acceptable carrier, diluent or excipient. Any of the compounds described herein or variations thereof may be included in the compositions of the invention. These compositions may also be referred to as pharmaceutical compositions.
As discussed above, the present invention provides Toll-Like Receptor 2 protein (TLR2) agonist compounds and their compositions. In humans, TLR2 plays a fundamental role in the recognition of pathogens and activation of the innate immunity response. It is encoded by the TLR2 gene and is expressed on the surface of specific cells.
Without wishing to be bound by any theory or mode of action, it is believed that the compounds of the invention described herein are agonists of TLR2 and show activity by binding at TLR2 and stimulating the innate immune system. The innate immune system forms an immediate defence against pathogens such as pathogens that infect and replicate in cells lining the respiratory tract. Research has shown that agents which stimulate the innate immune system may be useful for limiting respiratory infections, which may provide protection from infections both in isolation and during the period between inoculation and the formation of antibodies and immune cells. Such agents are considered to be useful for the treatment and/or prevention of respiratory infections, or respiratory conditions caused by or associated with infectious agents such as a virus (such as Influenza A) or bacterium (such as pneumonia) in a non-antigen specific manner.
In this regard, compounds of the invention as described herein may have activity, both activation of human TLR2 and inhibition of viral progression, that is at least comparable to other TLR2 agonists such as Pam2Cys-Ser-K4, Pam2Cys-Ser-Ser-PEG and Pam3Cys-Ser-PEG.
As used herein, 'Ser' refers to the amino acid serine and 'Cys' refers to the amino acid cysteine.
As used herein, 'PEG' refers to a moiety comprising or consisting of a polymeric section of polyethylene glycol. Unless otherwise defined, reference to 'PEG' includes any length polymer of ethylene oxide. Reference to PEG also includes substituted PEG. In some embodiments, substituted PEG may be defined by formulas B-I or B-II as described herein.
In one aspect, therefore, the present invention provides a method of treating and/or preventing a disease, comprising raising an innate immune response in a subject by administering an effective amount of a compound of the

invention as described herein or a pharmaceutically acceptable salt, solvate or prodrug thereof to the subject in need thereof.

In another aspect, the present invention provides a method of treating and/or preventing a disease caused by an infectious agent, comprising administering to a subject in need thereof an effective amount of a compound of the invention as described herein or a pharmaceutically acceptable salt, solvate or prodrug thereof.

In another aspect, the present invention provides a method of treating and/or preventing a respiratory disease or condition associated with a viral or bacterial infection, comprising administering to a subject in need thereof a compound of the invention as described herein or a pharmaceutically acceptable salt, solvate or prodrug thereof.

In another aspect, the present invention provides a method of treating and/or preventing a respiratory infection, comprising administering to a subject in need thereof a compound of the invention as described herein or a pharmaceutically acceptable salt, solvate or prodrug thereof. Preferably the method further comprises a step of identifying a subject having a respiratory infection.

In another aspect, the present invention provides a method for reducing airway inflammation, comprising administering to a subject in need thereof a compound of the invention as described herein or a pharmaceutically acceptable salt, solvate or prodrug thereof.

The present invention also provides a method of improving the ability of a subject to control a respiratory disease or condition during a respiratory viral infection, the method comprising administering to a subject in need thereof a compound of the invention as described herein or a pharmaceutically acceptable salt, solvate or prodrug thereof. Preferably the infection is not a rhinovirus infection.

The present invention also provides a method of treating and/or preventing a disease or condition associated with the TLR2 receptor, the method comprising administering to a subject in need thereof a compound of the invention as described herein or a pharmaceutically acceptable salt, solvate or prodrug thereof.

The present invention also provides a method of agonising TLR2 activity in a cell, the method comprising contacting the cell with a compound of the invention as described herein or a pharmaceutically acceptable salt, solvate or prodrug thereof. In some embodiments, the cell is contacted with the compound by administration of the compound or pharmaceutically acceptable salt, solvate or prodrug thereof, or composition comprising the compound, pharmaceutically acceptable salt, solvate or prodrug thereof, to a subject in need thereof. In some embodiments, the cell is provided in the form of a cell culture.

In another aspect, the present invention provides for use of a compound of the invention as described herein or a pharmaceutically acceptable salt, solvate or prodrug thereof in the preparation of a medicament for raising an innate immune response in a subject.

In another aspect, the present invention provides for use of a compound of the invention as described herein or a pharmaceutically acceptable salt, solvate or prodrug thereof in the preparation of a medicament for treating and/or preventing a disease caused by an infectious agent.

In another aspect, the present invention further provides for use of a compound of the invention as described herein or a pharmaceutically acceptable salt, solvate or prodrug thereof in the preparation of a medicament for treating and/or preventing a respiratory disease or condition associated with a viral or bacterial infection in a subject.

In another aspect, the present invention further provides for use of a compound of the invention as described herein or a pharmaceutically acceptable salt, solvate or prodrug thereof in the preparation of a medicament for treating and/or preventing a respiratory infection in a subject.

In yet another aspect, the present invention provides for use of a compound of the invention as described herein or a pharmaceutically acceptable salt, solvate or prodrug thereof in the preparation of a medicament for treating and/or preventing a respiratory infection.

In another aspect, the present invention further provides use of a compound of the invention as described herein or a pharmaceutically acceptable salt, solvate or prodrug thereof in the preparation of a medicament for reducing airway inflammation.

In another aspect, the present invention further provides use of a compound of the invention as described herein or a pharmaceutically acceptable salt, solvate or prodrug thereof in the preparation of a medicament for improving the ability of a subject to control a respiratory disease or condition during a respiratory viral infection. Preferably the infection is not a rhinovirus infection.

In another aspect, the present invention further provides use of a compound of the invention as described herein or a pharmaceutically acceptable salt, solvate or prodrug thereof in the preparation of a medicament for treating and/or preventing a disease or condition associated with the TLR2 receptor.

In one aspect, the present invention provides for use of a compound of the invention as described herein or a pharmaceutically acceptable salt, solvate or prodrug thereof, for raising an innate immune response in a subject.

In another aspect, the present invention provides for use of a compound of the invention as described herein or a pharmaceutically acceptable salt, solvate or prodrug thereof, for preventing a disease caused by an infectious agent, in a subject.

In another aspect, the present invention provides for use of a compound of the invention as described herein or a pharmaceutically acceptable salt, solvate or prodrug thereof, for treating and/or preventing a respiratory disease or condition associated with a viral or bacterial infection in a subject.

In a further aspect, the invention provides for use of a compound of the invention as described herein or a pharmaceutically acceptable salt, solvate or prodrug thereof, for (a) treating and/or preventing a respiratory infection in a subject; (b) reducing airway inflammation in a subject; (c) controlling a respiratory disease or condition during a respiratory viral infection in a subject; (d) for treating and/or preventing a disease or condition associated with the TLR2 receptor.

In any of these aspects, the compound may be administered in a composition. Typically, the composition further comprises a pharmaceutically acceptable carrier, diluent or excipient. The composition may be formulated for administration to the upper and/or lower respiratory tract, for example by inhalation or intranasally.

In any aspect of the invention, the compound of the invention as described herein or a pharmaceutically acceptable salt, solvate or prodrug thereof may be conjugated with other compounds. Other compounds are any of those described herein.

In any aspect of the invention, the compound of the invention as described herein or a pharmaceutically acceptable salt, solvate or prodrug thereof is administered once daily or once weekly.

In any aspect of the invention, where prevention or prophylaxis is intended or required, the compound is administered to the subject before any clinically or biochemically detectable symptoms of viral infection.

In any aspect of the invention, administration of the compound of the invention as described herein or a pharmaceutically acceptable salt, solvate or prodrug thereof to a subject reduces viral load in a subject. Preferably, the viral load is reduced in the respiratory tract, for example the upper and/or lower respiratory tract. Preferably, the viral load is reduced in the lungs.

In any aspect herein, the infectious agent may be a virus. Preferably, the virus is one associated with infection of the respiratory tract. Even more preferably, the virus is influenza. In any aspect, the virus is not a rhinovirus.

Influenza (commonly referred to as "the flu") is an infectious disease caused by RNA viruses of the family Orthomyxoviridae (the influenza viruses) that affects birds and mammals. The most common symptoms of the disease are chills, fever, sore throat, muscle pains, severe headache, coughing, weakness/fatigue and general discomfort.

The influenza viruses make up three of the five genera of the family Orthomyxoviridae. Influenza Type A and Type B viruses co-circulate during seasonal epidemics and can cause severe influenza infection. Influenza Type C virus infection is less common but can be severe and cause local epidemics.

Influenza Type A virus can be subdivided into different serotypes or subtypes based on the antibody response to these viruses. Influenza A viruses are divided into subtypes based on two proteins on the surface of the virus: the hemagglutinin (H) and the neuraminidase (N). There are 18 different hemagglutinin subtypes and 11 different neuraminidase subtypes. (H1 through H18 and N1 through N11 respectively.) The sub types that have been confirmed in humans are H1N1, H1N2, H2N2, H3N2, H5N1, H7N2, H7N3, H7N7, H9N2 and H10N7.

Influenza has an enormous impact on public health with severe economic implications in addition to the devastating health problems, including morbidity and even mortality. Accordingly, there is a need for therapeutic agents which can prevent infection, or reduce severity of infection in individuals.

In any aspect or embodiment of the invention, the influenza infection for which treatment or prevention is required is an infection with a virus selected from the group consisting of influenza Types A, B or C.

The term 'respiratory disease' or 'respiratory condition' refers to any one of several ailments that involve inflammation and affect a component of the respiratory system including the upper (including the nasal cavity, pharynx and larynx) and lower respiratory tract (including trachea, bronchi and lungs). The inflammation in the upper and lower respiratory tract may be associated with or caused by viral infection or an allergen. It is expected that the anti-inflammatory activity of the compounds either alone or when co-administered with a glucocorticoid would make them particularly suitable for treatment of these disease or conditions.

A symptom of respiratory disease may include cough, excess sputum production, a sense of breathlessness or chest tightness with audible wheeze. Exercise capacity may be quite limited. In asthma the FEV1.0 (forced expiratory volume in one second) as a percentage of that predicted nomographically based on weight, height and age, may be decreased as may the peak expiratory flow rate in a forced expiration. In COPD the FEV1.0 as a ratio of the FVC is typically reduced to less than 0.7. The impact of each of these conditions may also be measured by days of lost work/school, disturbed sleep, requirement for bronchodilator drugs, requirement for glucocorticoids including oral glucocorticoids.

The existence of, improvement in, treatment of or prevention of a respiratory disease may be determined by any clinically or biochemically relevant method of the subject or a biopsy therefrom. For example, a parameter measured may be the presence or degree of lung function, signs and symptoms of obstruction; exercise tolerance; nighttime awakenings; days lost to school or work; bronchodilator usage; Inhaled corticosteroid (ICS) dose; oral glucocorticoid (GC) usage; need for other medications; need for medical treatment; hospital admission.

As used herein, the term respiratory infection means an infection by virus or bacteria anywhere in the respiratory tract. Examples of respiratory infection include but are not limited to colds, sinusitis, throat infection, tonsillitis, laryngitis,

bronchitis, pneumonia or bronchiolitis. Preferably, in any embodiment of the invention the respiratory infection is a cold.

An individual may be identified as having a respiratory tract infection by viral testing and may exhibit symptoms of itchy watery eyes, nasal discharge, nasal congestion, sneezing, sore throat, cough, headache, fever, malaise, fatigue and weakness. In one aspect, a subject having a respiratory infection may not have any other respiratory condition. Detection of the presence or amount of virus may be by PCR/sequencing of RNA isolated from clinical samples (nasal wash, sputum, BAL) or serology.

The term "pharmaceutically acceptable" may be used to describe any pharmaceutically acceptable salt, hydrate or prodrug, or any other compound which upon administration to a subject, is capable of providing (directly or indirectly) a compound of the invention as described herein, or a pharmaceutically acceptable salt, solvate or prodrug thereof, or an active metabolite or residue thereof.

Suitable pharmaceutically acceptable salts may include, but are not limited to, salts of pharmaceutically acceptable inorganic acids such as hydrochloric, sulphuric, phosphoric, nitric, carbonic, boric, sulfamic, and hydrobromic acids, or salts of pharmaceutically acceptable organic acids such as acetic, propionic, butyric, tartaric, maleic, hydroxymaleic, fumaric, malic, citric, lactic, mucic, gluconic, benzoic, succinic, oxalic, phenylacetic, methanesulphonic, toluenesulphonic, benzenesulphonic, salicylic, sulphanilic, aspartic, glutamic, edetic, stearic, palmitic, oleic, lauric, pantothenic, tannic, ascorbic and valeric acids.

Base salts may include, but are not limited to, those formed with pharmaceutically acceptable cations, such as sodium, potassium, lithium, calcium, magnesium, zinc, ammonium, alkylammonium such as salts formed from triethylamine, alkoxyammonium such as those formed with ethanolamine and salts formed from ethylenediamine, choline or amino acids such as arginine, lysine or histidine. General information on types of pharmaceutically acceptable salts and their formation is known to those skilled in the art and is as described in general texts such as "Handbook of Pharmaceutical salts" P.H.Stahl, C.G.Wermuth, 1st edition, 2002, Wiley-VCH.

In the case of compounds that are solids, it will be understood by those skilled in the art that the inventive compounds, agents, solvates and salts may exist in different crystalline or polymorphic forms, all of which are intended to be within the scope of the present invention and specified formulae.

The term "polymorph" includes any crystalline form of compounds of the invention as described herein, such as anhydrous forms, hydrous forms, solvate forms and mixed solvate forms.

It will be understood that compounds of the invention may possess a chiral centre and may therefore exist in an *R*- or *S*- configuration. The compounds may be provided in the form of a racemate or in an enatio- or diastereo-enriched form. Enantio- and diastereo-enriched forms of the compounds may be obtained either through asymmetric synthesis, the incorporation of chiral pool materials or through a stereoselective resolution. The compounds may therefore be provided as a purified enantiomer or diastereomer, or as a mixture of any ratio thereof. The isomers may be separated conventionally by chromatographic methods or using a resolving agent. Alternatively the individual isomers may be prepared by asymmetric synthesis using chiral intermediates. Where the compound has a carbon-carbon double bond, it may occur in *Z*- or *E*- form and all isomeric forms of the compounds being included in the present invention.

The compounds of the invention are intended to include, where applicable, solvated as well as unsolvated forms of the compounds. As used herein, the term "solvate" refers to a complex of variable stoichiometry formed by association of a solvent with a compound of the invention. The solvate may therefore comprise sub-stoichiometric amounts of the solvent, eqimolar amounts of the solvent or super-stoichiometric amounts of the solvent relative to the compound of the invention. Such solvents for the purpose of the invention may not interfere with the biological activity of the solute. Examples of suitable solvents include, but are not limited to, water, methanol, ethanol and acetic acid. Preferably the solvent used is a pharmaceutically acceptable solvent. Examples of suitable pharmaceutically acceptable solvents include, without limitation, water, ethanol and acetic acid. Most preferably the solvent used is water. Solvates wherein the solvent is water may be referred to as hydrates.

Basic nitrogen-containing groups may be quarternised with such agents as lower alkyl halide, such as methyl, ethyl, propyl, and butyl chlorides, bromides and iodides; dialkyl sulfates like dimethyl and diethyl sulfate; and others.

The compounds as described herein are to also include isotope variations, such as the replacement of hydrogen for deuterium.

Compounds of the present invention may exist in and be isolated in optically active and racemic forms. As would be understood by a person skilled in the art, the present invention is intended to encompass any racemic, optically active or stereoisomeric form, or mixtures thereof, of compounds of the invention which possess the useful properties described herein. It is well known in the art how to prepare such forms (for example, by resolution of racemic mixtures by recrystallization, by synthesis from optically-active starting materials, by chiral synthesis, or by chiral chromatographic separation). The compounds of formula (I) contain at least 6 potential stereogenic centres depending on the selection of substituents. These centres are designated with an * in formula (I*) below.

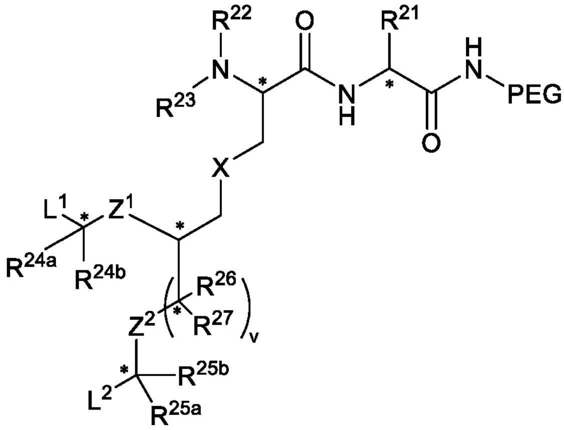

(I*)

wherein $R^{22}$, $R^{23}$, v, X, $Z^1$, $Z^2$, $L^1$ and $L^1$ have the meanings indicated above, and wherein $R^{24a}$ is different to $R^{24b}$, $R^{25a}$ is different to $R^{25b}$, $R^{21}$ is not H, and $R^{26}$ is different to $R^{27}$. Any of these stereocentres may be in the R or S configuration.

In some embodiments, $R^{26}$ and $R^{27}$ are the same (e.g. each being H). In these embodiments, the remaining potential stereocentres are denoted with an * in the following formula (I**):

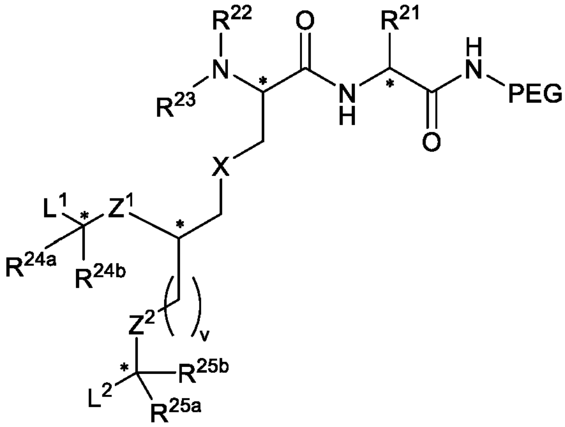

In some embodiments, the compound of formula (I) is chiral with the conformations shown in formula (IV):

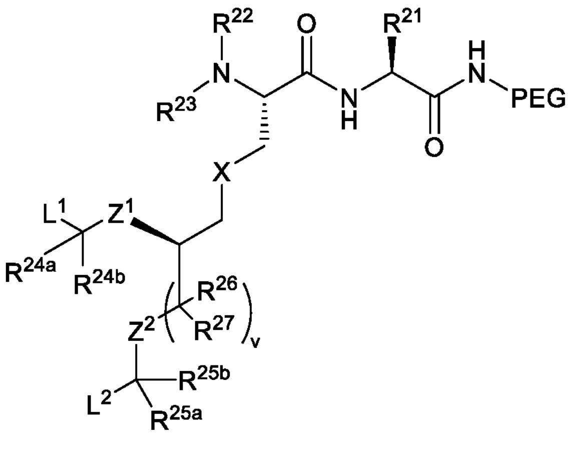

(IV)

wherein each of $R^{21}$, $R^{22}$, $R^{23}$, $R^{24a}$, $R^{24b}$, $R^{25a}$, $R^{25b}$, X, $Z^1$, $Z^2$, v, $L^1$, $L^2$ and PEG have the meanings provided in any compound of the invention, and wherein any aliphatic, heteroaliphatic, cycloalkyl and heterocyclyl present in any of $R^{21}$, $R^{22}$, $R^{23}$, $R^{24a}$, $R^{24b}$, $R^{25a}$, $R^{25b}$, $R^{26}$, $R^{27}$, $L^1$, $L^2$ and $L^3$ is optionally substituted.

In any aspect of the present invention, 1%, 5%, 10%, 20%, 30%, 40%, 50%, 60%, 70%, 80%, 90%, 95%, 96%, 97%, 98%, 99% or more than 99% of the compound present in a composition is the R diastereomer around any one or more of the chiral centres denoted * in formula (I*) or (I**) of the compound described herein.

In any aspect of the present invention, 1%, 5%, 10%, 20%, 30%, 40%, 50%, 60%, 70%, 80%, 90%, 95%, 96%, 97%, 98%, 99% or more than 99% of the compound present in a composition is the S diastereomer around any one or more of the chiral centred denoted * in formula (I*) or (I**) of the compound described herein.

The compounds of the invention demonstrate improved solution stability under accelerated degradation conditions relative to other related compounds. Solution stability may be assess by measuring the concentration of compound in a solution at day 0 and comparing the concentration of the compound after a period of time, such as 14 days. Solution stability may be assessed under ambient conditions, eg 25°C and 65% relative humidity, or under accelerated conditions, eg 40°C and 75% relative humidity. Typically, an acceptable stability for a compound of interest for the indications of the invention when stored for 14 days in solution under accelerated conditions would be retention of at least 80% concentration in the solution of the compound relative to the initial concentration of the compound in the solution. Typically, the solution may be a saline solution (eg 0.9% aq. NaCl) or phosphate-buffered saline (PBS; eg pH 7.4). In some embodiments, the compounds of the invention after 14 day storage in pH 7.4 PBS buffer is at least about 80%, 85%, 90%, 91%, 92% or greater relative to the amount of compound detected in the solution at day 0.

A "prodrug" is a compound that may not fully satisfy the structural requirements of the compounds provided herein, but is modified *in vivo*, following administration to a subject or patient, to produce a compound of the invention as described herein. For example, a prodrug may be an acylated derivative of a compound as provided herein. Prodrugs include compounds wherein hydroxy, carboxy, amine or sulfhydryl groups are bonded to any group that, when administered to a mammalian subject, cleaves to form a free hydroxy, carboxy, amino, or sulfhydryl group, respectively. Examples of prodrugs include, but are not limited to, acetate, formate, phosphate and benzoate derivatives of alcohol and amine functional groups within the compounds provided herein. Prodrugs of the compounds provided herein may be prepared by modifying functional groups present in the compounds in such a way that the modifications are cleaved *in vivo* to generate the parent compounds.

Prodrugs include compounds wherein an amino acid residue, or a polypeptide chain of two or more (eg, two, three or four) amino acid residues which are covalently joined to free amino, and amido groups of compounds of the invention. The amino acid residues include the 20 naturally occurring amino acids commonly designated by three letter symbols and also include, 4-hydroxyproline, hydroxylysine, demosine, isodemosine, 3-methylhistidine, norvlin, beta-alanine, gamma-aminobutyric acid, citrulline, homocysteine, homoserine, ornithine and methionine sulfone. Prodrugs also include compounds wherein carbonates, carbamates, amides and alkyl esters which are covalently bonded to the above substituents of a compound of the invention.

The compounds of the invention as described herein or a pharmaceutically acceptable salt, solvate or prodrug thereof may be covalent irreversible or covalent reversible agonists of the active site of a protein.

Where a protecting group (PG) is referred to, a person skilled in the art would readily understand what type of protecting group would be suitable. Examples of suitable amine protecting groups for the purposes described herein include (but are not limited to) tert-butyloxycarbonyl (t-Boc) and 9H-fluoren-9-ylmethoxycarbonyl (Fmoc).

Pharmaceutical compositions may be formulated from compounds of the invention as described herein for any appropriate route of administration including, for example, topical (for example, transdermal or ocular), oral, buccal, respiratory (for example, nasal, inhalation, intrapulmonary), vaginal, rectal or parenteral administration. The term parenteral as used herein includes subcutaneous, intradermal, intravascular (for example, intravenous), intramuscular, spinal, intracranial, intrathecal, intraocular, periocular, intraorbital, intrasynovial and intraperitoneal injection, as well as any similar injection or infusion technique. Suitable oral forms include, for example, tablets, troches, lozenges, aqueous or oily suspensions, dispersible powders or granules, emulsions, hard or soft capsules, or syrups or elixirs. For intravenous, intramuscular, subcutaneous, or intraperitoneal administration, one or more compounds may be combined with a sterile aqueous solution which is preferably isotonic with the blood of the recipient. Such formulations may be prepared by dissolving solid active ingredient in water containing physiologically compatible substances such as sodium chloride or glycine, and having a buffered pH compatible with physiological conditions to produce an aqueous solution, and rendering said solution sterile. The formulations may be present in unit or multi-dose containers such as sealed ampoules or vials. Examples of components are described in Martindale - The Extra Pharmacopoeia (Pharmaceutical Press, London 1993) and Martin (ed.), Remington's Pharmaceutical Sciences. Preferably, the compositions are formulated for administration to the respiratory tract, for example, by intrapulmonary administration (eg. inhalation) or intranasal administration. The compositions may be administered to the upper and/or lower respiratory tract.

Preferably, the pharmaceutical compositions are in a form suitable for administration via the respiratory route, and may be in any form such as a powder, liquid or suspension. Such compositions may target tissue including pulmonary tissue (including alveolus, terminal bronchiole, bronchiole, and bronchus) or the nasal cavity (including paranasal cavity, frontal sinus, ethmoid sinus, maxillary sinus, sphenoidal sinus, superior turbinate, middle turbinate, and inferior turbinate).

In the context of this specification the term "administering" and variations of that term including "administer" and "administration", includes contacting, applying, delivering or providing a compound or composition of the invention to an organism, or a surface by any appropriate means.

The dose of the biologically active compound according to the invention may vary within wide limits and may be

adjusted to individual requirements. Active compounds according to the present invention are generally administered in a therapeutically effective amount.

A composition according to the present invention is to be administered in an effective amount. The phrase 'therapeutically effective amount' or 'effective amount' generally refers to an amount of a compound of the invention described herein, a pharmaceutically acceptable salt, polymorph or prodrug thereof of the present invention that (i) treats the particular disease, condition, or disorder, (ii) attenuates, ameliorates, or eliminates one or more symptoms of the particular disease, condition, or disorder, or (iii) delays the onset of one or more symptoms of the particular disease, condition, or disorder described herein. Undesirable effects, e.g. side effects, are sometimes manifested along with the desired therapeutic effect; hence, a practitioner balances the potential benefits against the potential risks in determining what is an appropriate "effective amount".

The exact amount required will vary from subject to subject, depending on the species, age and general condition of the subject, mode of administration and the like. Thus, it may not be possible to specify an exact "effective amount". However, an appropriate "effective amount" in any individual case may be determined by one of ordinary skill in the art using only routine experimentation. In one aspect, the dose administered to a subject is any dose that reduces viral load. Preferably, the dose does not significantly increase inflammation, for example does not significantly increase absolute neutrophil numbers or the proportion of neutrophils of total BAL cells in the lung. The terms "therapeutically effective amount" or "effective amount" may also refer to an amount of the compound of the invention or a pharmaceutically acceptable salt, solvate or prodrug thereof, which results in an improvement or remediation of the symptoms of a respiratory infection, or respiratory disease or condition associated with a viral or bacterial infection.

In some embodiments, an effective amount for a human subject lies in the range of about 250 nmoles/kg body weight/dose to 0.005 nmoles/kg body weight/dose. Preferably, the range is about 250 nmoles/kg body weight/dose to 0.05 nmoles/kg body weight/dose. In some embodiments, the body weight/dose range is about 250 nmoles/kg, to 0.1 nmoles/kg, about 50 nmoles/kg to 0.1 nmoles/kg, about 5 nmoles/kg to 0.1 nmol/kg, about 2.5 nmoles/kg to 0.25 nmoles/kg, or about 0.5 nmoles/kg to 0.1 nmoles/kg body weight/dose. In some embodiments, the amount is at, or about, 250 nmoles, 50 nmoles, 5 nmoles, 2.5 nmoles, 0.5 nmoles, 0.25 nmoles, 0.1 nmoles or 0.05nmoles/kg body weight/dose of the compound. Dosage regimes are adjusted to suit the exigencies of the situation and may be adjusted to produce the optimum therapeutic dose.

Compounds of the invention described herein may be compositions formulated as inhaled formulations, including dry powder, sprays, mists, or aerosols. This may be particularly preferred for treatment of a respiratory infection. For inhalation formulations, the composition or combination provided herein may be delivered via any inhalation methods known to a person skilled in the art. Such inhalation methods and devices include, but are not limited to, metered dose inhalers with propellants such as CFC or HFA or propellants that are physiologically and environmentally acceptable. Other suitable devices are breath operated inhalers, multidose dry powder inhalers and aerosol nebulizers. Aerosol formulations for use in the subject method typically include propellants, surfactants and co-solvents and may be filled into conventional aerosol containers that are closed by a suitable metering valve.

Inhalant compositions may comprise liquid or powdered compositions containing the active ingredient that are suitable for nebulization and intrabronchial use, or aerosol compositions administered via an aerosol unit dispensing metered doses. Suitable liquid compositions comprise the active ingredient in an aqueous, pharmaceutically acceptable inhalant solvent such as isotonic saline or bacteriostatic water. The solutions are administered by means of a pump or squeeze-actuated nebulized spray dispenser, or by any other conventional means for causing or enabling the requisite dosage amount of the liquid composition to be inhaled into the patient's lungs. Suitable formulations, wherein the carrier is a liquid, for administration, as for example, a nasal spray or as nasal drops, include aqueous or oily solutions of the active ingredient. Alternatively, the composition may be a dry powder and administered to the respiratory tract as defined herein.

It will be understood, that the specific dose level for any particular patient will depend upon a variety of factors including the activity of the specific compound employed, the age, body weight, general health, sex, diet, time of administration, route of administration, and rate of excretion, drug combination (i.e. other drugs being used to treat the patient), and the severity of the particular disorder undergoing therapy.

It will be understood, however, that the specific dose level for any particular subject will depend upon a variety of factors including the activity of the specific compound employed, the age, body weight, general health, sex, diet, time of administration, route of administration, and rate of excretion, drug combination (i.e. other drugs being used to treat the subject), and the severity of the particular disorder undergoing therapy. The dosage will generally be lower if the compounds are administered locally rather than systemically, and for prevention rather than for treatment. Such treatments may be administered as often as necessary and for the period of time judged necessary by the treating physician. A person skilled in the art will appreciate that the dosage regime or therapeutically effective amount of the compound of the invention, or a pharmaceutically acceptable salt, solvate or prodrug thereof, to be administered may need to be optimized for each individual. The pharmaceutical compositions may contain active ingredient in the range of about 0.1 to 2000 mg, preferably in the range of about 0.5 to 500 mg and most preferably between about 1 and 200 mg. A daily dose of about 0.01 to 100 mg/kg body weight, preferably between about 0.1 and about 50 mg/kg body weight, may be

appropriate. The daily dose can be administered in a single or multiple doses per day.

It will also be appreciated that different dosages may be required for treating different disorders.

As used herein, the terms "treatment" or "treating" of a subject includes the application or administration of a compound or composition of the invention to a subject (or application or administration of a compound of the invention to a cell or tissue from a subject) with the purpose of delaying, slowing, stabilizing, curing, healing, alleviating, relieving, altering, remedying, less worsening, ameliorating, improving, or affecting the disease or condition, the symptom of the disease or condition, or the risk of (or susceptibility to) the disease or condition. The term "treating" refers to any indication of success in the treatment or amelioration of an injury, pathology or condition, including any objective or subjective parameter such as abatement; remission; lessening of the rate of worsening; lessening severity of the disease; stabilization, diminishing of symptoms or making the injury, pathology or condition more tolerable to the subject; slowing in the rate of degeneration or decline; making the final point of degeneration less debilitating; or improving a subject's physical or mental well-being.

As used herein, "preventing" or "prevention" is intended to refer to at least the reduction of likelihood of the risk of (or susceptibility to) acquiring a disease or disorder (i.e., causing at least one of the clinical symptoms of the disease not to develop in a patient that may be exposed to or predisposed to the disease but does not yet experience or display symptoms of the disease). Biological and physiological parameters for identifying such patients are provided herein and are also well known by physicians.

"Subject" includes any human or non-human animal. Thus, in addition to being useful for human treatment, the compounds of the present invention may also be useful for veterinary treatment of mammals, including companion animals and farm animals, such as, but not limited to dogs, cats, horses, cows, sheep, and pigs.

The compounds of the present invention may be administered along with a pharmaceutical carrier, diluent or excipient as described above.

| Compound Structure | Compound ID |
|---|---|
| 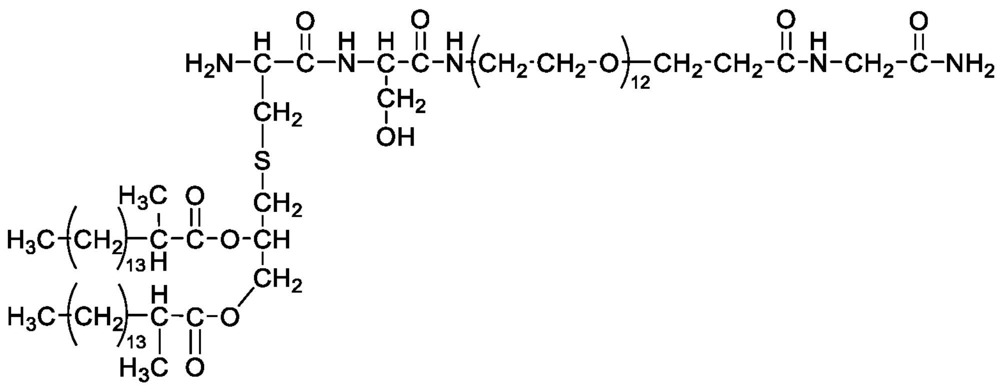 | B1 |
| 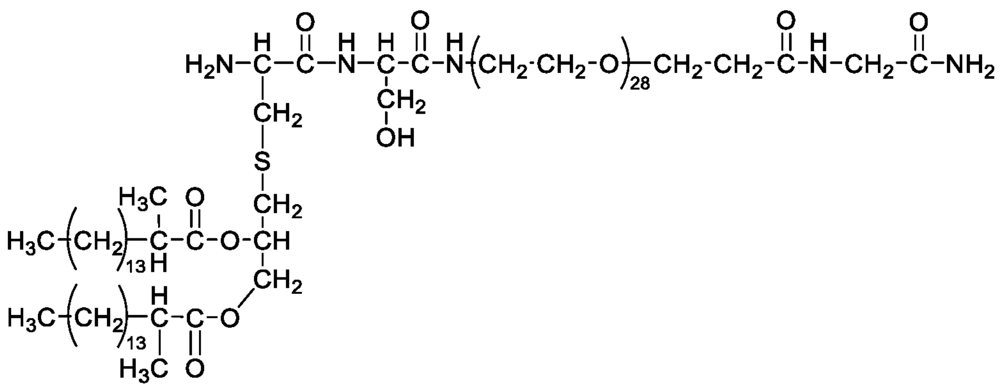 | B2 |

(continued)

| Compound Structure | Compound ID |
|---|---|
| 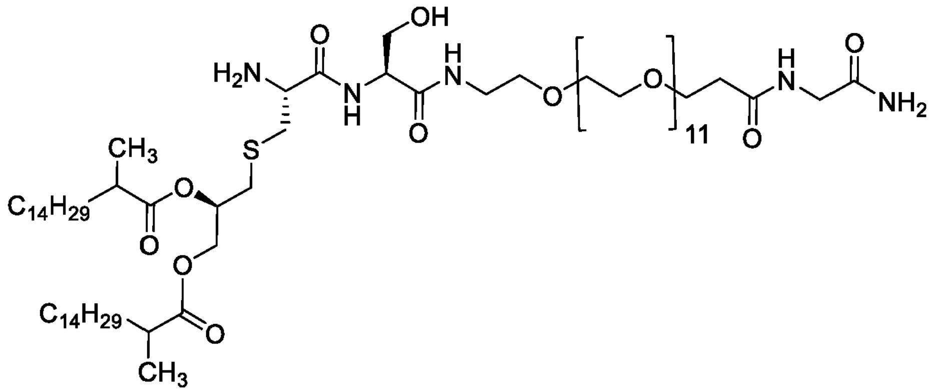 | B3 |
| 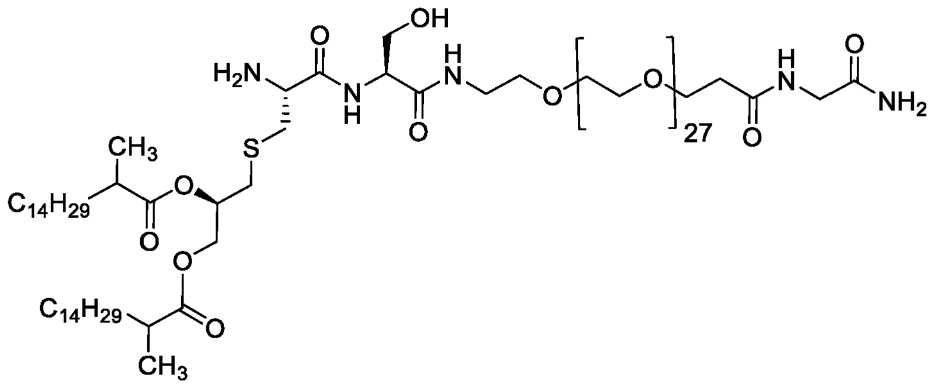 | B4 |
| 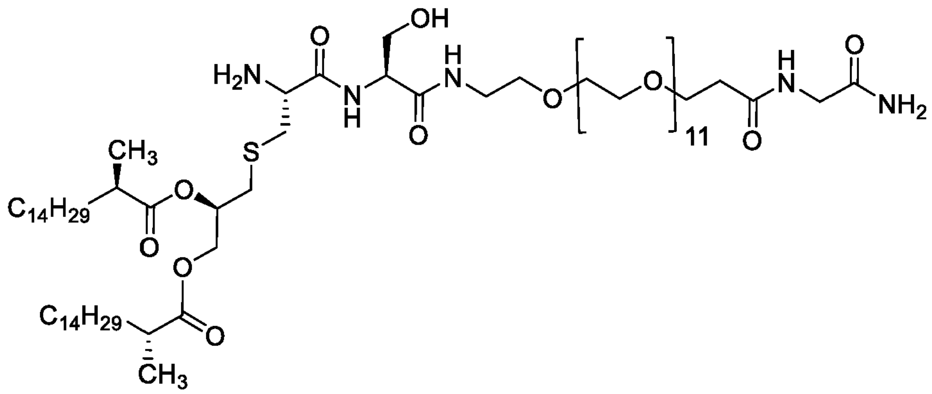 | B5 |
| 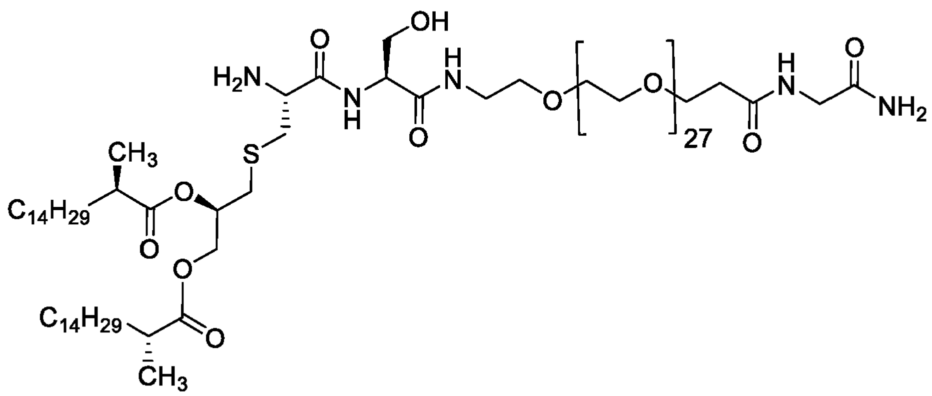 | B6 |

(continued)

| Compound Structure | Compound ID |
|---|---|
| 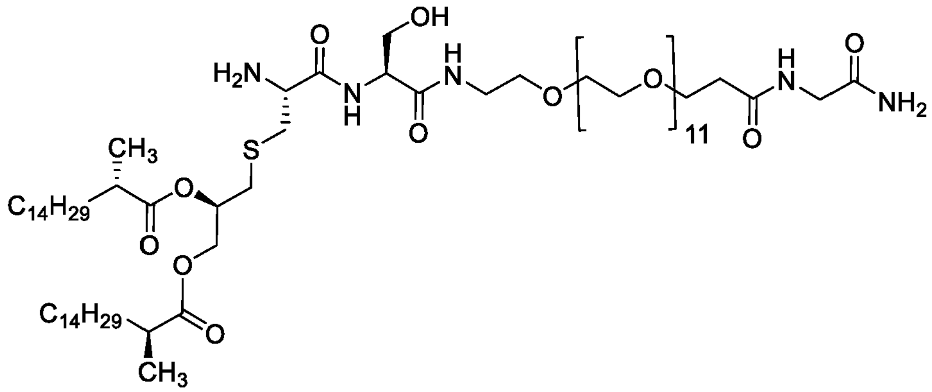 | B7 |
| 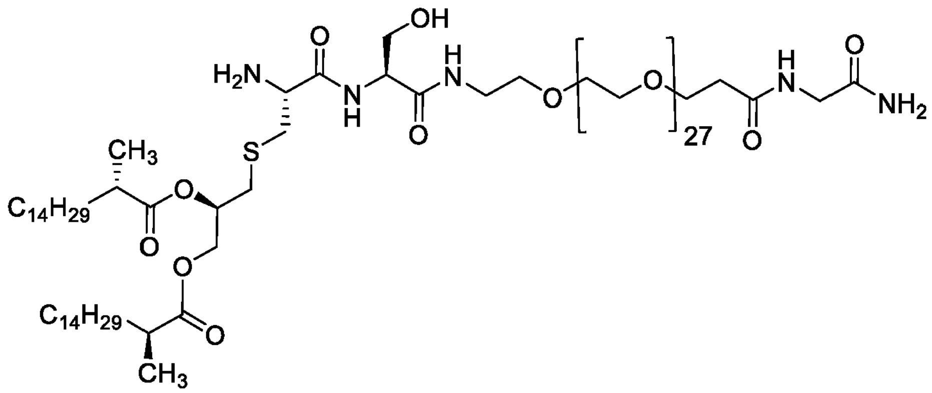 | B8 |
| 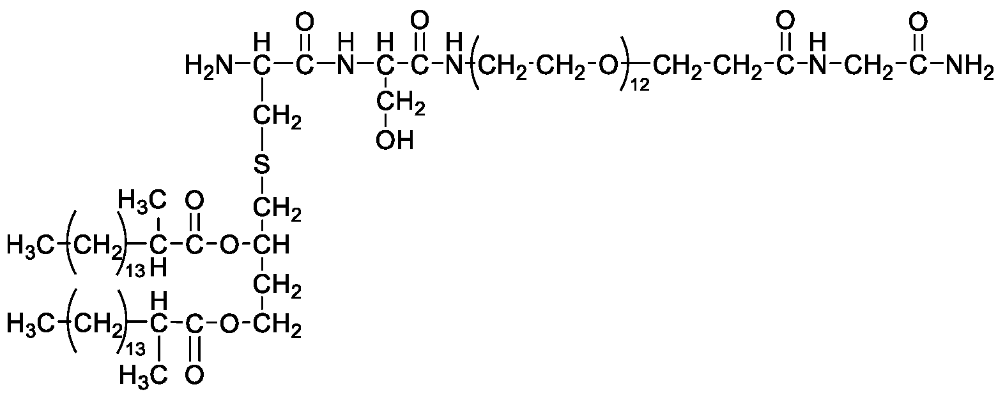 | B9 |
| 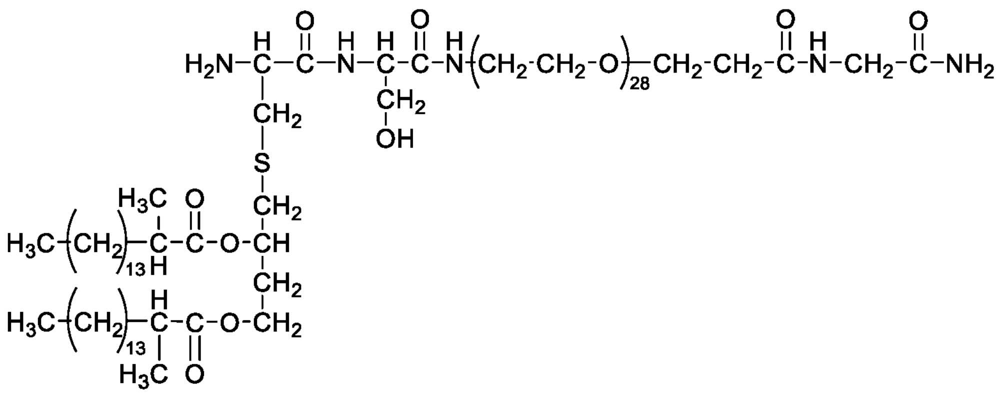 | B10 |

(continued)

| Compound Structure | Compound ID |
|---|---|
| 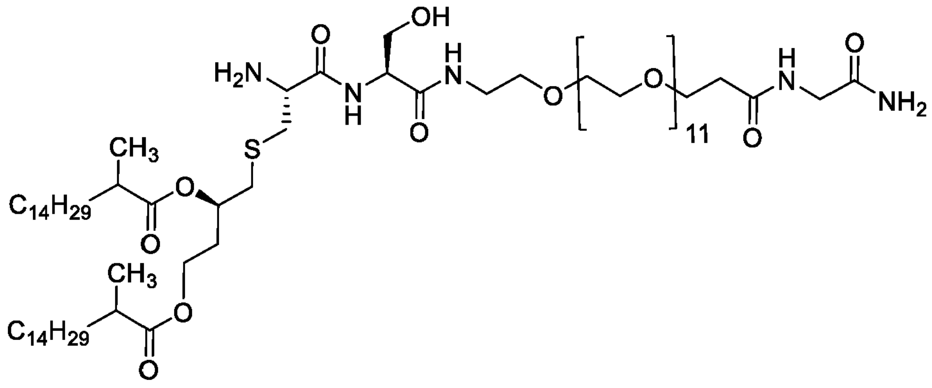 | B11 |
| 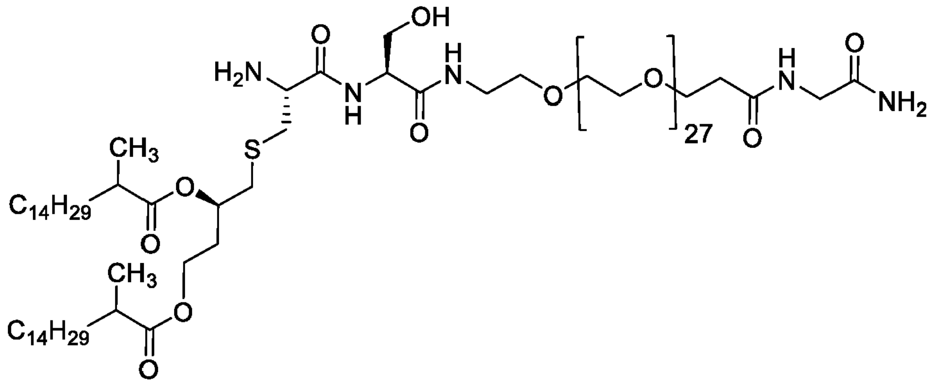 | B12 |
| 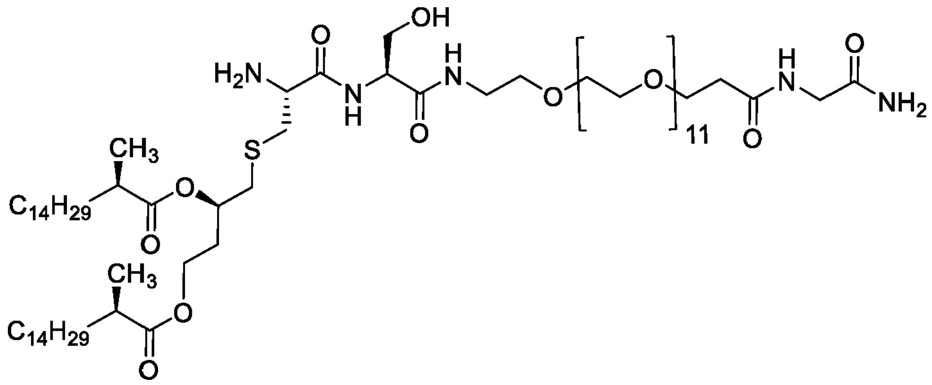 | B13 |
| 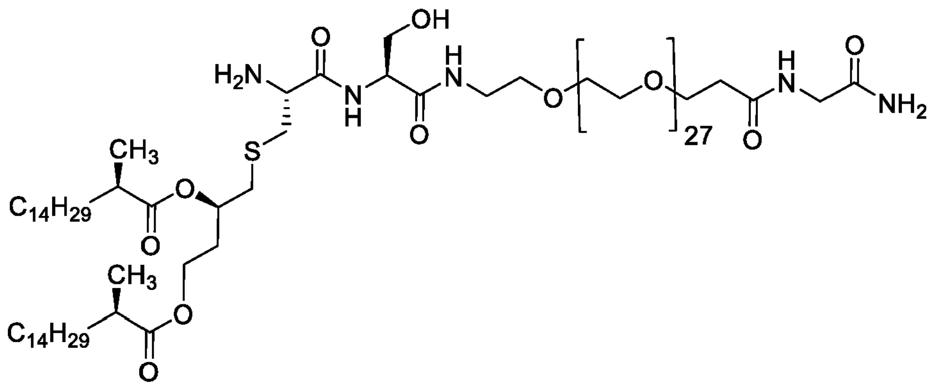 | B14 |

(continued)

| Compound Structure | Compound ID |
|---|---|
| 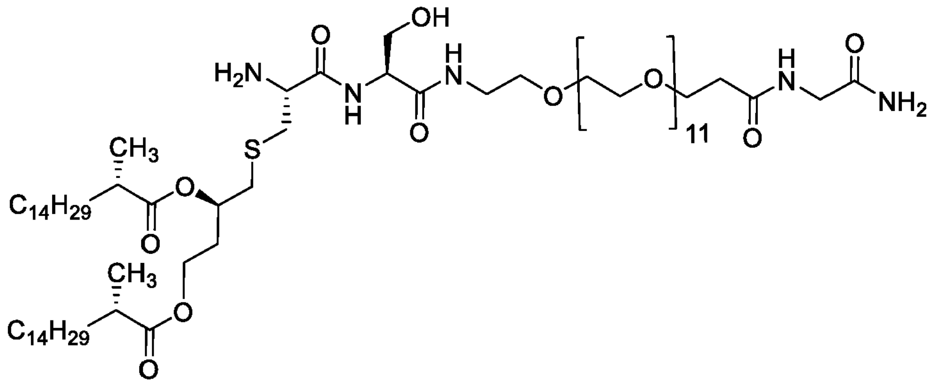 | B15 |
| 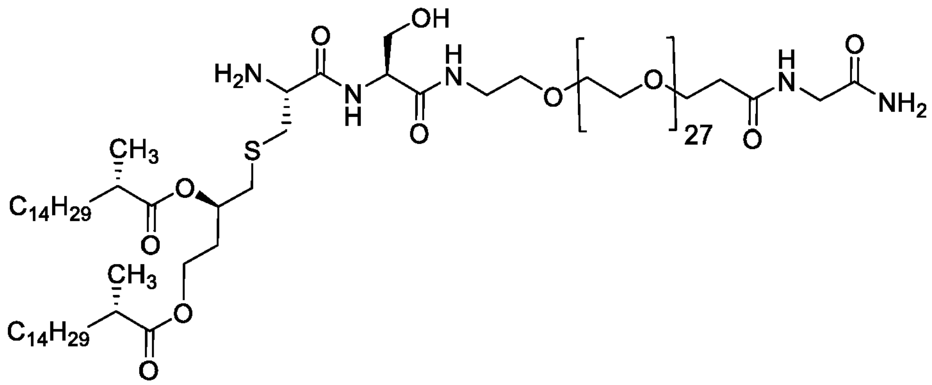 | B16 |

### Example 1 - Synthesis of compounds

Example 1.1 - synthesis A using Fmoc solid phase chemistry

Compounds of the invention, including those according to Formula (I), may be provided by coupling a compound of the formula A-I:

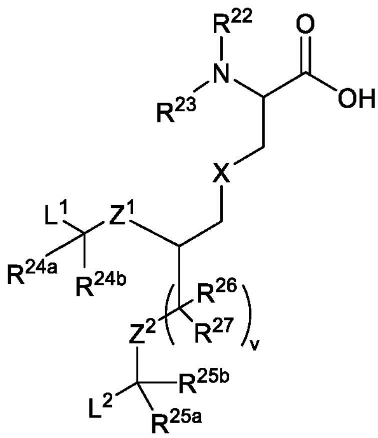

wherein $R^{21}$, $R^{22}$, $R^{24a}$, $R^{24b}$, $R^{25a}$, $R^{25b}$, $R^{26}$, $R^{27}$, X, $Z^1$, $Z^2$, v, $L^1$ and $L^2$ have the meanings as defined for any compound of the invention defined herein and $R^{22}$ is an amino protecting group

with a compound of formula YB-I:

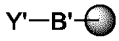

wherein

Y' is

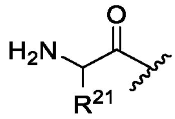

wherein $R^{21}$ is selected from the group consisting of H, $-CH_2OH$, $-CH_2CH_2OH$, $-CH(CH_3)OH$, $-CH_2OPO(OH)_2$, $-CH_2C(=O)NH_2$, $-CH_2CH_2C(=O)OH$ and $-CH_2CH_2C(=O)OR_8$, wherein any one of the alkyl hydrogens can be replaced with a halogen;

$R_8$ is selected from the group consisting of H and a straight or branched $C_1$-$C_6$ alkyl;

B' is a radical of PEG as defined for any compound of the invention; and

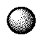

is a solid support resin.

In some embodiments, B' comprises a substituted PEG of Formula B-I. In these embodiments, the following sequence of solid phase reactions may be employed:

a) Optionally coupling 1 to 10 alpha amino acids or compounds derived from a natural alpha amino acid, that constitutes L, to a solid phase resin using Fmoc chemistry

b) Coupling PG-NH-$(CH_2)_p$-O-$(CH_2CH_2O)_n$-(CH2)$_m$-COOH to a solid phase resin or substituted resin if L is present, wherein PG represents an amino protecting group compatible with Fmoc chemistry;

c) Removing PG;

d) Coupling PG-NH-$CR_{13}R_{14}$-COOH, wherein PG' represents an amino protecting group compatible with Fmoc chemistry;

e) Removing PG';

f) Coupling an acid of the formula (A-I);

g) Optionally removing $R^{22}$ and optionally acylating and/or alkylating to introduce $R^{22}$ and/or $R^{22}$; and

h) Removing the compound from the solid phase support

In some embodiments, B' comprises a substituted PEG according to formula (B-II) and the following sequence of solid phase reactions may be employed:

a) Optionally coupling 1 to 10 alpha amino acids or compounds derived from a natural alpha amino acid, that constitute L, to a solid phase resin using Fmoc chemistry

b) Coupling PG-NH-$(CH_2)_t$-O-$(CH_2CH_2O)_k$-$(CH_2)_h$-COOH to a solid phase resin or substituted resin if L is present, wherein PG represents an amino protecting group compatible with Fmoc chemistry;

c) Removing PG;

d) Coupling PG'-NH-$(CH_2)_p$-O-$(CH_2CH_2O)_n$-$(CH2)_m$-COOH, wherein PG' represents an amino protecting group compatible with Fmoc chemistry;

e) Removing PG';

f) Coupling PG"-NH-$CR_{13}R_{14}$-COOH, wherein PG" represents an amino protecting group compatible with Fmoc chemistry;

g) Removing PG";

h) Coupling an acid of the formula (A-I);

i) Optionally removing $R^{22}$ and optionally acylating and/or alkylating to incorporate $R^{22}$ and/or $R^{22}$; and

j) Removing the compound from the solid phase resin.

It will be appreciated that the exact sequence of events can be varied from that outlined, and additional steps added where necessary and synthetically expedient, for example oxidation of the cysteine sulfur to the sulfoxide.

Example 1.2 - synthesis of intermediate for use in the solid phase coupling A

Some embodiments of the intermediate acid of formula A-II:

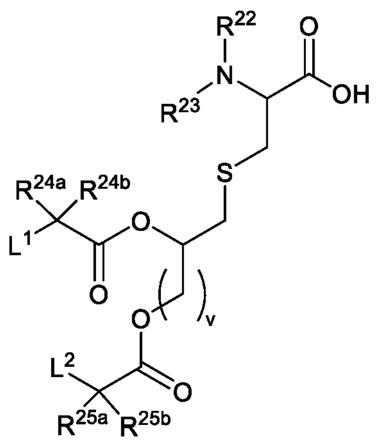

wherein $R^{22}$, $R^{23}$, $R^{24a}$, $R^{24b}$, $R^{25a}$, $R^{25b}$, $L^1$, $L^2$ and v are as defined for the compound of formula A-I above; may be prepared by the synthesis shown in Scheme 1.

**Scheme 1**

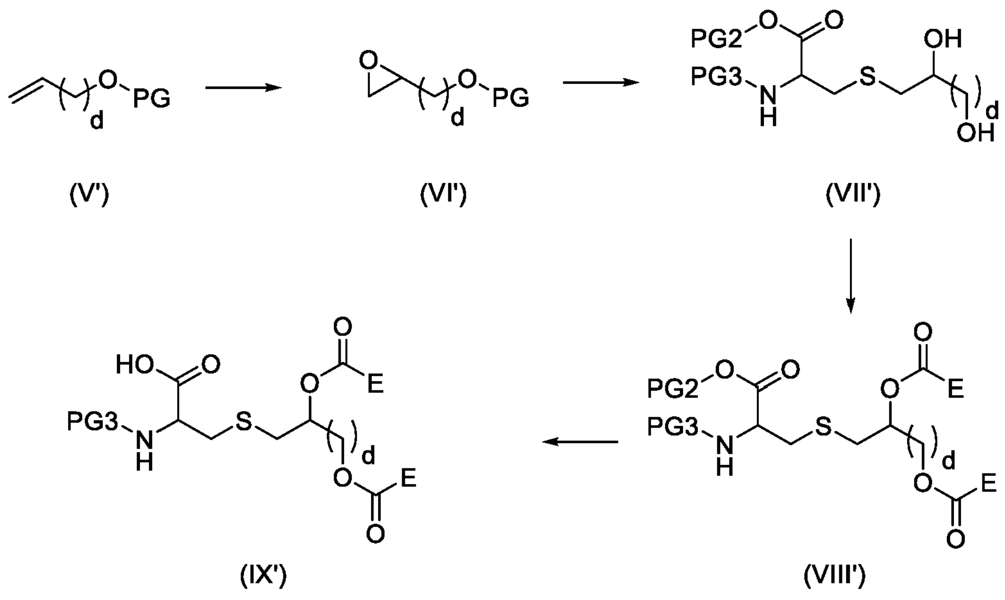

(V') (VI') (VII') (IX') (VIII')

In scheme 1, PG represents an alcohol protecting group, PG2 represents an carboxylic acid protecting group, PG3 represents an amide protecting group (and corresponds to $R^{23}$) and E represents:

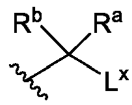

wherein $L^x$ is as defined for $L^1$ and $L^2$, $R^a$ is as defined for $R^{24a}$ and $R^{25a}$ and $R^b$ is as defined for $R^{24b}$ and $R^{25b}$.

Reaction of protected alkene alcohols of the formula (V'), where PG is a suitable protecting group, for example a silyl group such as TBDMS, forms an epoxide of the formula (VI'). It will be appreciated that the epoxide formation may be carried out to give the product racemically or to give enantioenriched material. If a racemic or scalemic mixture of enantiomers is produced preparative chiral chromatography is employed to separate the enantiomers if required.

Epoxides of the formula (VI') are reacted with suitably protected cystine analogues, for example *tert*-butyl *N*-(((9H-fluoren-9-yl)methoxy)carbonyl)-S-(((R)-2-((((9H-fluoren-9-yl)methoxy)carbonyl)amino)-3-(*tert*-butoxy)-3-oxopropyl)thio)-D-cysteinate, where PG2 is a *tert*-butyl ester and PG3 is Fmoc, under reducing conditions to give alcohols of the formula (VII'). It will be appreciated that alcohols of the formula (VII') can be comprised of more than one stereoisomer and where stereoisomers are present these can be separated by chiral preparative chromatography as required.

Alcohols of the formula (VII') can be acylated to give carbonyl containing adducts of the formula (VIII') using suitable reagents, for example with a suitably substituted acid chloride reacted in the presence of suitable bases and solvents. Carbonyl containing adducts of the formula (VIII') can then be deprotected to reveal carboxylic acids of the formula (IX') using suitable reagents, for example where PG2 is *tert*-butyl, trifluoroacetic acid can be used to preferentially remove the *tert*-butyl group.

Acids of the formula (IX') can then be used as reagents in solid phase synthesis such as those described herein.

<u>Example 1.3 - synthesis B using Fmoc solid phase chemistry</u>

Compounds of the invention, including those according to Formula (I) may be provided by preparing a resin bound peptide of the following formula:

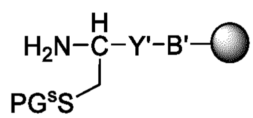

wherein

Y' is

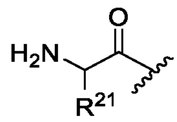

wherein $R^{21}$ is selected from the group consisting of H, $-CH_2OH$, $-CH_2CH_2OH$, $-CH(CH_3)OH$, - $CH_2OPO(OH)_2$, $-CH_2C(=O)NH_2$, $-CH_2CH_2C(=O)OH$ and $-CH_2CH_2C(=O)OR_8$, wherein any one of the alkyl hydrogens can be replaced with a halogen;

$R_8$ is selected from the group consisting of H and a straight or branched $C_1$-$C_6$ alkyl;

B' is a Polyethylene Glycol (PEG);

$PG^s$ is H or a sulphur protecting group, such as tert-butyl; and

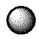

is a solid support resin.

Following optional sulphur deprotection, this resin bound peptide may be reacted with a 1,2-epoxy-alkanol of the following formula:

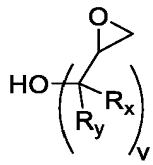

wherein $R_x$, $R_y$ and v have the meanings given for Formula (I)

to provide an alkylated thiol of formula S-1:

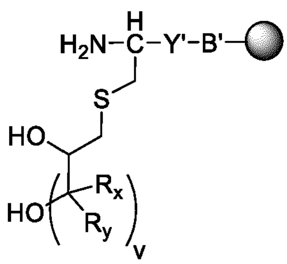

S-1

wherein Y' and B' have the meaning given above, and v has the meaning given for the compound of formula (I), or a sulfone or sulfoxide thereof.

The diol moieties of resin bound compound S-1 may be further reacted with an appropriately substituted carboxylic acid (typically in an activated form such as acid chloride, mixed anhydride, etc. or in the presence of a coupling reagent, such as diisopropylcarbodiimide (DIC), dicyclohexyl carbodiimide (DCC), Hydroxybenzotiazole (HOBt, available as Oxyma Pure), etc., optionally together with a catalyst such as 4-dimethylaminopyridine (DMAP)) to provide a compound of the invention.

Example 1.4 - Synthesis and characterisation of compounds B4, B6, B8, B12, B14 and B16

**Synthesis of compound B12.** Compound B12 was synthesized by standard Fmoc Solid Phase Peptide Synthesis, starting with Fmoc-RINK MBHA PS Resin. Removal of the Fmoc group after each coupling was achieved using 20% Piperidine in DMF. Couplings of Fmoc-Gly-OH (2-fold excess), Fmoc-NH-$PEG_{28}$-$CH_2CH_2COOH$ (1.4-fold excess), Fmoc-Ser(tBu)-OH (2-fold excess), and N-(Boc)-S-((R)-2,3-dihydroxybutyl)-L-cysteine (1.5-fold excess) were performed in diemethyl formamide (DMF) using equivalent excess of Oxyma Pure and DIC as coupling agents. 2-Methyl-Palmitic Acid coupling was performed using 2-Methyl-Palmitic Acid (20 eq. vs. moles resin), DIC (20 eq.), DMAP (2eq.) in dichloromethane (DCM)/tetrahydrofuran (THF) (85/15) (v/v) for ~20 hours at room temperature (~25°C). Optionally following each coupling step any unreacted peptide fragments are capped with an acetyl group by reaction with acetic anhydride, which may assist in purification of the compound from deletion products.

Cleavage of the peptide from the resin, removal of N-terminal Boc group, and serine side-chain deprotection were achieved by exposure of the resin to a solution of 93% TFA, 5% $H_2O$, 3% TIPS for 1.5 hours. Following the cleavage reaction, the mixture was evaporated and the resulting residue was redissolved in 30% Acetonitrile/ Water and lyophilized.

***Scheme 2 – synthesis of compounds B4, B6, B8, B14 and B16***

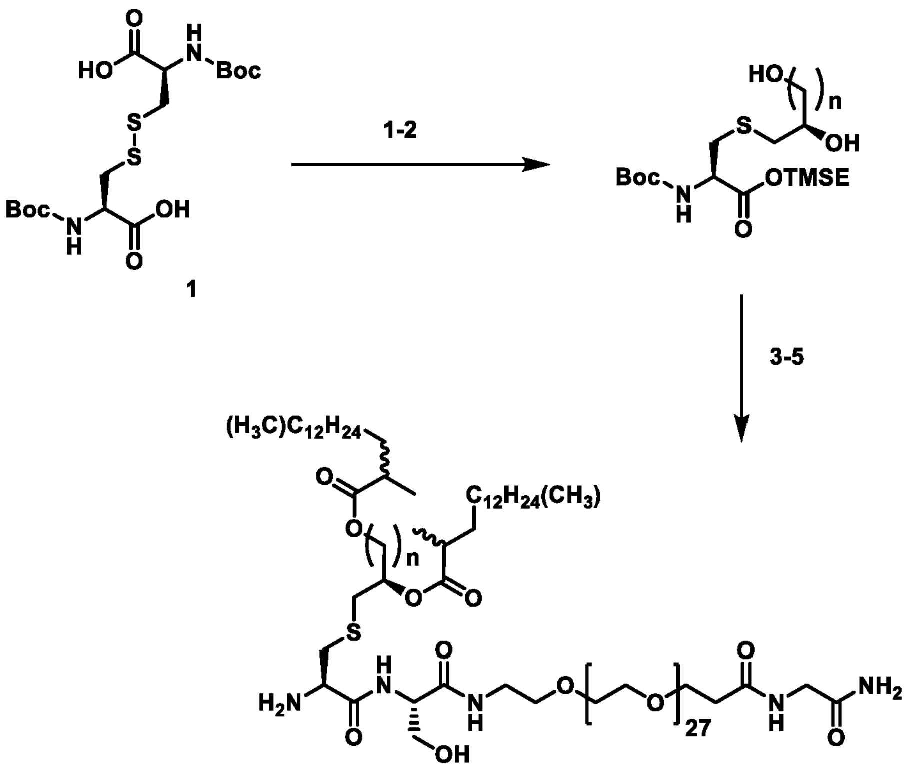


1

**2 n = 1**
**3 n = 1**

**4 n = 1, B6 (*R,R*)**
**5 n = 1, B8 (*S,S*)**
**6 n = 2, B14 (*R,R*)**
**7 n = 2, B16 (*S,S*)**
**8 n = 1, B4 (*rac*)**

**1.** ACN, DMF, DIC, TMSE-OH, pyridine. **2.** DCM, Zn dust, MeOH, HCl, $H_2SO_4$, epoxide. **3.** 2-Methylpalmitic acid, DIC, DMAP, THF. **4.** 1M TBAF THF. **5.** (i) Rink-Gly-$PEG_{28}$-Ser(OtBu)-$NH_2$, PyBOP, collidine, DCM (ii) TFA

Procedures for synthetic steps of Scheme 2

Synthesis of Rink-Gly-PEG28-Ser(OtBu)-NH2

1. p-[(R,S)-α-[1-(9H-Fluoren-9-yl)-methoxyformamido]- 2,4-dimethoxybenzyl]- phenoxyacetic (Fmoc-Rink) amide AM resin 0.47meq/g, 5g2.35mmol). The resin was swollen in dimethyl formamide (DMF; 30 mL) for 15 minutes (min) and then the solvent filtered off.

2. The resin was then treated with 20mL of 20% piperidine/DMF twice (1 X 5min and then 1 X 10min)

3. The resin was washed with DMF X 2 and then DCM X 2. Bromophenol blue (BPB) test positive

4. Fmoc-Gly-OH (3equivalents (eq), 7.05 mmol, 2.1g) in DMF 15mL was added PyBOP (7.05 mmol, 3.67g) and then diisopropylethyl amine (DIPEA; 4eq, 9.4 mmol, 1.64 mL) and mixed and left to stand for 5-10min.

5. The mixture was added to the resin and the resin/mixture was shaken for 2 hours (h). After 2h BPB test was negative.

6. The resin was filtered and then washed with DMF X 2 and then dichloromethane (DCM) X 2 and finally DMF.

7. The resin was then treated with 20mL of 20% piperidine/DMF twice (1 X 5min and then 1 X 10min)

8. The resin was washed with DMF X 2 and then DCM X 2. BPB test positive

9. 200mg of the NH2-Gly-Rink-resin (assume 0.47meq/g, 0.094mmol) was swollen in DCM, then filtered and washed several times with DCM.

10. Fmoc-NH-PEG28-(CH2)2-CO2H (MW = 1544.75, 1.5eq, 0.141 mmol 145mg) was taken up in 4mL DCM and PyBOP (1.6eq, 0.150mmol 78mg) was added followed by DIPEA (4eq, 0.376mmol 0.065mL) and stirred for several minutes then added to the resin, which was shaken overnight.

11. BPB test was negative. The resin was washed with DMF X 2 and then DCM X 2 and finally DMF.

12. The resin was then treated with 1mL of 20% piperidine/DMF twice (1 X 5min and then 1 X 10min).

13. The resin was washed with DMF X 2 and then DCM X 2. BPB test positive

14. Fmoc-Ser(OtBu)OH (MW = 383.4, 1.5eq, 0.141mmol 54mg) was taken up in 4mL DMF and PyBOP (1.6eq, 0.150mmol 78mg) was added followed by DIPEA (4eq, 0.376mmol 0.065mL) and stirred for several minutes then added to the resin, which was shaken overnight.

15. BPB test was negative. The resin was washed with DMF X 2 and then DCM X 2 and finally DMF.

16. The resin was then treated with 1mL of 20% piperidine/DMF twice (1 X 5min and then 1 X 10min).

17. The resin was washed with DMF X 2 and then DCM X 2. BPB test positive.

**Step 1**

N'N''-Bis-Boc-L-cystine 1 (6.61g, 15 mmol) was dissolved in ACN (30mL) and DMF (11.25mL) in a two neck 100mL flask in an $N_2$ atm and chilled on an ice bath. To this mixture was added 2-(trimethylsilyl)ethanol (5.11mL, 35.63mmol) and pyridine (4.80mL, 59.33mmol) and left to stir on an ice bath for ten minutes then DCC (6.75g, 32.70mmol) was added and stirred on an ice bath for 16h without recharging the ice bath. To the mixture was added solid citric acid and stirred a further 1-2h. The mixture was diluted with ether and filtered through a silica plug. The organic layer was washed with 5% citric acid, water, bicarbonate then brine, dried and concentrated to a clear resin that was used without further purification 10.3g 1H NMR (401 MHz, CDCl3) δ 5.38 (d, J = 7.1 Hz, 1H), 4.56 (d, J = 7.1 Hz, 1H), 4.33 - 4.16 (m, 3H), 3.16 (s, 2H), 1.46 (d, J = 5.2 Hz, 14H), 1.03 (dd, J = 9.0, 8.4 Hz, 3H), 0.11 - 0.02 (m, 13H).

**Step 2**

The product of step 1 (10.3g, 15mmol based on intermediate 1 reaction) was taken up in DCM (80mL) and Zn dust (8.12g, 124.15mmol) was added. The mixture was cooled on an ice bath. To the mixture was added freshly prepared methanol (MeOH):cHCl:c$H_2SO_4$ (100:7:1) (32mL) and stirred on ice for 0.5h and then the ice bath was removed, (*R*)-(+)-oxirane-2-methanol added and stirred at 40 °C overnight. The mixture was cooled to room temperature (rt), diluted with dichloromethane (DCM) and filtered through celite. The organic phase was washed with water and then brine. The combined aqueous phases were back extracted with diethyl ether. The combined organics were dried, filtered and concentrated to provide intermediate compound **2** as a clear colourless resin 11.52g 98% yield and used without further

purification.

Intermediate compound **3** was prepared by a similar route to that followed for the preparation of intermediate compound **2**, except (*R*)-(+)-Oxirane-2-ethanol was used instead of (*R*)-(+)-Oxirane-2-methanol). $^{1}$H NMR (400 MHz, $CDCl_3$) δ 4.62 - 4.36 (m, 1H), 4.32 - 4.18 (m, 2H), 3.97 - 3.79 (m, 3H), 3.02 (dt, J = 18.4, 9.2 Hz, 1H), 2.94 - 2.70 (m, 2H), 2.65 - 2.46 (m, 2H), 1.85 - 1.66 (m, 2H), 1.45 (s, 9H), 1.12 - 0.95 (m, 2H), 0.07 - 0.02 (m, 9H).

**Steps 3-5**

**Synthesis of compound B6 (*R, R*).** A portion of intermediate compound **2** (100mg, 0.253mmol) was taken up in anhydrous THF (1.5mL) in an $N_2$ atmosphere and to this mixture was added (*R*)-2-methylpalmitic acid (205mg, 0.758mmol), N-dimethylaminopyridine (DMAP; 12mg, 0.101mmol) and finally diisopropyl carbodiimide (DIC; 118μL, 0.758mmol) at rt in an $N_2$ atmosphere and stirred at rt overnight. Then diluted in ether and filtered. The organic layer was washed with 1M HCl, water, bicarbonate, water then brine, dried ($MgSO_4$), filtered and concentrated to a residue that solidifies to a waxy solid. The crude material was taken up in 1M tert-butylammonium fluroride complex with tetrahydrofuran (TBAF THF; 1.4mL) and stirred at rt for 3h. The mixture was diluted in ether and washed with 1M HCl, then water X 4 and finally brine. The organic layer was dried ($MgSO_4$), filtered and concentrated to a residue (89mg). The crude material was taken up in DCM (1mL) and benzotriazol-1-yl-oxytripyrrolidinophosphonium hexafluorophosphate (PyBOP) added (65mg), followed by collidine (27μL) stirred for 1min, then added to Rink-Gly-PEG28-Ser(OtBu)-NH2 resin (100mg) in DCM (2 mL). After 2h BPB test was negative. The resin was thoroughly washed with DMF X 2 and then DCM X4, MeOH and then diethyl ether X 4. Then left under high vacuum overnight. The resin was treated with 95% trifluoroacetic acid (TFA) 5% tiisopropylsilane (TIPS) for 2h. Then filtered and TFA removed under $N_2$ flow. The remaining residue was taken up in water and freeze dried. The lyophilised material was purified on by high-performance liquid chromatography (HPLC) isocratic flow 80:20 A:B (A = 50:50 acetonitrile (ACN):MeOH, B = 1% A in water) to provide after lyophilisation 35.2mg of amorphous solid 6.5% yield. LCMS $R_f$ (min) = 6.73. MS m/z 1074.4.0 (M + 2H)/2, 722.4 (M + 3H + H2O)/3. HR-ESI calcd for $C_{104}H_{203}N_5O_{37}S$ (M + 2H)/2, 1074.7028; found, 1074.7030.

**Synthesis of compound B8 (S, S).** A portion of intermediate compound **2** (100mg, 0.253mmol) was taken up in anhydrous THF (1.5mL) in an $N_2$ atmosphere and to this mixture was added (S)-2-methylpalmitic (205mg, 0.758mmol), DMAP (12mg, 0.101 mmol) and finally DIC (118μL, 0.758mmol) at rt in an $N_2$ atmosphere and stirred at rt overnight. Then diluted in diethyl ether and filtered. The organic layer was washed with 1M HCl, water, sodium bicarbonate (aq.), water then brine, dried ($MgSO_4$), filtered and concentrated to a residue that solidifies to a waxy solid. The crude material was taken up in 1M TBAF THF (1.5mL) and stirred at rt for 3h. The mixture was diluted in diethyl ether and washed with 1M HCl, then water X 4 and finally brine. The organic layer was dried ($MgSO_4$), filtered and concentrated to a residue 104mg. The crude material was taken up in DCM (1mL) and PyBOP added (76mg), followed by collidine (32μL) stirred for 1min, then added to Rink-Gly-PEG28-Ser(OtBu)-NH2 resin (117mg) in DCM (2 mL). After 2h BPB test was negative. The resin was thoroughly washed with DMF X 2 and then DCM X4, MeOH and then diethyl ether X 4. Then left under high vacuum overnight. The resin was treated with 95% TFA 5%TIPS for 2h. Then filtered and TFA removed under $N_2$ flow. The remaining residue was taken up in water and freeze dried. The lyophilised material was purified on by HPLC isocratic flow 80:20 A:B (A = 50:50 ACN:MeOH, B = 1% A in water) to provide after lyophilisation 44.2mg of amorphous solid 8.1% yield. LCMS $R_f$ (min) = 6.64. MS m/z 1074.4.0 (M + 2H)/2, 722.4 (M + 3H + H2O)/3. HR-ESI calcd for $C_{104}H_{203}N_5O_{37}S$ (M + 2H)/2, 1074.7028; found, 1074.7038.

**Synthesis of compound B14 (*R, R*).** A portion of intermediate compound **3** (104mg, 0.253mmol) was taken in anhydrous THF (1.5mL) in an $N_2$ atmosphere and to this mixture was added (*R*)-2-methylpalmitic (205mg, 0.758mmol), DMAP (12mg, 0.101 mmol) and finally DIC (118μL, 0.758mmol) at rt in an $N_2$ atmosphere and stirred at rt overnight. Then diluted in diethyl ether and filtered. The organic layer was washed with 1M HCl, water, sodium bicarbonate (aq.), water then brine, dried ($MgSO_4$), filtered and concentrated to a residue that solidifies to a waxy solid. The crude material was taken up in 1M TBAF THF (1.4mL) and stirred at rt for 3h. The mixture was diluted in diethyl ether and washed with 1M HCl, then water X 4 and finally brine. The organic layer was dried ($MgSO_4$), filtered and concentrated to a residue (150mg). The crude material was taken up in DCM (1mL) and PyBOP added (110mg), followed by collidine (46μL) stirred for 1min, then added to Rink-Gly-PEG28-Ser(OtBu)-$NH_2$ resin (168mg) in DCM (2 mL). After 2h BPB test was negative. The resin was thoroughly washed with DMF X 2 and then DCM X4, MeOH and then diethyl ether X 4. Then left under high vacuum overnight. The resin was treated with 95% TFA 5%TIPS for 2h. Then filtered and TFA removed under $N_2$ flow. The remaining residue was taken up in water and freeze dried. The lyophilised material was purified on by HPLC isocratic flow 80:20 A:B (A = 50:50 ACN:MeOH, B = 1% A in water) to provide after lyophilisation 70mg of amorphous solid 12.8% yield. LCMS $R_f$ (min) = 6.25. MS m/z 1081.7 (M + 2H)/2, 727.1 (M + 3H + H2O)/3. HR-ESI calcd for $C_{105}H_{205}N_5O_{37}S$ (M + 2H)/2, 1081.7107; found, 1081.7108.

**Synthesis of compound B16 (*S, S*).** A portion of intermediate compound **3** (104mg, 0.253mmol) was taken in anhydrous THF (1.5mL) in an $N_2$ atmosphere and to this mixture was added (S)-2-methylpalmitic (205mg, 0.758mmol), DMAP (12mg, 0.101 mmol) and finally DIC (118μL, 0.758mmol) at rt in an $N_2$ atmosphere and stirred at rt overnight. Then

diluted in ether and filtered. The organic layer was washed with 1M HCl, water, sodium bicarbonate (aq.), water then brine, dried ($MgSO_4$), filtered and concentrated to a residue that solidifies to a waxy solid. The crude material was taken up in 1M TBAF THF (1.4mL) and stirred at rt for 3h. The mixture was diluted in diethyl ether and washed with 1M HCl, then water X 4 and finally brine. The organic layer was dried ($MgSO_4$), filtered and concentrated to a residue (77mg). The crude material was taken up in DCM (1mL) and PyBOP added (56mg), followed by collidine (23$\mu$L) stirred for 1min, then added to Rink-Gly-PEG28-Ser(OtBu)-$NH_2$ resin (87mg) in DCM (2 mL). After 2h BPB test was negative. The resin was thoroughly washed with DMF X 2 and then DCM X4, MeOH and then diethyl ether X 4. Then left under high vacuum overnight. The resin was treated with 95% TFA 5%TIPS for 2h. Then filtered and TFA removed under $N_2$ flow. The remaining residue was taken up in water and freeze dried. The lyophilised material was purified on by HPLC isocratic flow 80:20 A:B (A = 50:50 ACN:MeOH, B = 1% A in water) to provide after lyophilisation 47.7mg of amorphous solid 8.7% yield. LCMS $R_f$(min) = 6.35. MS m/z 1081.7 (M + 2H)/2, 727.1 (M + 3H + H2O)/3. HR-ESI calcd for $C_{105}H_{205}N_5O_{37}S$ (M + 2H)/2, 1081.7107; found, 1081.7132.

**Synthesis of compound B4.** A portion of intermediate compound 2 (100mg, 0.253mmol) shown in scheme 2 was taken in anhydrous THF (1.5mL) in an $N_2$ atmosphere and to this mixture was added 2-methylpalmitic acid (205mg, 0.758mmol), DMAP (12mg, 0.101 mmol) and finally DIC (118$\mu$L, 0.758mmol) at rt in an $N_2$ atm and stirred at rt overnight. Then diluted in ether and filtered. The organic layer was washed with 1M HCl, water, bicarbonate, water then brine, dried ($MgSO_4$), filtered and concentrated to a residue that solidifies to a waxy solid. The crude material was taken up in 1M TBAF THF (1.5mL) and stirred at rt for 3hr. The mixture was diluted in ether and washed with 1M HCl, then water X 4 and finally brine. The organic layer was dried ($MgSO_4$), filtered and concentrated to a residue (75mg). The crude material was taken up in DCM (1mL) and PyBOP added (55mg), followed by collidine (23$\mu$L) stirred for 1min, then added to Rink-Gly-PEG28-Ser(OtBu)-NH2 resin (84mg) in DCM (2 mL). After 2h BPB test was negative. The resin was thoroughly washed with DMF X 2 and then DCM X4, MeOH and then Ether X 4. Then left under high vac overnight. The resin was treated with 95% TFA 5%TIPS for 2h. Then filtered and TFA removed under N2 flow. The remaining residue was taken up in water and freeze dried. The lyophilised material was purified on by HPLC isocratic flow 80:20 A:B (A = 50:50 ACN:MeOH, B = 1% A in water) to provide after lyophilisation 9.2mg of amorphous solid 1.7% yield. HR-ESI calc'd for $C_{104}H_{203}N_5O_{37}S$ (M + 2H)/2, 1074.7028; found, 1074.7058.

### Purification and characterisation

*Purification and characterisation:* Following cleavage from the solid support, each of the analogs were purified by reversed-phase HPLC conducted using a Novasep Axial Compression Column (5-cm diameter) loaded with cyano media (Daisogel SP-120-CN-P), with a gradient of Acetonitrile in [0.1%TFA/Water]. Following intermediate lyophilization, ion-exchange was performed on Dowex ion-exchange resin in order to obtain the peptide as the acetate salt.

Identification and purity determination of the target materials were carried out using an in-line analytical reverse phase HPLC with a cyano column (Daiso Fine Chem, SP-120-3-CN-P, 150 x 4.6 mm, 3$\mu$m, 120Å). The peptide was also analyzed by ESI LC-MS in Positive Ion Mode, using a Finnigan LCQ Deca XPMax.

Compounds B4, B6, B8, B12, B14 and B16 prepared and purified as described above, were found to be greater than 95% pure.

Experimental masses (*m*/*z*) accorded with calculated molecular weights for each compound.

### Peptide quantitation

Quantitation of compounds was carried out by *in vacuo* hydrolysis at 110°C of samples in sealed glass vials in the presence of 6N HCl containing 0.1% phenol. Derivatisation of amino acids was then carried out using Waters AccQTag reagents according to the manufacturer's instructions followed by analysis on a Waters Acquity UPLC System (Waters Millipore) using an AccQTag ultra column (2.1mm x 100mm; Waters Millipore).

1.5 - Synthesis of sulfone and sulfoxide analogues of compounds

Sulfone and sulfoxide derivatives of compounds of this invention may be accessed by a similar synthetic routes as described above, with the omission of ethylmethylsulfide scavenger, and optional omission of nitrogen sparging, from the carbamate formation step. This reaction may yield a mixture of thiol, sulfone and sulfoxide derivatives, which may be separated and purified by HPLC.

Alternatively, sulfone or sulfoxide derivatives may be prepared by oxidation of the corresponding sulfide with an oxidant such as meta-chloroperoxybenzoic acid (MCPBA) or *tert*-butyl hydroperoxide (t-BuOOH) under appropriate conditions.

## Example 2 - Activation of human TLR2

The potency of the compounds as activators of human and mouse TLR-2s is tested in an *in vitro* assay. The assay assesses NF-kB activation in the HEKBlue-mTLR-2 cell line. These cells have been stably transfected with mouse TLR-2 and express TLR-1 and TLR-6 endogenously at sufficient levels to allow for fully-functional TLR-1/2 and TLR-2/6 activation.

Toll-Like Receptor 2 (TLR2) stimulation is tested by assessing NF-kB activation in the HEKBlue-hTLR2 cell line. These cells have been stably transfected with human TLR2 and express TLR1 and TLR6 endogenously at a level sufficient to allow for fully-functional TLR1/2 and TLR2/6 activation. The activity of the test articles are tested on human TLR2 as potential agonists. The test articles are evaluated at seven concentrations and compared to control ligands. These steps are performed in triplicate.

*NF-kB reporter gene assay protocol*: This assay is carried out as described previously (Jackson et al. 2004; Lau et al. 2006; Sandor et al. 2003; Zeng et al 2010). HEK293T cells were cultured in 96-well plates at 4 x $10^4$ cells/well and transfected 24 h later with 100ng of the NF-kB luciferase reporter gene [50ng of TK-Renilla-luciferase expressing plasmid (Promega corporation, Madison, USA)] with or without 5ng TLR2-expressing plasmid in the presence of 0.8μl Fugene 6 (Roche Diagnostic). Compounds are added to the wells 24h later at the concentrations indicated in the histograms. Cell lysates are prepared 5h after stimulation using reporter lysis buffer (Promega Corporation, Madison, USA). Luciferase activities in the cell lysates were determined using a reagent kit (Promega Corporation, Madison, USA) and using a FLUOstar microplate reader (BMG Labtech, Ortenberg, Germany). The NF-kB-dependent firefly luciferase activity is normalised with NF-kB-independent renilla luciferase activity. The relative stimulation is calculated as the ratio of the stimulated to non-stimulated samples.

## Example 3 - URT virus challenge

In these Examples, an upper respiratory tract (URT) influenza virus challenge model is utilised in mice, using a dose of infectious virus which replicates in the URT and then progress to the lungs. The URT model is used to determine which compounds can prevent replication and dissemination of influenza virus from the URT to the lungs.

Cytokine and chemokine profiles in the nasal turbinates, trachea, lungs and sera of animals following URT treatment with three doses or a single dose of the compounds are also measured.

The cytokine profiles of mice which were pre-treated with three doses of compounds of the invention followed by challenge with Udorn virus are also measured.

### Experimental animals

Groups of male or female C57BL/6 mice of similar age (e.g. about 6-8 week old) are used for all studies. After administration of saline, the compound or viral challenge, mice are monitored daily for weight changes, and behavioural or physical changes.

### URT administration of compounds

Mice are anaesthetized by isoflurane inhalation and saline or various doses of the compounds, diluted in saline, are administered intranasally using a pipettor. For the multi-treatment experiments, mice receive 3 doses of the compounds of the invention every second day over a 5 day period.

### Preparation of influenza virus

A/Udorn/307/72 (H3N2) influenza virus (ie. Udorn virus) is propagated in the allantoic cavity of 10 day-old embryonated hens' eggs. Eggs are inoculated with approximately $10^3$ pfu of virus in 0.1ml of saline. After 2 days incubation at 35°C the eggs are chilled at 4°C and allantoic fluid harvested and clarified by centrifugation. Viral infectivity titre (pfu/mL) is determined by plaque assay as described below and aliquots of the allantoic fluid were stored at -80°C until used.

### URT virus challenge

Mice are anaesthetised with isofluorane and inoculated intranasally with 500 pfu of *Udorn* virus in 10μl of saline, using a pipettor. On day 5 post-challenge, the nasal turbinates, trachea and lung are harvested to assess viral loads.

**Extraction and preparation of nasal turbinates, trachea and lung homogenates**

Mice are killed by $CO_2$ asphyxiation 24 hours post-treatment or 5 days post-influenza challenge. Nasal turbinates, trachea and lungs from each mouse are collected in 1.5mL of RPMI-1640 medium with antibiotics (100ug/mL penicillin, 180ug/mL streptomycin and 24ug/mL gentamicin) and kept on ice until processed. Tissues were homogenised using a tissue homogeniser and the resulting organ homogenates then centrifuged at 2,000rpm for 5 min to remove cell debris. Supernatants are collected and stored at -80°C for subsequent measurements.

**Assessment of viral titres**

Titres of infectious *Udorn* virus are determined by plaque assay on confluent monolayers of Madin Darby canine kidney (MDCK) cells. Six-well tissue culture plates were seeded with $1.2x10^6$ MDCK cells per well in 3 ml of RP10 (RPMI-1640 medium supplemented with 10% (v/v) heat inactivated FCS, 260ug/mL glutamine, 200ug/mL sodium pyruvate and antibiotics). After overnight incubation at 37°C in 5% $CO_2$ confluent monolayers were washed with RPMI. Test supernatants serially diluted in RPMI with antibiotics, are added to duplicate wells of monolayers. After incubation at 37°C in 5% $CO_2$ for 45 min, monolayers are overlaid with 3mL of agarose overlay medium containing 0.9% agarose and 2ug/mL trypsin-TPCK treated in Leibovitz L15 medium pH6.8 with glutamine and antibiotics. Plates are incubated for 3 days at 37°C in 5% $CO_2$ and virus-mediated cell lysis then counted as plaques on the cell layer. The total organ viral titres (plaque forming units, PFU) for individual animals are calculated.

**Determination of cytokine levels in nasal turbinates, trachea, lungs and sera**

IFN-$\gamma$, IL-2, IL-4, TNF, IL-10, IL-6, KC, MCP-1, RANTES, IL-12/IL-23p40 and IL-17A present in nasal turbinates, trachea, lung homogenates and serum samples were measured using a BD Cytometric Bead Array (CBA) Flex Kit according to the manufacturer's instructions with the exception that a total of 0.15$\mu$l of each capture bead suspension and 0.15$\mu$l of each PE-detection reagent is used in each 50$\mu$l sample. Samples were analysed using a Bection Dickinson FACSCanto II flow cytometer and the data analysed using FCAP Array multiplex software.

**Statistical analyses**

A one-way analysis of variance (ANOVA) with Tukey comparison of all column tests may be used. A two-way ANOVA with Bonferroni's test may be used to compare the same treatment groups in the single and 3 repeat dose regimes. A *p*-value $\leq$ 0.0322 was considered statistically significant. Statistical analyses are performed using suitable software, such as GraphPad Prism, version 7.0.

**Example 4** - **Assessing the effect of pre-treatment with different doses of compounds of the invention on the outcome of URT challenge with Udorn virus**

This experiment is performed to determine the anti-viral effect of URT pre-treatment with various doses of the compounds of the invention.

On day 0 mice (5 animals/group) receive either saline, 5nmoles, 0.1nmoles or 0.005nmoles of compound of the invention, administered intranasally in 10$\mu$l after being anaesthetized with isoflurane. On day 1 following administration with compound of the invention, mice are challenged intranasally with 500 pfu of Udorn virus in a volume of 10$\mu$l after being anaesthetized with isoflurane. Mice are killed on day 5 and nasal turbinates trachea and lungs were removed, homogenised and frozen for subsequent analyses.

The experimental design is summarised in the schematic below

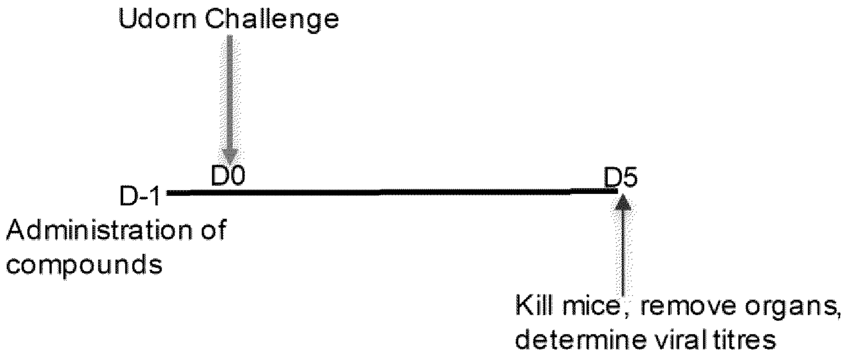


**Example 5** - **TLR2 activation by various compounds**

Comparison of the abilities of various compounds to stimulate luciferase activity in an NF-κB cell-based reporter system is determined. HEK293T cells, transiently co-transfected with a human TLR2 plasmid and a luciferase-NF-κB plasmid reporter system, are exposed to various dilutions of each compound. Successful receptor binding and subsequent signal transduction events are determined by measuring the luminescence due to luciferase activity

**Example 6** - **TLR binding and specificity**

The compound of the invention is assessed for its ability to activate a range of other TLR pattern recognition receptors. These assessments are conducted using both human and mouse TLR panels. These assays detect a secreted embryonic alkaline phosphatase (SEAP) reporter under the control of a promoter which is inducible by NF-κB activation in HEK293 cells.

The secreted embryonic alkaline phosphatase (SEAP) reporter is under the control of a promoter inducible by the transcription factor NF-κB. This reporter gene allows the monitoring of signaling through the TLR, based on the activation of NF-κB. In a 96-well plate (200 μL total volume) containing the appropriate cells (50,000 - 75,000 cells/well), 20 μL of the test article or the positive control ligand is added to the wells. The media added to the wells is designed for the detection of NF-κB induced SEAP expression. After a 16-24 hr incubation the optical density (OD) is read at 650 nm on a Molecular Devices SpectraMax 340PC absorbance detector.

Control Ligands

hTLR2: HKLM (heat-killed Listeria monocytogenes) at 1x108 cells/mL

hTLR3: Poly(I:C) HMW at 1 μg/mL

hTLR4: E. coli K12 LPS at 100 ng/mL

hTLR5: S. typhimurium flagellin at 100 ng/mL

hTLR7: CL307 at 1 μg/mL

hTLR8: CL075 at 1 μg/mL

hTLR9: CpG ODN2006 at 1 μg/mL.

**Example 7** - **Stability I**

Stability is assessed by tracking changes in the absolute peak area and % peak area of the compound subjected to the following conditions to the peak area and % peak area obtained from freshly prepared solutions of the relevant compound. The compound is formulated in each of the following formulations:

1. Phosphate buffered saline (PBS), pH 7.4. For example, the PBS buffer may comprise 8g NaCl, 0.2g KCl, 1.15g

disodium hydrogen phosphate and 0.2g potassium dihydrogen phosphate in 1 litre of MilliQ water.
2. 0.9% w/w saline (pH 5.8). For example, saline solution may be prepared by dissolving sodium chloride (1.855 g) in 200 mL of Milli-Q water.

Stability for each formulation is assessed under the following conditions:

1. 25°C/60% relative humidity (ICH ambient)
2. 40°C/75% relative humidity (ICH accelerated)

**Sample Preparation**

Solutions of approximately 1 mg/mL of each compound (2 mL) are accurately prepared in the PBS and saline diluent systems.
All compounds are heated to approximately 60°C under hot running tap water for approximately 30 seconds, followed by vortex mixing for a further 30 seconds, and then are further sub-aliquoted into 3 separate HPLC vials which are then placed into storage at 4-8°C (fridge), 25°C/65% RH and 40°C/75% relative humidity (RH) for 2 weeks. The vials are wrapped in aluminium foil to exclude light for the storage duration.

**Equipment and Operational Parameters**

A Shimadzu Nexera UHPLC with diode array detector is used to monitor peak area changes at t=0 and t=2 weeks.
A Shimadzu LCMS-8030 system is used to identify impurity and degradant peaks, and to verify the selectivity of the HPLC methods by checking across the main HPLC peak for possible co-eluting components. Exemplary ultra high-performance liquid chromatography (UHPLC) parameters are outlined below.

**UHPLC Parameters**

Column - Phenomenex Kinetex Biphenyl, 50 x 2.1 mm, 2.6 μm, part no. 00B-4622-AN
Vials - Agilent clear glass, 2 mL with multi-injection septa, part no. 226-50512-00
Mobile Phase A - 5 mM ammonium formate in Milli-Q water
Mobile Phase B - acetonitrile, Merck LC-MS grade
Needle Rinse Solution - 1:1 water:methanol

| | |
|---|---|
| Injection Volume: | 1 μL |
| Column Temperature: | 40°C |
| Autosampler Temp: | 20°C |
| Total Flow Rate: | 0.5 mL/min |
| Total Run Time: | 10 min |
| UV-vis wavelength: | 205 nm |

**Table 1:** Gradient 1

| Time (min) | %A | %B |
|---|---|---|
| Init | 55 | 45 |
| 0.1 | 55 | 45 |
| 8.0 | 25 | 75 |
| 8.5 | 25 | 75 |
| 8.6 | 55 | 45 |

**Table 2:** Gradient 2

| Time (min) | %A | %B |
|---|---|---|
| Init | 55 | 45 |

(continued)

| Time (min) | %A | %B |
|---|---|---|
| 0.1 | 55 | 45 |
| 8.0 | 25 | 65 |
| 8.5 | 25 | 65 |
| 8.6 | 55 | 45 |

**LCMS Parameters**

| | |
|---|---|
| LC injection volume: | 0.1 μL |
| Interface: | ESI |
| Interface Temperature: | 350°C |
| Desolvation Temperature: | 250°C |
| Nebuliser Flow: | 3 L/min |
| Heat Block: | 400°C |
| Drying Gas Flow: | 15 L/min |
| Q3 scan mode: | Positive |
| Start Time: | 1 min |
| End Time: | 8 min |
| Start m/z: | 400 |
| End m/z | 2000 (INNA-011) |
| Scan Speed: | 15000 μ/sec |

**Example 8** - **Stability II**

The relative stabilities of compound B12 and that of compound (8) of WO2019/119067 were evaluated under accelerated conditions (40°C/75% RH) for 9 days. Each compound was prepared at 1 mg/mL concentration in an aqueous formulation of 0.1% w/v ethylenediaminetetraacetic acid (EDTA) / 0.9% saline w/v buffered to pH 5.

The structure of compound (8) of WO2019/119067 is:

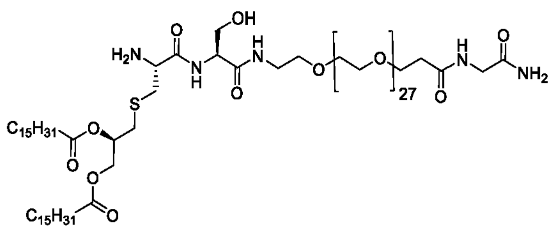

Stability was measured using reversed phase HPLC with a UV detector analytical wavelength of 205 nm. Peak areas of each compound at the 9 day time point were compared with areas at time zero. Compound stability at day 9 was calculated as a percentage of the time zero peak area data.

Compound stability was further assessed by comparison with a reference sample of the same compound. Reference samples were prepared at 1 mg/mL concentration in an aqueous formulation of 0.1% w/v EDTA / 0.9% saline w/v buffered to pH 5 and frozen during the period of testing. Thawed samples were sonicated and measured by HPLC.

Results are summarised in Table 3.

**Table 3**

| Compound | area% recovery (Day 9) | %RSD (relative standard deviation) in reference | %RSD in samples | Estimated total %RSD (RMS) |
|---|---|---|---|---|
| Compound (8) of WO2019/119067 | 94.4 | 0.01 | 0.02 | 0.02 |
| B12 | 98.4 | 0.00 | 0.23 | 0.23 |

Compound B12 is shown to possess substantial stability over the 9 day test period. Compound B12 also possesses superior stability under the accelerated conditions than the comparator compound.

**Example 9 - Activation of human TLR2 II**

The potency of the compounds as activators of human TLR-2s is tested in an *in vitro* assay in HEK-BLUE-hTLR2 cells.

Culturing of HEK-BLUE-hTLR2 cells

HEK-BLUE-hTLR2 cells are designed for studying the stimulation of human TLR2 (hTLR2) by monitoring the activation of NF-kB. HEK-BLUE-hTLR2 cells are obtained by co-transfection of the hTLR2 and SEAP (secreted embryonic alkaline phosphatase) reporter genes into HEK293 cells. Stimulation with a TLR2 ligand activates NF-kB which induces the production of SEAP.

HEK-BLUE-hTLR2 cells were purchased from InvivoGen (San Diego, CA, USA). Cells were grown in DMEM supplemented with 10% FCS, 100U/ml penicillin, 100ug/ml streptomycin and 2 mM L-glutamine, 100 μg/mL Normocin in the presence of selection antibiotic purchased from InvivoGen and passaged when 70% confluence was reached per manufacturer's recommendation. Cells were dislodged and resuspended in test media as suggested by manufacturer for testing.

Testing of compounds

i) A serial dilution of respective compounds were prepared in saline and added in 20ml of each dilution in triplicates per well in a flat bottom 96-well plate and placed in the incubator while waiting for the cells.
ii) Remove HEK-BLUE-hTLR2 cells in a T-75 flask from incubator and discard the growth media.
iii) Gently rinse the cells with prewarmed 10 ml of PBS
iv) Add 5ml of prewarmed PBS and place the cells in 37 °C for 2 mins and then detach the cells by gently pipetting up and down the PBS on the surface where the cells adhere.
v) Cells suspension at the density of 280,000 cells/ml is prepared in HEK-Blue™ Detection medium which is purchased from InvivoGen and prepared according to the manufacturer's instruction,
vi) Add immediately 180 ml of the cell suspension per well of the plate which contains the solution of the compounds. The plate is then returned to the incubator at 37°C for 16hr and was read at 620nm by using an ELISA reader.

The results of this assay for compounds B4 and B12 are outlined in Tables 4 (B4) and 5 (B12) and shown in Figure 1 (B4) and Figure 2 (B12). These data show that the compounds B4 and B12 exhibit significant activity at TLR2, where the $EC_{50}$ of compound B4 is 1.3 ng/mL and the $EC_{50}$ of compound B12 is 1.6 ng/mL.

**Table 4.** Human TLR2 dose response for compound B4.

| | Compound B4 (ng/mL) | | | | | | | |
|---|---|---|---|---|---|---|---|---|
| **Screening** | **125** | **31.3** | **7.8** | **2.0** | **0.5** | **0.1** | **0.31** | **0** |
| 1 | 2.451 | 2.438 | 2.214 | 1.660 | 0.719 | 0.182 | 0.126 | 0.093 |
| 2 | 2.411 | 2.327 | 2.163 | 1.529 | 0.594 | 0.206 | 0.115 | 0.115 |
| 3 | 2.467 | 2.350 | 2.214 | 1.498 | 0.617 | 0.197 | 0.115 | 0.101 |

(continued)

| | Compound B4 (ng/mL) | | | | | | | |
|---|---|---|---|---|---|---|---|---|
| **Screening** | **125** | **31.3** | **7.8** | **2.0** | **0.5** | **0.1** | **0.31** | **0** |
| | **Fold induction*** | | | | | | | |
| | 23.7 | 23.0 | 21.3 | 15.2 | 6.2 | 1.9 | 1.2 | 1.0 |
| Notes: Results are provided as optical density values (650nm)<br>* Ratio of average induced value to average non-induced value | | | | | | | | |

**Table 5.** Human TLR2 dose response for compound B12.

| | Compound B12 (ng/mL) | | | | | | | |
|---|---|---|---|---|---|---|---|---|
| **Screening** | **125** | **31.3** | **7.8** | **2.0** | **0.5** | **0.1** | **0.31** | **0** |
| 1 | 2.409 | 2.422 | 2.187 | 1.429 | 0.585 | 0.239 | 0.116 | 0.104 |
| 2 | 2.564 | 2.423 | 2.257 | 1.308 | 0.558 | 0.202 | 0.132 | 0.093 |
| 3 | 2.615 | 2.449 | 2.302 | 1.638 | 0.559 | 0.208 | 0.137 | 0.094 |
| | **Fold induction*** | | | | | | | |
| | 26.1 | 25.1 | 23.2 | 15.0 | 5.8 | 2.2 | 1.3 | 1.0 |
| Notes: Results are provided as optical density values (650nm)<br>* Ratio of average induced value to average non-induced value | | | | | | | | |

**Example 9 - Activation of human TLR2 III**

Toll-Like Receptor (TLR) stimulation is tested by assessing NF-KB activation in the TLR expressing cell lines. HEK-Blue h/mTLR2 cells have been stably transfected with human or mouse TLR2 and CD14. The activity of the test articles are tested on human and mouse TLR2, as potential agonists. The test articles are evaluated at seven concentrations and compared to control ligands (see list below). These steps are performed in triplicate.

**Control Ligands**

HEK-Blue hTLR2 Dose Response:
HKLM (heat-killed *Listeria monocytogenes*) at 1.0 x $10^8$, 2.5 x $10^7$, 6.25 x $10^6$, 1.56 x $10^6$, 3.91 x $10^5$, 9.76 x $10^4$ and 2.44 x $10^4$ cells/mL
HEK-Blue mTLR2 Dose Response:
HKLM (heat-killed *Listeria monocytogenes*) at 1.0 x $10^8$, 2.5 x $10^7$, 6.25 x $10^6$, 1.56 x $10^6$, 3.91 x $10^5$, 9.76 x $10^4$ and 2.44 x $10^4$ cells/mL

**TLR- Control Cell Lines**

HEK-Blue Null1 Dose Response:

TNF$\alpha$ at 100, 25, 6.25, 1.56, 0.39, 0.098 and 0.024 ng/mL
Control for human TLR2

HEK-Blue Null2 Dose Response:

TNF$\alpha$ at 100, 25, 6.25, 1.56, 0.39, 0.098 and 0.024 ng/mL
Control for mouse TLR2

**Test articles and materials**

| | |
|---|---|
| Article 1: | B4 |
| Weight: | 2.2 mg |
| Resuspension: | 1.1 mL PBS |
| Stock Concentration: | 2 mg/mL |
| Final Concentrations: | 500, 125, 31.25, 7.81, 1.95, 0.49 and 0.12 ng/mL |
| Storage Condition: | -20°C |

| | |
|---|---|
| Article 2: | B6 |
| Weight: | 2.4 mg |
| Resuspension: | 1.2 mL PBS |
| Stock Concentration: | 2 mg/mL |
| Final Concentrations: | 500, 125, 31.25, 7.81, 1.95, 0.49 and 0.12 ng/mL |
| Storage Condition: | -20°C |

| | |
|---|---|
| Article 3: | B8 |
| Weight: | 2.4 mg |
| Resuspension: | 1.2 mL PBS |
| Stock Concentration: | 2 mg/mL |
| Final Concentrations: | 500, 125, 31.25, 7.81, 1.95, 0.49 and 0.12 ng/mL |
| Storage Condition: | -20°C |

| | |
|---|---|
| Article 4: | B14 |
| Weight: | 2.6 mg |
| Resuspension: | 1.3 mL PBS |
| Stock Concentration: | 2 mg/mL |
| Final Concentrations: | 500, 125, 31.25, 7.81, 1.95, 0.49 and 0.12 ng/mL |
| Storage Condition: | -20°C |

| | |
|---|---|
| Article 5: | B12 |
| Weight: | 2.1 mg |
| Resuspension: | 1.05 mL PBS |
| Stock Concentration: | 2 mg/mL |
| Final Concentrations: | 500, 125, 31.25, 7.81, 1.95, 0.49 and 0.12 ng/mL |
| Storage Condition: | -20°C |

| | |
|---|---|
| Article 6: | B16 |
| Weight: | 2.2 mg |

(continued)

| | |
|---|---|
| Resuspension: | 1.1 mL PBS |
| Stock Concentration: | 2 mg/mL |
| Final Concentrations: | 500, 125, 31.25, 7.81, 1.95, 0.49 and 0.12 ng/mL |
| Storage Condition: | -20°C |

**Preparation of test articles**

A series of two 1:10 serial dilutions are prepared in sterile PBS starting from the 2 mg/mL stock solution of each of compounds B4, B6, B8, B12, B14 and B16 and ending with 20 μg/mL. A 5 μg/mL working stock was then prepared for each compound from the 20 μg/mL dilution in sterile PBS. Starting from the 5 μg/mL working stock, a series of six 1:4 serial dilutions were made by mixing 100 μL of the previous highest dilution with 300 μL sterile PBS.

**General Procedure**

The secreted embryonic alkaline phosphatase (SEAP) reporter is under the control of a promoter inducible by the transcription factor NF-κB. This reporter gene allows the monitoring of signaling through the TLR, based on the activation of NF-κB. In a 96-well plate (200 μL total volume) containing the appropriate cells (50,000 - 75,000 cells/well), 20 μL of the test article or the positive control ligand (HKLC) is added to the wells. The media added to the wells is designed for the detection of NF-κB induced SEAP expression. After a 16-24-hour incubation the optical density (OD) is read at 650 nm on a Molecular Devices SpectraMax 340PC absorbance detector.

**Results**

All test articles showed TLR2 agonist activity for both human TLR2 (hTLR2) and mouse TLR2 (mTLR2). Results for hTLR agonist are shown in Tables 6 to 12 and Figure 3.

**Table 6.** HEK-Blue hTLR2 Dose Response for compound B4. Results are provided as optical density values (650 nm)

| | **Concentration (pg/mL)** | | | | | | | |
|---|---|---|---|---|---|---|---|---|
| **Screening** | **0** | **0.12** | **0.49** | **1.95** | **7.81** | **31.25** | **125** | **500** |
| 1 | 0.071 | 0.653 | 1.409 | 1.795 | 2.288 | 2.440 | 2.569 | 2.717 |
| 2 | 0.072 | 0.759 | 1.507 | 1.757 | 2.168 | 2.485 | 2.508 | 2.590 |
| 3 | 0.074 | 0.809 | 1.521 | 1.916 | 2.323 | 2.518 | 2.487 | 2.526 |
| **Average** | **0.072** | **0.740** | **1.479** | **1.823** | **2.260** | **2.481** | **2.521** | **2.611** |
| | **Fold Induction*** | | | | | | | |
| | 1.0 | 10.3 | 20.5 | 25.3 | 31.4 | 34.5 | 35.0 | 36.3 |
| * Ratio of average induced value to average non-induced value. | | | | | | | | |

**Table 7.** HEK-Blue hTLR2 Dose Response for compound B6. Results are provided as optical density values (650 nm)

| | **Concentration (pg/mL)** | | | | | | | |
|---|---|---|---|---|---|---|---|---|
| **Screening** | **0** | **0.12** | **0.49** | **1.95** | **7.81** | **31.25** | **125** | **500** |
| 1 | 0.069 | 0.591 | 1.230 | 1.755 | 2.154 | 2.512 | 2.522 | 2.649 |
| 2 | 0.072 | 0.742 | 1.151 | 1.776 | 2.186 | 2.527 | 2.466 | 2.606 |
| 3 | 0.074 | 0.704 | 1.088 | 1.824 | 2.219 | 2.547 | 2.422 | 2.656 |
| **Average** | **0.072** | **0.679** | **1.156** | **1.785** | **2.186** | **2.529** | **2.470** | **2.637** |

(continued)

| | Concentration (pg/mL) | | | | | | | |
|---|---|---|---|---|---|---|---|---|
| **Screening** | **0** | **0.12** | **0.49** | **1.95** | **7.81** | **31.25** | **125** | **500** |
| | **Fold Induction*** | | | | | | | |
| | 1 | 9.4 | 16.1 | 24.8 | 30.4 | 35.1 | 34.3 | 36.6 |
| * Ratio of average induced value to average non-induced value. | | | | | | | | |

**Table 8.** HEK-Blue hTLR2 Dose Response for compound B8. Results are provided as optical density values (650 nm)

| | Concentration (pg/mL) | | | | | | | |
|---|---|---|---|---|---|---|---|---|
| **Screening** | **0** | **0.12** | **0.49** | **1.95** | **7.81** | **31.25** | **125** | **500** |
| 1 | 0.072 | 1.936 | 2.345 | 2.469 | 2.326 | 2.563 | 2.746 | 2.648 |
| 2 | 0.072 | 1.856 | 2.229 | 2.420 | 2.636 | 2.643 | 2.716 | 2.664 |
| 3 | 0.083 | 1.992 | 2.154 | 2.507 | 2.734 | 2.751 | 2.743 | 2.693 |
| **Average** | **0.076** | **1.928** | **2.243** | **2.465** | **2.565** | **2.652** | **2.735** | **2.668** |
| | **Fold Induction*** | | | | | | | |
| | 1 | 25.4 | 29.5 | 32.4 | 33.8 | 34.9 | 36 | 35.1 |
| * Ratio of average induced value to average non-induced value. | | | | | | | | |

**Table 9.** HEK-Blue hTLR2 Dose Response for compound B12. Results are provided as optical density values (650 nm)

| | Concentration (pg/mL) | | | | | | | |
|---|---|---|---|---|---|---|---|---|
| **Screening** | **0** | **0.12** | **0.49** | **1.95** | **7.81** | **31.25** | **125** | **500** |
| 1 | 0.071 | 1.443 | 1.892 | 2.149 | 2.420 | 2.492 | 2.608 | 2.584 |
| 2 | 0.072 | 1.313 | 1.785 | 2.008 | 2.446 | 2.500 | 2.541 | 2.404 |
| 3 | 0.077 | 1.329 | 1.902 | 1.979 | 2.366 | 2.380 | 2.555 | 2.426 |
| **Average** | **0.073** | **1.362** | **1.860** | **2.045** | **2.411** | **2.457** | **2.568** | **2.471** |
| | **Fold Induction*** | | | | | | | |
| | 1 | 18.7 | 25.5 | 28 | 33 | 33.7 | 35.2 | 33.9 |
| * Ratio of average induced value to average non-induced value. | | | | | | | | |

**Table 10.** HEK-Blue hTLR2 Dose Response for compound B14. Results are provided as optical density values (650 nm)

| | Concentration (pg/mL) | | | | | | | |
|---|---|---|---|---|---|---|---|---|
| **Screening** | **0** | **0.12** | **0.49** | **1.95** | **7.81** | **31.25** | **125** | **500** |
| 1 | 0.073 | 0.826 | 1.256 | 1.994 | 2.569 | 2.820 | 2.928 | 2.722 |
| 2 | 0.073 | 0.869 | 1.364 | 2.061 | 2.545 | 2.720 | 2.800 | 2.765 |
| 3 | 0.072 | 0.774 | 1.406 | 2.000 | 2.571 | 2.691 | 2.713 | 2.791 |
| **Average** | **0.073** | **0.823** | **1.342** | **2.018** | **2.562** | **2.744** | **2.814** | **2.759** |
| | **Fold Induction*** | | | | | | | |
| | 1 | 18.7 | 25.5 | 28 | 33 | 33.7 | 35.2 | 33.9 |
| * Ratio of average induced value to average non-induced value. | | | | | | | | |

**Table 11.** HEK-Blue hTLR2 Dose Response for compound B16. Results are provided as optical density values (650 nm)

| | Concentration (pg/mL) | | | | | | | |
|---|---|---|---|---|---|---|---|---|
| **Screening** | **0** | **0.12** | **0.49** | **1.95** | **7.81** | **31.25** | **125** | **500** |
| 1 | 0.071 | 0.688 | 1.080 | 1.478 | 1.903 | 2.093 | 2.531 | 2.466 |
| 2 | 0.074 | 0.591 | 1.097 | 1.513 | 1.897 | 2.297 | 2.544 | 2.488 |
| 3 | 0.072 | 0.718 | 1.121 | 1.573 | 2.133 | 2.367 | 2.545 | 2.472 |
| **Average** | **0.072** | **0.666** | **1.099** | **1.521** | **1.978** | **2.252** | **2.540** | **2.475** |
| | **Fold Induction*** | | | | | | | |
| | 1 | 9.2 | 15.3 | 21.1 | 27.5 | 31.3 | 35.3 | 34.4 |
| * Ratio of average induced value to average non-induced value. | | | | | | | | |

**Table 12.** HEK-Blue hTLR2 Dose Response for HKLM. Results are provided as optical density values (650 nm)

| | Concentration (pg/mL) | | | | | | | |
|---|---|---|---|---|---|---|---|---|
| Screening | **0** | **24400** | **97700** | **391000** | **1560000** | **6200000** | **25000000** | **100000000** |
| 1 | 0.079 | 0.124 | 0.260 | 0.826 | 1.809 | 2.574 | 2.777 | 2.607 |
| 2 | 0.081 | 0.124 | 0.253 | 0.769 | 1.866 | 2.605 | 2.747 | 2.574 |
| 3 | 0.079 | 0.118 | 0.271 | 0.783 | 1.653 | 2.382 | 2.681 | 2.715 |
| **Average** | **0.080** | **0.122** | **0.261** | **0.793** | **1.776** | **2.520** | **2.735** | **2.632** |
| | **Fold Induction*** | | | | | | | |
| | 1 | 1.5 | 3.3 | 10 | 22.5 | 31.9 | 34.6 | 33.3 |
| * Ratio of average induced value to average non-induced value. | | | | | | | | |

**Table 13.** $EC_{50}$ values for agonism of hTLR2

| **Compound** | **$EC_{50}$ (hTLR2)** |
|---|---|
| B4 | 0.46 ng/mL |
| B6 | 0.73 ng/mL |
| B8 | n.d. |
| B12 | 0.11 ng/mL |
| B14 | 0.57 ng/mL |
| B16 | 1.09 ng/mL |
| HKLM | $9.24 \times 10^5$ cells/mL |
| n.d. = $EC_{50}$ value could not be calculated due to lack of confidence in extrapolation of sigmoidal response curve due to high activity at lowest concentration tested. | |

**Conclusions**

Each of compounds B4, B6, B8, B12, B14 and B16 possesses stimulatory activity for hTLR2 and mTLR2 in the described HEK-Blue assay. The stimulatory response was not observed in HEK-Blue Null1 (human) or HEK-Blue Null2 (mouse) cells, confirming that the compounds' efficacy is mediated by hTLR2 or mTLR2.

**Example 10 - Activation of human TLR2 IV**

The potency of compound 4 of WO2020/257870 and compound B12 as activators of human TLR-2s is tested in an *in vitro* assay in HEK-BLUE-hTLR2 cells.

The structure of compound 4 of WO2020/257870 is:

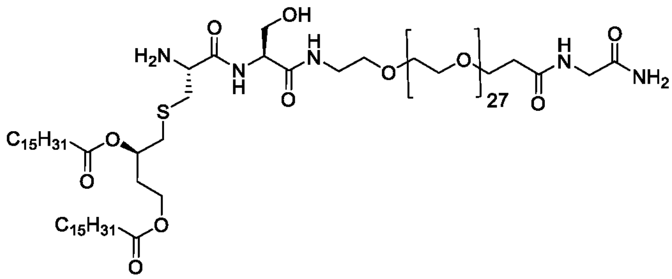

**Culturing of HEK-BLUE-hTLR2 cells**

HEK-BLUE-hTLR2 cells are designed for studying the stimulation of human TLR2 (hTLR2) by monitoring the activation of NF-kB. HEK-BLUE-hTLR2 cells are obtained by co-transfection of the hTLR2 and SEAP (secreted embryonic alkaline phosphatase) reporter genes into HEK293 cells. Stimulation with a TLR2 ligand activates NF-kB which induces the production of SEAP.

HEK-BLUE-hTLR2 cells were purchased from InvivoGen (San Diego, CA, USA). Cells were grown in DMEM supplemented with 10% FCS, 100U/ml penicillin, 100ug/ml streptomycin and 2 mM L-glutamine, 100 μg/mL Normocin in the presence of selection antibiotic purchased from InvivoGen and passaged when 70% confluence was reached per manufacturer's recommendation. Cells were dislodged and resuspended in test media as suggested by manufacturer for testing.

Testing of compounds

vii) A serial dilution of respective compounds were prepared in saline and added in 20ml of each dilution in triplicates per well in a flat bottom 96-well plate and placed in the incubator while waiting for the cells.
viii) Remove HEK-BLUE-hTLR2 cells in a T-75 flask from incubator and discard the growth media.
ix) Gently rinse the cells with prewarmed 10 ml of PBS
x) Add 5ml of prewarmed PBS and place the cells in 37 °C for 2 mins and then detach the cells by gently pipetting up and down the PBS on the surface where the cells adhere.
xi) Cells suspension at the density of 280,000 cells/ml is prepared in HEK-Blue™ Detection medium which is purchased from InvivoGen and prepared according to the manufacturer's instruction,
xii) Add immediately 180 ml of the cell suspension per well of the plate which contains the solution of the compounds. The plate is then returned to the incubator at 37°C for 16hr and was read at 620nm by using an ELISA reader.

The results of this assay for compound 4 of WO2020/257870 and compound B12 are shown in Figure 4. These data show that these compounds exhibit significant activity at TLR2.

**Example 11** - **Stability III**

Following a similar procedure to that described in Example 8, compounds B12, B14, B16 and compound 4 of WO2020/257870 were subjected to accelerated aging conditions at 40 °C for either 3 weeks or 6 weeks. Recovery of the target compound was assessed by HPLC, and the values as a percentage of the reference sample are reported in Table 14 and in Figure 5.

**Table 14.** Percentage recovery results at 40 °C

| | **Recovery compared to reference sample (%)** | |
|---|---|---|
| **Compound ID** | **3 weeks** | **6 weeks** |
| Compound 4 of WO2020/257870 | 79.40% | 60.70% |
| B14 | 97.91% | 83.99% |
| B12 | 101.60% | 101.33% |
| B16 | 89.97% | 88.35% |

These data show that each of compounds B12, B14 and B16 possess excellent stabilities under the accelerated storage conditions. Further, each of compounds B12, B14 and B16 demonstrated improved storage stability compared with compound 4 of WO2020/257870.

**Example 12** - **Stability IV**

Following a similar procedure to that described in Example 8, compound 8 of WO2019/119067, compound 4 of WO2020/257870, and compounds B4 and B12 were assessed for storage stability under accelerated conditions over a period of 28 days.

Each compound was formulated in 0.9% w/w saline with addition of 0.1% w/w EDTA at about pH 5. The formulations were stored in the dark under accelerated conditions (40 °C/75% RH) and reference formulations were stored at -80 °C. At days 0, 9 and 28, samples were analysed by UHPLC and pH was measured.

This experiment shows that following storage under the accelerated conditions after 28 days, B4 and B12 are the most stable compounds.

**Sample preparation**

Approximately 1.5 mg of each compound was weighed into glass HPLC vials and 1.5 mL of saline/EDTA formulation adjusted to pH 5.0 was added. The vials were vortex mixed then warmed under running hot water until full dissolution was observed. Actual weights and concentrations of compounds are presented in Table 15.

**Table 15.** Weights and concentrations of formulated compound

| Compound | Mass (mg) | Final Volume (mL) | Concentration (mg/mL) |
|---|---|---|---|
| Compound 8 of WO2019/119067 | 1.658 | 1.5 | 1.1 |
| Compound 4 of WO2020/257870 | 1.566 | 1.5 | 1.0 |
| B12 | 1.758 | 1.5 | 1.2 |
| B4 | 1.627 | 1.5 | 1.1 |

4 x 100 uL aliquots of each solution were transferred to Agilent polypropylene 200 μL HPLC vials for storage under accelerated and reference conditions (two aliquots at each condition). These vials were removed at each time point for HPLC analysis. The frozen reference solutions at Day 9 and Day 28 were gently warmed under running hot water followed by vortex mixing for ~ 10 sec and sonication for 10 min to encourage complete dissolution.

The remaining ~ 1 mL of solution in the clear glass HPLC vials was stored at 40°C/75% RH and was used to measure pH at each time point.

**UHPLC analysis**

UHPLC analysis of compound 8 of WO2019/119067, compound 4 of WO2020/257870, B4 and B12, was performed using the following conditions:

Column: Phenomenex Kinetex Biphenyl, 50 mm x 2.1 mm, 2.6 μm, part no. 00B-4622-AN
Mobile Phase A: 5 mM ammonium formate in Milli-Q water, pH unadjusted
Mobile Phase B: acetonitrile, Merck LC-MS grade
Needle Rinse Solution: 1:1 water:methanol
Injection Volume: 5 μL
Column Temperature: 40°C
Autosampler Temp: 20°C
Total Flow Rate: 0.5 mL/min
Total Run Time: 10 min
UV-vis wavelength: 205 nm

Compound 8 of WO2019/119067 and compound 4 of WO2020/257870 were analysed according to Gradient 1:

**Table 16.** Gradient 1

| Time (min) | %A | %B |
|---|---|---|
| Init | 55 | 45 |
| 0.1 | 55 | 45 |
| 8.0 | 25 | 75 |
| 8.5 | 25 | 75 |
| 8.6 | 55 | 45 |
| 10.0 | 55 | 45 |

Compounds B4 and B12 were analysed according to Gradient 2:

**Table 17.** Gradient 2

| Time (min) | %A | %B |
|---|---|---|
| Init | 55 | 45 |
| 0.1 | 55 | 45 |
| 7.0 | 25 | 80 |
| 8.5 | 25 | 80 |
| 8.6 | 55 | 45 |
| 10.0 | 55 | 45 |

**UHPLC results**

UHPLC results were analysed in a similarly to that described in Example 8. Results are provided as percentage recovery of main peak area (Table 18).

Percentage recovery of main peak area is calculated according to the following equation (1):

$$Percent\ Recovery = \frac{Area\ of\ Main\ Peak\ (accelerated)}{Area\ of\ Main\ Peak\ (frozen)} \times 100 \qquad (1)$$

**Table 18.** Stability results calculated as percentage recovery of main peak area

| | Day 0 | | Day 9 | | Day 28 | |
|---|---|---|---|---|---|---|
| | % Recovery | Standard Error (% RSD) | % Recovery | Standard Error (% RSD) | % Recovery | Standard Error (% RSD) |
| Compound 8 of WO2019/119067 | 100 | NA | 94.5 | 0.14 | 71.3 | 11.50 |
| Compound 4 of WO2020/257870 | 100 | NA | 92.6 | 0.41 | 70.3 | 4.80 |
| B12 | 100 | NA | 98.9 | 1.60 | 94.3 | 0.36 |
| B4 | 100 | NA | 102.9 | 1.15 | 92.9 | 0.44 |

The greatest differentiation in compound stability was observed at the 28-day time point (Table 18). Too little degradation had occurred at Day 9 for any clear distinctions between compounds to be observed. On the basis of the 28-day data in Table 18, compounds B4 and B12 are the most stable compounds.

**pH results**

The formulation pH data recorded at each time point are compiled in Table 19. There was a general trend for the pH to increase with time but the effect was not pronounced. This indicates that gross degradation had not occurred.

**Table 19.** pH results at 0, 9 and 28 days

| Compound | 0-days | 9-days | 28-days |
|---|---|---|---|
| Compound 8 of WO2019/119067 | 5.5 | 5.6 | 5.7 |
| Compound 4 of WO2020/257870 | 5.0 | 5.2 | 5.2 |
| B12 | 5.6 | 5.7 | 5.7 |
| B4 | 5.6 | 5.7 | 5.7 |

**Conclusions**

These UHPLC and pH stability data suggest that the compounds of this invention demonstrate improved stability compared to other structurally related TLR2 agonists.

**Claims**

1. A compound of formula (I):

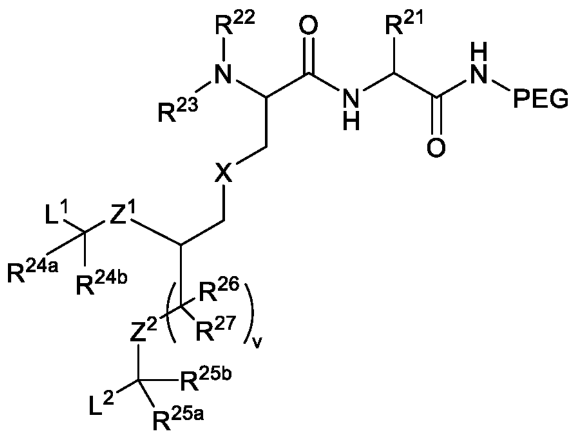

(I)

wherein:

$R^{21}$ is selected from the group consisting of H, $-CH_2OH$, $-CH_2CH_2OH$, $-CH(CH_3)OH$, $-CH_2OPO(OH)_2$, $-CH_2C(=O)NH_2$, $-CH_2CH_2C(=O)OH$ and $-CH_2CH_2C(=O)OR_8$, wherein any one of the alkyl hydrogens can be replaced with a halogen;
$R^{22}$ is H, $C_1$-$C_6$ aliphatic, an amino protecting group, $L^3$-C(=O)-, or $A^2$;
$L^1$ and $L^2$ are each independently $C_6$-$C_{21}$ aliphatic or $C_5$-$C_{20}$ heteroaliphatic;
$L^3$ is $C_1$-$C_{21}$ aliphatic or $C_2$-$C_{20}$ heteroaliphatic;
$A^2$ is an amino acid or a peptide;
$R^{23}$ is H or $C_1$-$C_6$ aliphatic;
$R^{24a}$ and $R^{25a}$ are each independently selected from $C_1$-$C_6$ aliphatic and $C_1$-$C_6$ heteroaliphatic and $R^{24b}$ and $R^{25b}$ are each independently selected from H, $C_1$-$C_6$ aliphatic and $C_1$-$C_6$ heteroaliphatic, or $R^{24a}$ and $R^{24b}$ together with the carbon atom to which they are attached form a $C_{3-8}$cycloalkyl or 3-8 membered heterocyclyl group, and/or $R^{25a}$ and $R^{25b}$ together with the carbon atom to which they are attached form a $C_{3-8}$cycloalkyl or 3-8 membered heterocyclyl group;
X is selected from -S-, -S(=O)- and $-S(=O)_2$-;
v is an integer from 1-3
$R^{26}$ and $R^{27}$ are each independently selected from H and $C_1$-$C_6$ aliphatic;

$Z^1$ and $Z^2$ are independently selected from the group consisting of -C(=O)O-, -OC(=O)-, -C(=O)NR-, -NRC(=O)-, -C(=O)S-, -SC(=O)-, -OC(=O)O-, -NRC(=O)O-, -OC(=O)NR-, and -NRC(=O)NR-;
PEG is a polyethylene glycol;
wherein any aliphatic, heteroaliphatic, cycloalkyl and heterocyclyl present in any of $R^{21}$, $R^{22}$, $R^{23}$, $R^{24a}$, $R^{24b}$, $R^{25a}$, $R^{25b}$, $R^{26}$, $R^{27}$, $L^1$, $L^2$ and $L^3$ is optionally substituted
or a pharmaceutically acceptable salt, solvate or prodrug thereof.

**2.** The compound of claim 1, or a pharmaceutically acceptable salt, solvate or prodrug thereof, wherein the PEG is a substituted polyethylene glycol of formula B-I:

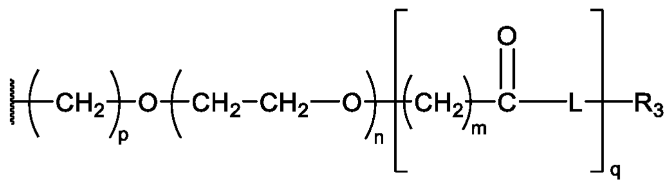

(B-I)

wherein

n is 3 to 100;
m is 1, 2, 3 or 4;
p is 2, 3 or 4;
q is null or 1;
$R_3$ is H, -$NH_2$ or -OH, wherein when q is null, $R_3$ is H and when q is 1, $R_3$ is -$NH_2$ or -OH;
L is null or consists of 1 to 10 units, wherein each unit is a natural alpha amino acid or derived from a natural alpha amino acid, and has the formula:

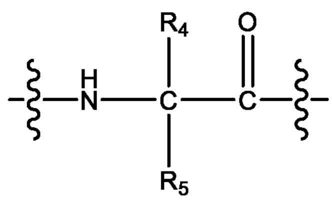

wherein $R_4$ is H; and
$R_5$ is the side chain, or second hydrogen of the amino acid.

**3.** The compound of claim 1 or 2, or a pharmaceutically acceptable salt, solvate or prodrug thereof, wherein $R^{24b}$ and $R^{25b}$ are H.

**4.** The compound of any one of claims 1-3, or a pharmaceutically acceptable salt, solvate or prodrug thereof, wherein $R^{24a}$ and $R^{25a}$ are independently a $C_1$-$C_6$ aliphatic, preferably wherein $R^{24a}$ and $R^{25a}$ are independently a $C_1$-$C_6$ alkyl, more preferably wherein $R^{24a}$ and $R^{25a}$ are each methyl.

**5.** The compound of any one of claims 1-4, or a pharmaceutically acceptable salt, solvate or prodrug thereof, wherein v is 1 or 2.

**6.** The compound of any one of claims 1-5, or a pharmaceutically acceptable salt, solvate or prodrug thereof, wherein X is -S-.

**7.** The compound of any one of claims 1-6, or a pharmaceutically acceptable salt, solvate or prodrug thereof, wherein $R^{22}$ is H.

**8.** The compound of any one of claims 1-7, or a pharmaceutically acceptable salt, solvate or prodrug thereof, wherein $R^{23}$ is H.

**9.** The compound of any one of claims 1-8, or a pharmaceutically acceptable salt, solvate or prodrug thereof, wherein $R^{26}$ is H.

**10.** The compound of any one of claims 1-9, or a pharmaceutically acceptable salt, solvate or prodrug thereof, wherein $R^{27}$ is H.

**11.** The compound of any one of claims 1-10, or a pharmaceutically acceptable salt, solvate or prodrug thereof, wherein $R^{21}$ is $-CH_2OH$.

**12.** The compound of any one of claims 1-11, or a pharmaceutically acceptable salt, solvate or prodrug thereof, wherein $Z^1$ and $Z^2$ are each -C(=O)-O-.

**13.** The compound according to any one of the preceding claims selected from any one of the following compounds:

| **Compound Structure** | **Compound ID** |
|---|---|
| 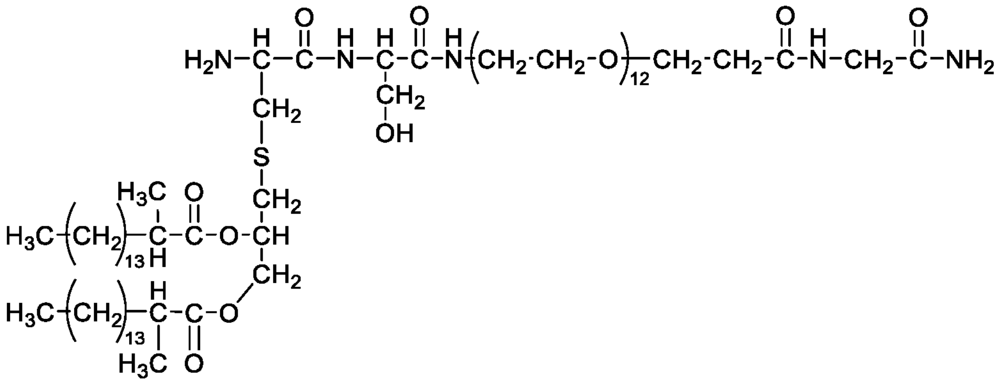 | B1 |
| 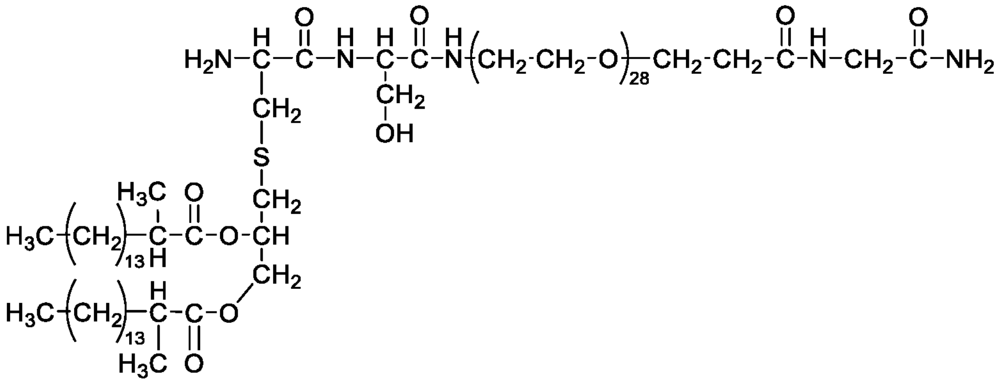 | B2 |
| 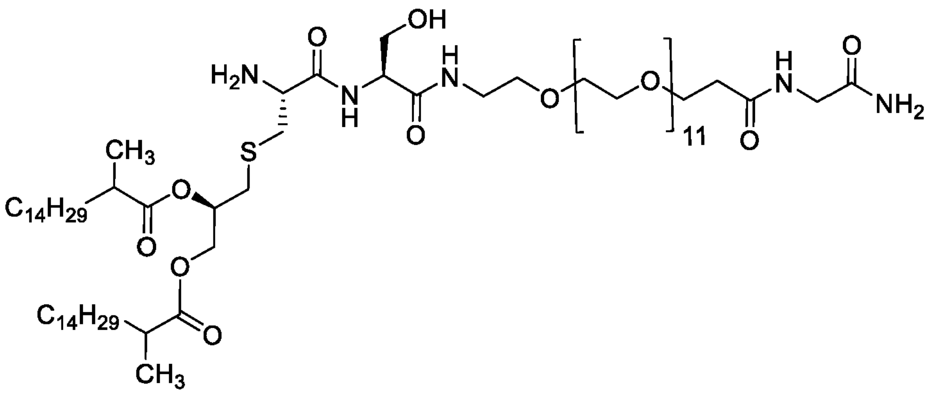 | B3 |

(continued)

| Compound Structure | Compound ID |
|---|---|
| 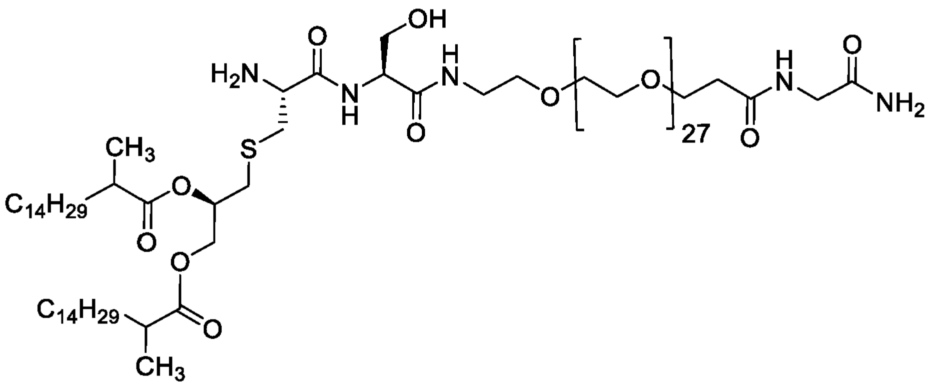 | B4 |
| 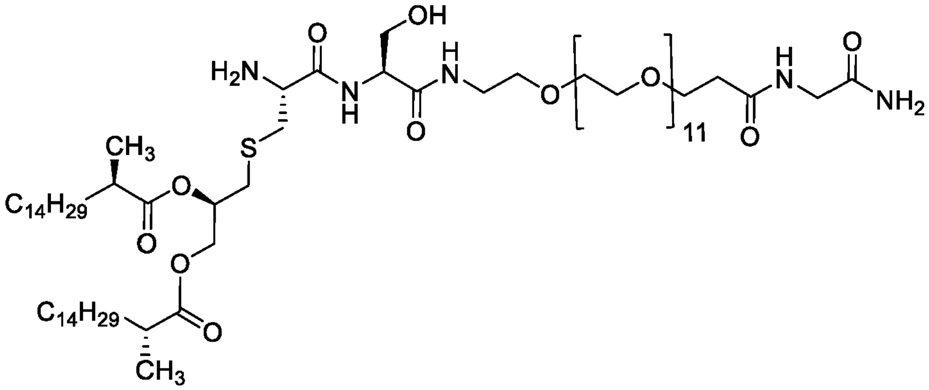 | B5 |
| 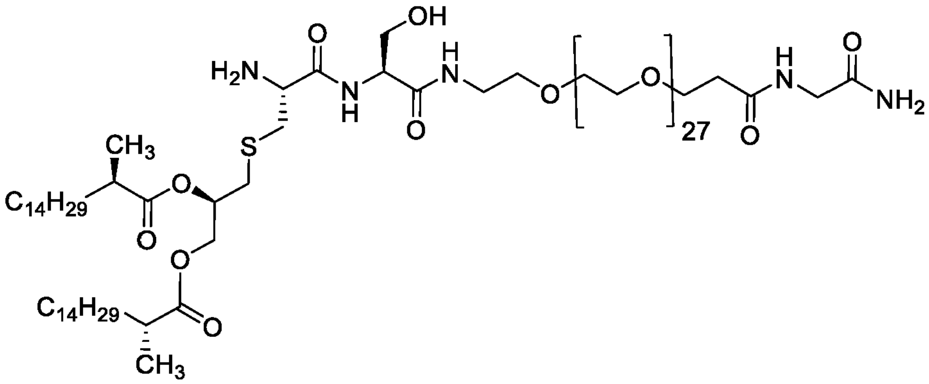 | B6 |
| 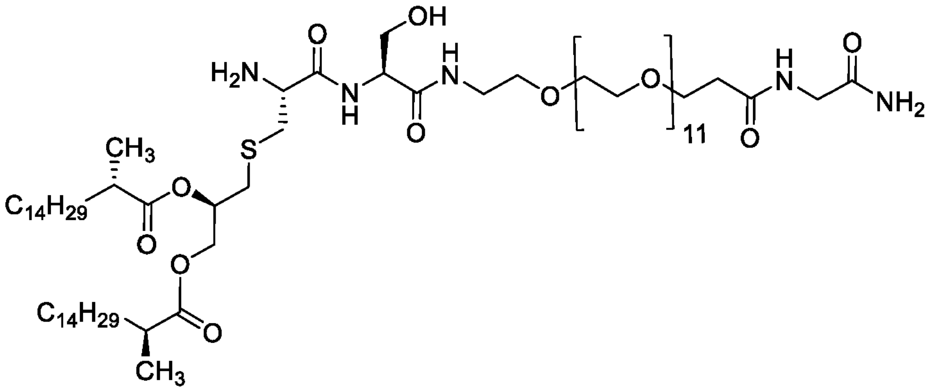 | B7 |

(continued)

| Compound Structure | Compound ID |
|---|---|
| 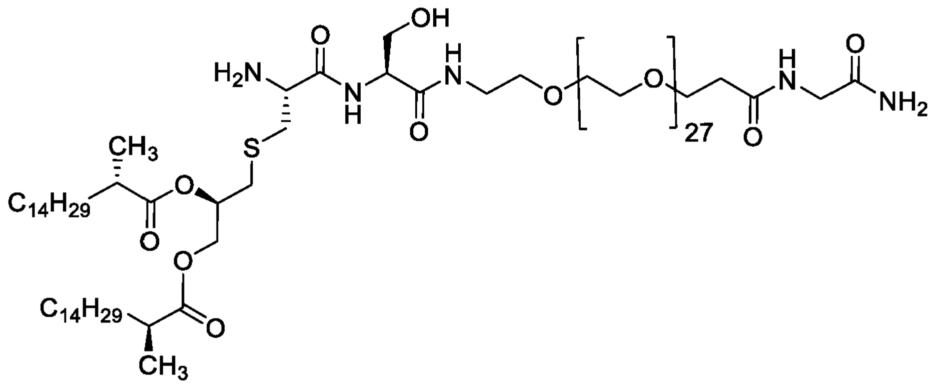 | B8 |
| 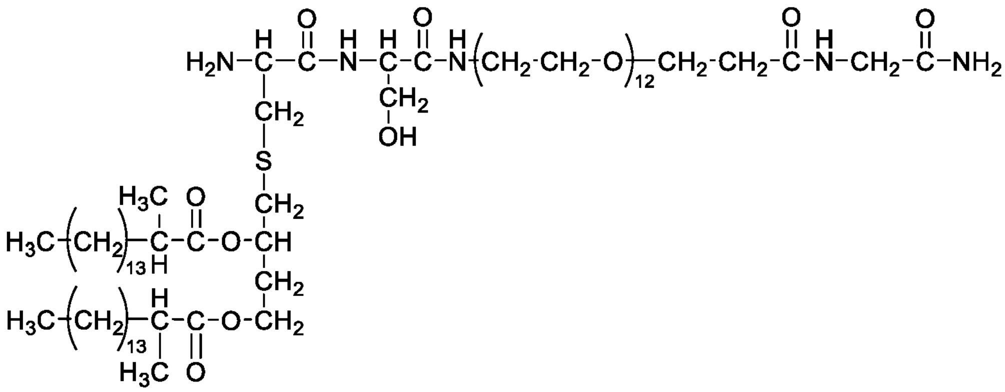 | B9 |
| 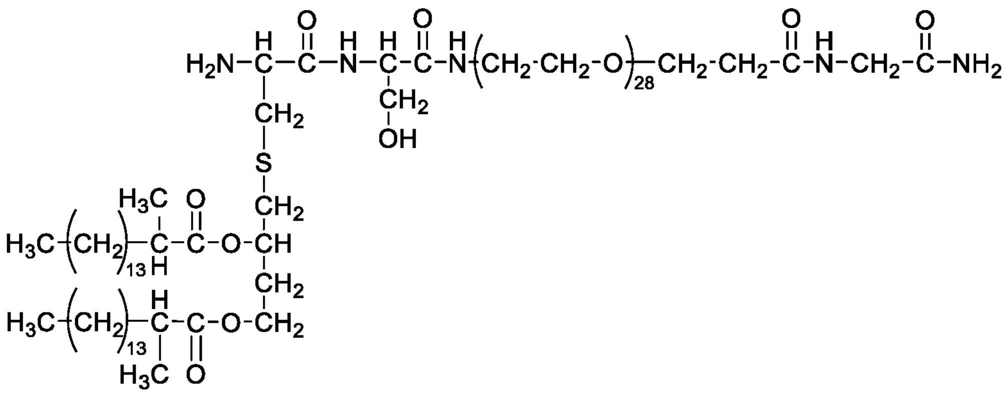 | B10 |
| 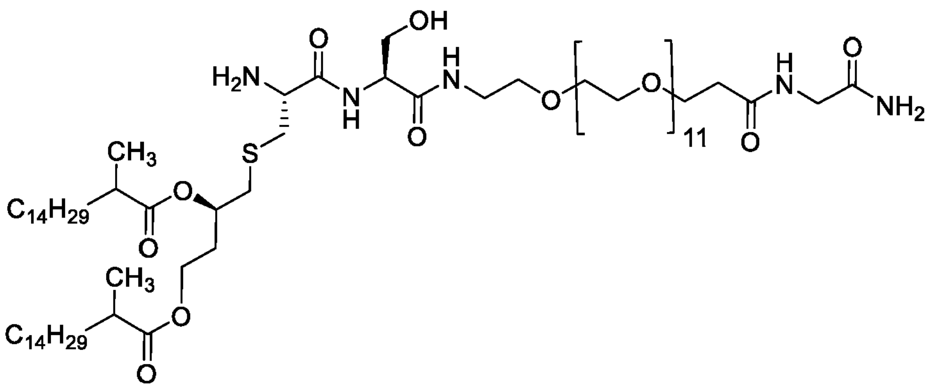 | B11 |

(continued)

| Compound Structure | Compound ID |
|---|---|
| 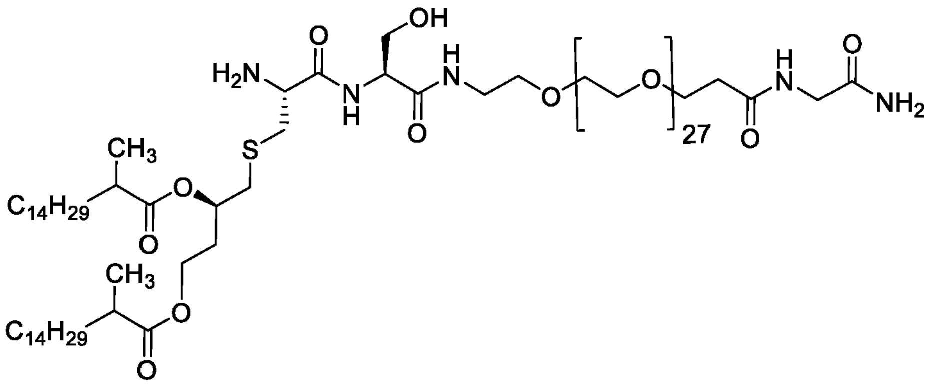 | B12 |
| 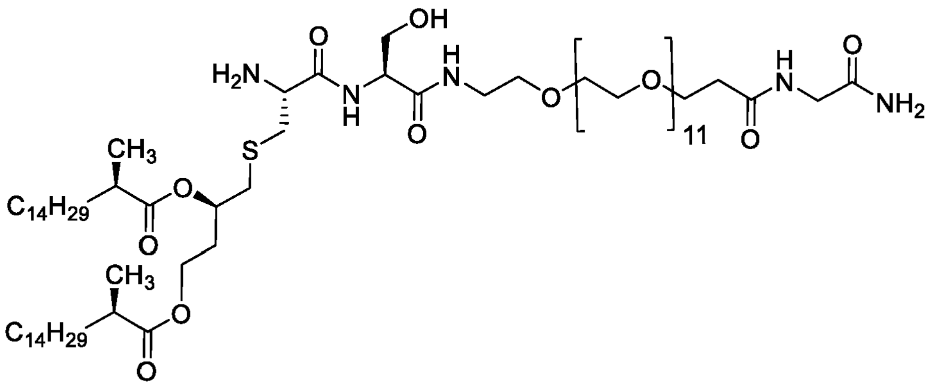 | B13 |
| 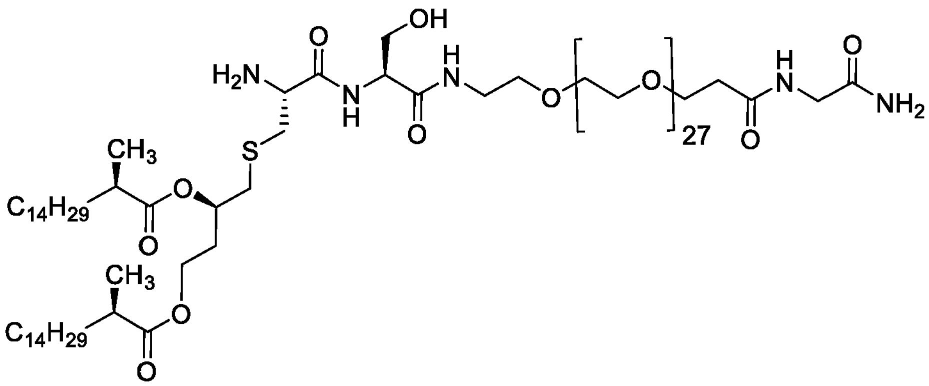 | B14 |
| 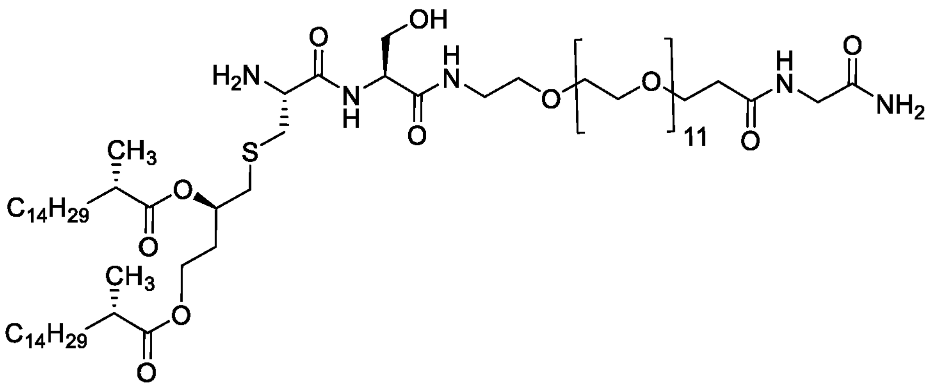 | B15 |

(continued)

| Compound Structure | Compound ID |
| --- | --- |
| 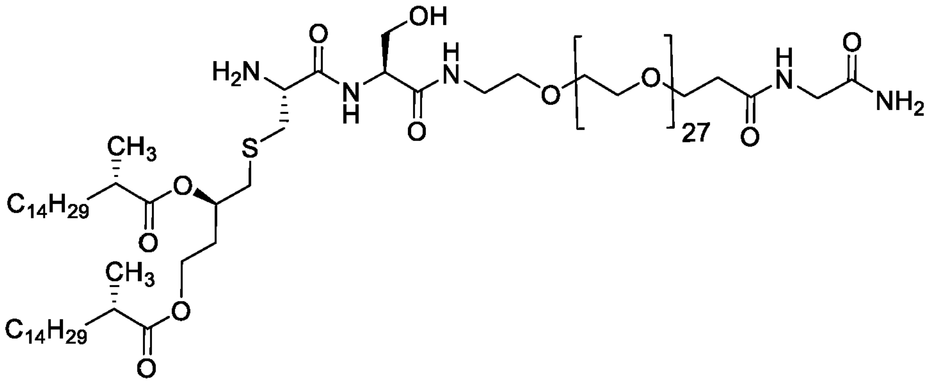 | B16 |

or a pharmaceutically acceptable salt, solvate or prodrug thereof.

**14.** A composition comprising a compound according to any one of the preceding claims, or a pharmaceutically acceptable salt, solvate or prodrug thereof, and optionally a pharmaceutically acceptable carrier, diluent or excipient.

**15.** A compound of any one of claims 1-13 or a pharmaceutically acceptable salt, solvate or prodrug thereof for use in any of:

- raising an innate immune response; and/or
- treating and/or preventing a disease caused by an infectious agent; and/or
- treating and/or preventing a respiratory disease or condition associated with a viral or bacterial infection; and/or
- treating and/or preventing a respiratory infection; and/or
- reducing airway inflammation; and/or
- improving the ability of a subject to control a respiratory disease or condition during a respiratory viral infection; and/or
- treating and/or preventing a disease or condition associated with the TLR2 receptor.

**Patentansprüche**

**1.** Verbindung von Formel (I):

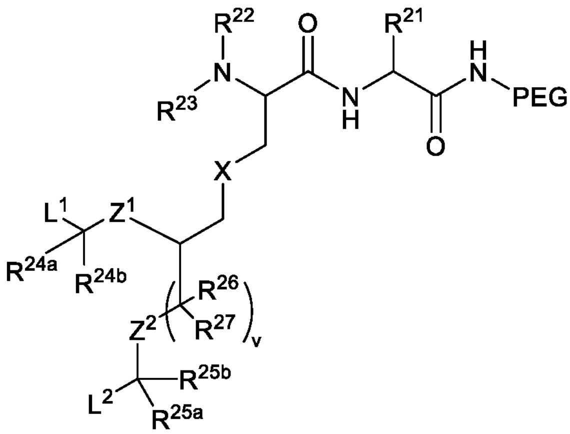

(I)

wobei:

$R^{21}$ ausgewählt ist aus der Gruppe bestehend aus H, $-CH_2OH$, $-CH_2CH_2OH$, $-CH(CH_3)OH$, $-CH_2OPO(OH)_2$, $-CH_2C(=O)NH_2$, $-CH_2CH_2C(=O)OH$ und $-CH_2CH_2C(=O)OR_8$, wobei ein beliebiger der Alkylwasserstoffe durch ein Halogen ersetzt sein kann;
$R^{22}$ H, $C_1$-$C_6$ aliphatisch, eine Aminoschutzgruppe, $L^3$-C(=O)- oder $A^2$ ist;
$L^1$ und $L^2$ jeweils unabhängig voneinander $C_6$-$C_{21}$ aliphatisch oder $C_5$-$C_{20}$ heteroaliphatisch sind;
$L^3$ $C_1$-$C_{21}$ aliphatisch oder $C_2$-$C_{20}$ heteroaliphatisch ist;
$A^2$ eine Aminosäure oder ein Peptid ist;
$R^{23}$ H oder $C_1$-$C_6$ aliphatisch ist;
$R^{24a}$ und $R^{25a}$ jeweils unabhängig voneinander ausgewählt sind aus $C_1$-$C_6$ aliphatisch und $C_1$-$C_6$ heteroaliphatisch und
$R^{24b}$ und $R^{25b}$ jeweils unabhängig voneinander ausgewählt sind aus H, $C_1$-$C_6$ aliphatisch und $C_1$-$C_6$ heteroaliphatisch, oder
$R^{24a}$ und $R^{24b}$ zusammen mit dem Kohlenstoffatom, an das sie gebunden sind, eine $C_{3-8}$-Cycloalkyl- oder 3-8-gliedrige Heterocyclylgruppe bilden, und/oder
$R^{25a}$ und $R^{25b}$ zusammen mit dem Kohlenstoffatom, an das sie gebunden sind, eine $C_{3-8}$-Cycloalkyl- oder 3-8-gliedrige Heterocyclylgruppe bilden;
X ausgewählt ist aus -S-, -S(=O)- und $-S(=O)_2$-;
v eine ganze Zahl von 1 - 3 ist
$R^{26}$ und $R^{27}$ jeweils unabhängig voneinander ausgewählt sind aus H und $C_1$-$C_6$ aliphatisch;
$Z^1$ und $Z^2$ unabhängig voneinander ausgewählt sind aus der Gruppe bestehend aus -C(=O)O-, -OC(=O)-, -C(=O)NR-, -NRC(=O)-, -C(=O)S-, -SC(=O)-, -OC(=O)O-, -NRC(=O)O-, -OC(=O)NR- und -NRC(=O)NR-;
PEG ein Polyethylenglykol ist;
wobei jedes beliebige aliphatische, heteroaliphatische, Cycloalkyl und Heterocyclyl, das in einem beliebigen von $R^{21}$, $R^{22}$, $R^{23}$, $R^{24a}$, $R^{24b}$, $R^{25a}$, $R^{25b}$, $R^{26}$, $R^{27}$, $L^1$, $L^2$ und $L^3$ vorhanden ist, gegebenenfalls substituiert ist
oder ein pharmazeutisch annehmbares Salz, Solvat oder Prodrug davon.

2. Verbindung nach Anspruch 1 oder ein pharmazeutisch annehmbares Salz, Solvat oder Prodrug davon, wobei das PEG ein substituiertes Polyethylenglykol von Formel B-I ist:

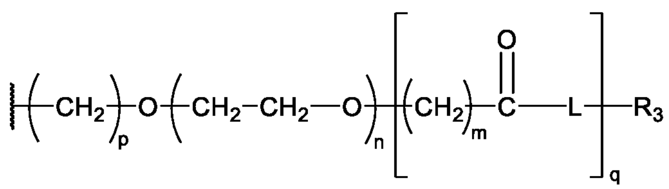

(B-I)

wobei

n 3 bis 100 ist;
m 1, 2, 3 oder 4 ist;
p 2, 3 oder 4 ist;
q Null oder 1 ist;
$R_3$ H, $-NH_2$ oder -OH ist, wobei, wenn q Null ist, $R_3$ H ist und wenn q 1 ist, $R_3$ $-NH_2$ oder -OH ist;
L Null ist oder aus 1 bis 10 Einheiten besteht, wobei jede Einheit eine natürliche alpha-Aminosäure ist oder von einer natürlichen alpha-Aminosäure abgeleitet ist und die Formel hat:

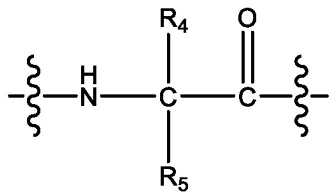

wobei $R_4$ H ist; und
$R_5$ die Seitenkette oder zweiter Wasserstoff der Aminosäure ist.

**3.** Verbindung nach Anspruch 1 oder 2 oder ein pharmazeutisch annehmbares Salz, Solvat oder Prodrug davon, wobei $R^{24b}$ und $R^{25b}$ H sind.

**4.** Verbindung nach einem beliebigen der Ansprüche 1 - 3 oder ein pharmazeutisch annehmbares Salz, Solvat oder Prodrug davon, wobei $R^{24a}$ und $R^{25a}$ unabhängig voneinander ein aliphatischer $C_1$-$C_6$-Rest sind, vorzugsweise wobei $R^{24a}$ und $R^{25a}$ unabhängig voneinander ein $C_1$-$C_6$-Alkyl sind, mehr bevorzugt wobei $R^{24a}$ und $R^{25a}$ jeweils Methyl sind.

**5.** Verbindung nach einem beliebigen der Ansprüche 1 - 4 oder ein pharmazeutisch annehmbares Salz, Solvat oder Prodrug davon, wobei v 1 oder 2 ist.

**6.** Verbindung nach einem beliebigen der Ansprüche 1 - 5 oder ein pharmazeutisch annehmbares Salz, Solvat oder Prodrug davon, wobei X -S- ist.

**7.** Verbindung nach einem beliebigen der Ansprüche 1 - 6 oder ein pharmazeutisch annehmbares Salz, Solvat oder Prodrug davon, wobei $R^{22}$ H ist.

**8.** Verbindung nach einem beliebigen der Ansprüche 1 - 7 oder ein pharmazeutisch annehmbares Salz, Solvat oder Prodrug davon, wobei $R^{23}$ H ist.

**9.** Verbindung nach einem beliebigen der Ansprüche 1 - 8 oder ein pharmazeutisch annehmbares Salz, Solvat oder Prodrug davon, wobei $R^{26}$ H ist.

**10.** Verbindung nach einem beliebigen der Ansprüche 1 - 9 oder ein pharmazeutisch annehmbares Salz, Solvat oder Prodrug davon, wobei $R^{27}$ H ist.

**11.** Verbindung nach einem beliebigen der Ansprüche 1 - 10 oder ein pharmazeutisch annehmbares Salz, Solvat oder Prodrug davon, wobei $R^{21}$ -CHzOH ist.

**12.** Verbindung nach einem beliebigen der Ansprüche 1 - 11 oder ein pharmazeutisch annehmbares Salz, Solvat oder Prodrug davon, wobei $Z^1$ und $Z^2$ jeweils -C(=O)-O- sind.

**13.** Verbindung nach einem beliebigen der vorhergehenden Ansprüche, ausgewählt aus einer der folgenden Verbindungen:

| Verbindungsstruktur | Verbindungs-ID |
|---|---|
| 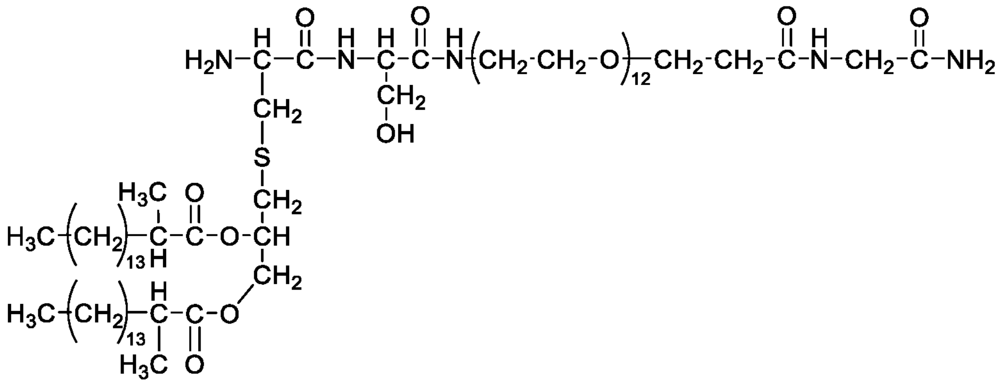 | B1 |

(continued)

| Verbindungsstruktur | Verbindungs-ID |
|---|---|
| 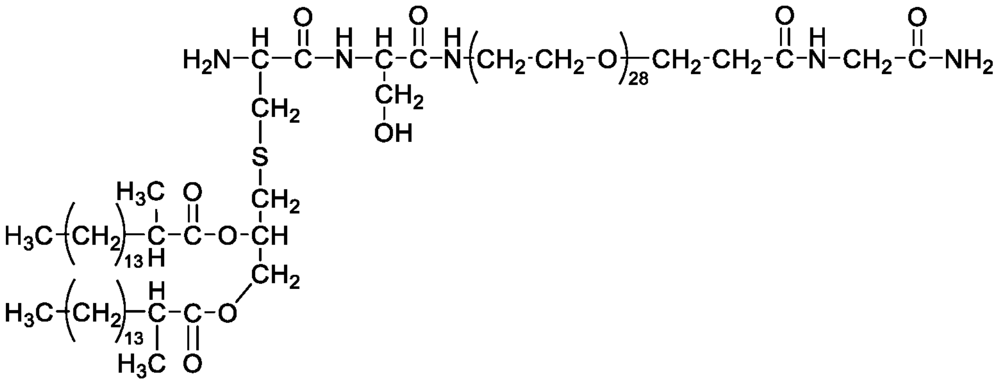 | B2 |
| 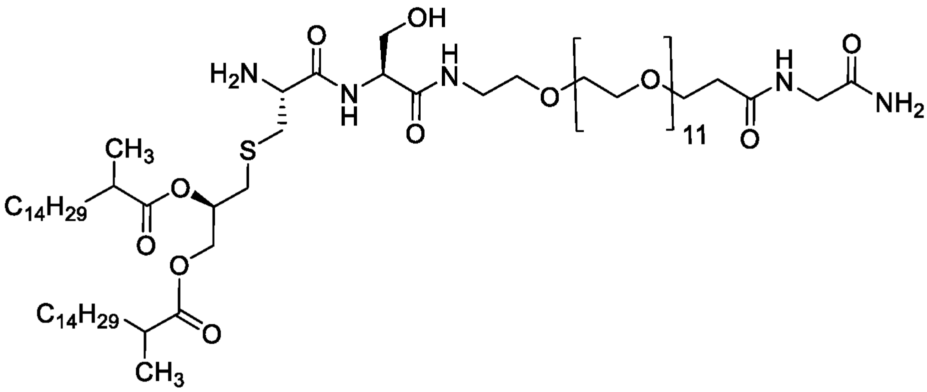 | B3 |
| 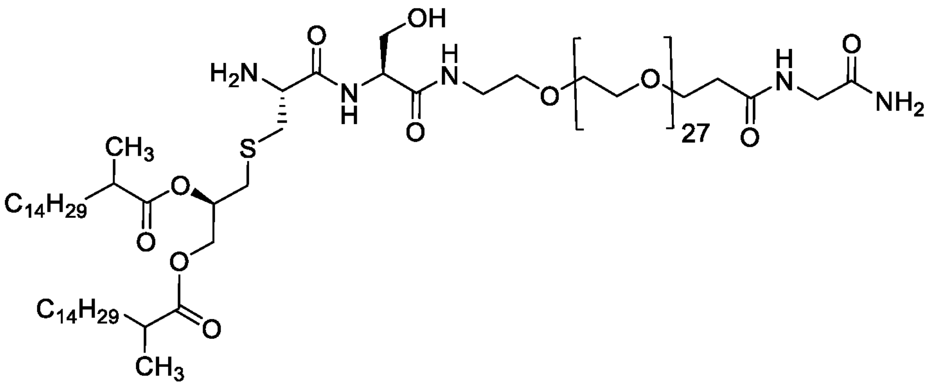 | B4 |
| 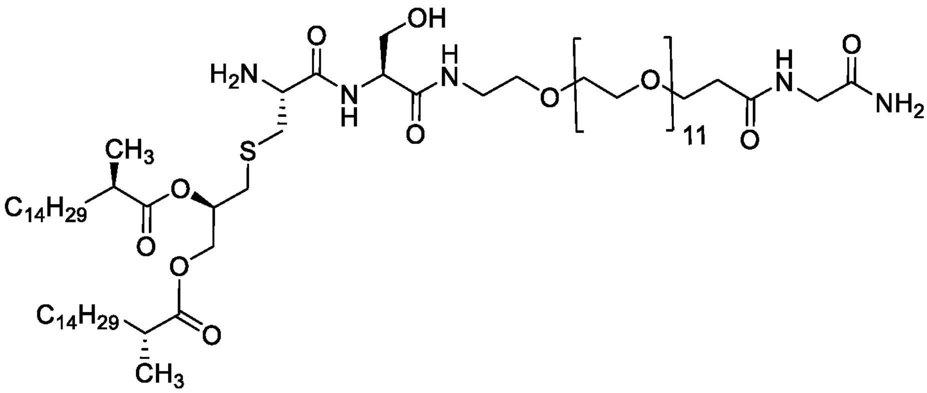 | B5 |

(continued)

| **Verbindungsstruktur** | **Verbin- dungs-ID** |
|---|---|
| 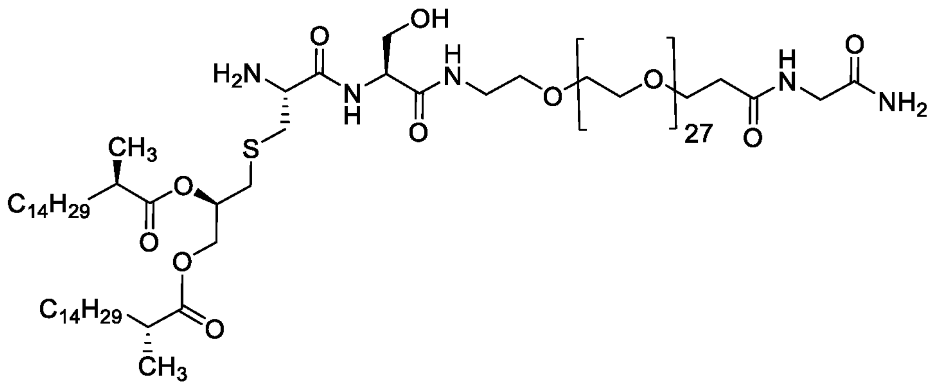 | B6 |
| 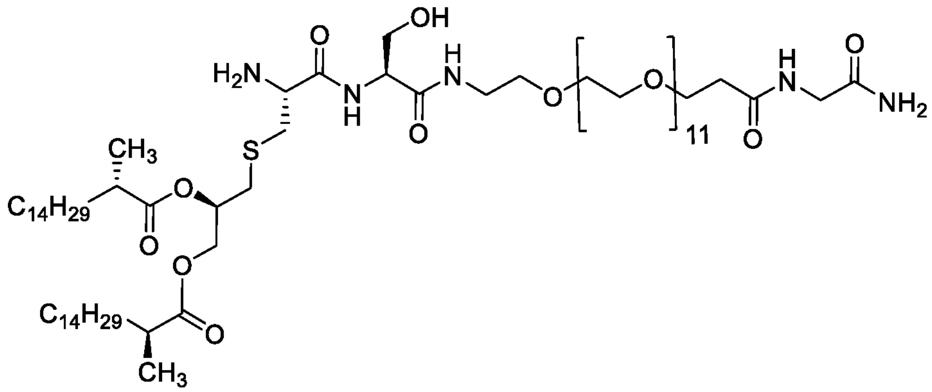 | B7 |
| 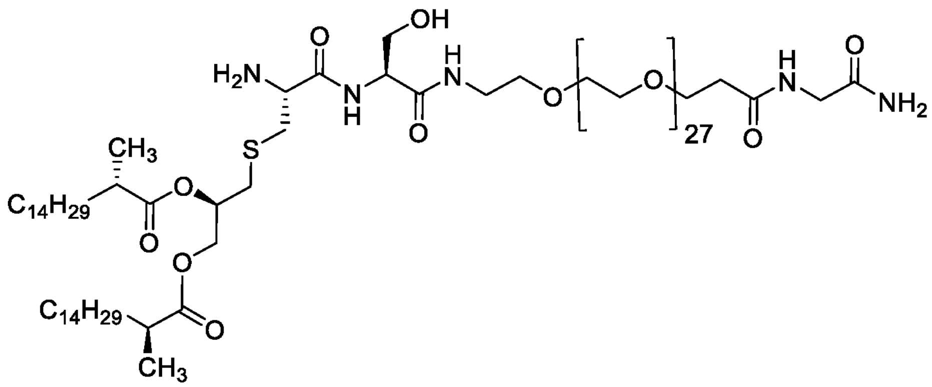 | B8 |
| 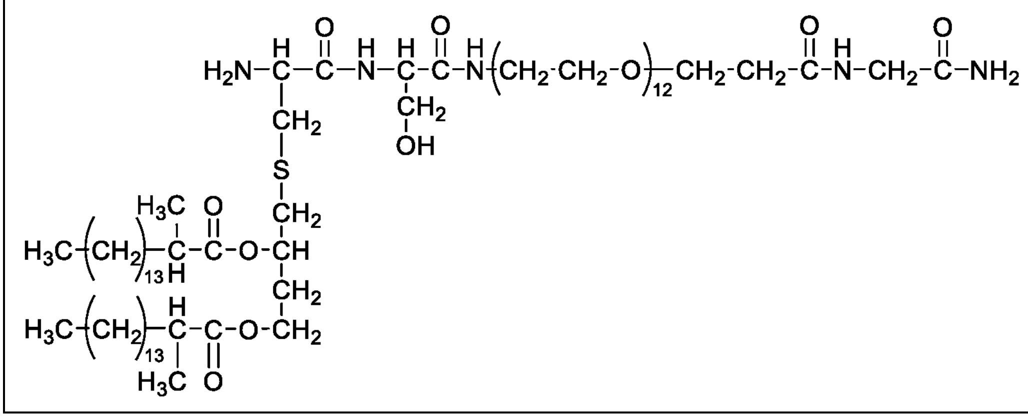 | B9 |

(continued)

| Verbindungsstruktur | Verbin-dungs-ID |
|---|---|
| 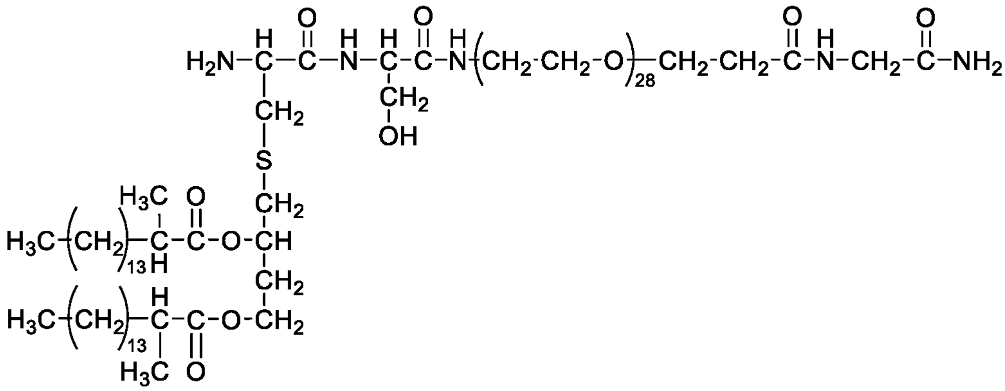 | B10 |
| 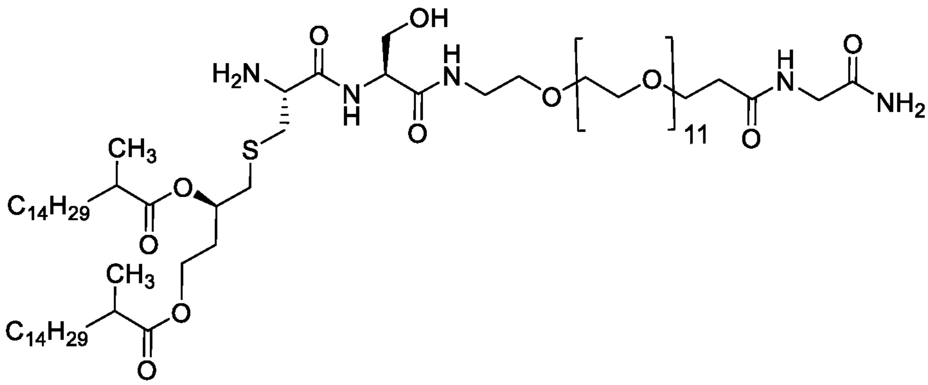 | B11 |
| 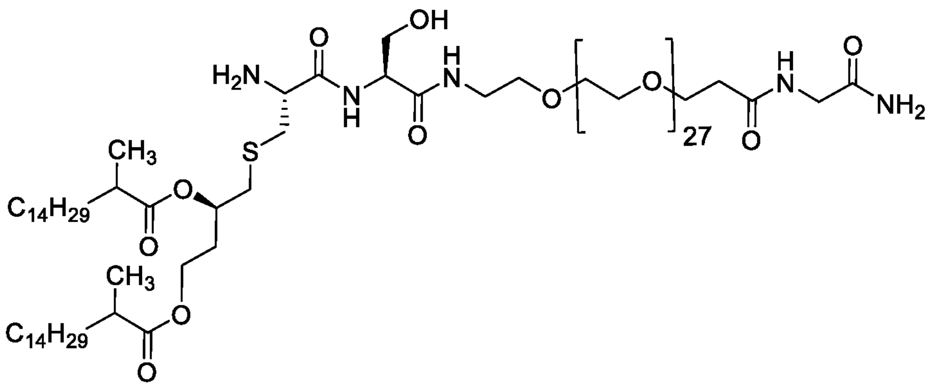 | B12 |
| 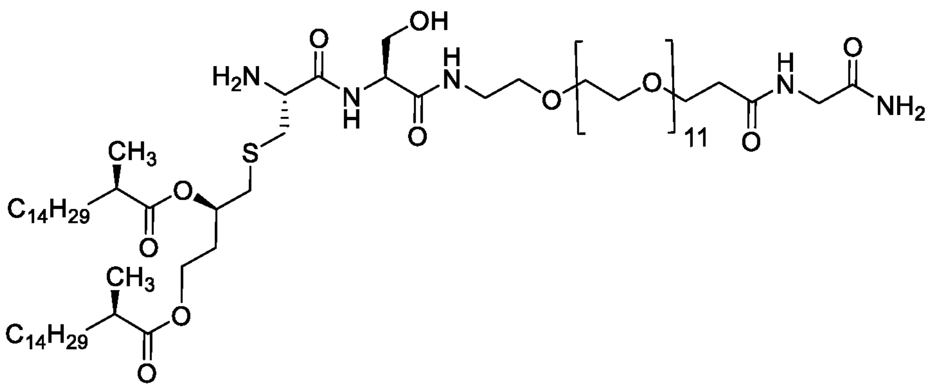 | B13 |

(continued)

| Verbindungsstruktur | Verbin-dungs-ID |
|---|---|
| 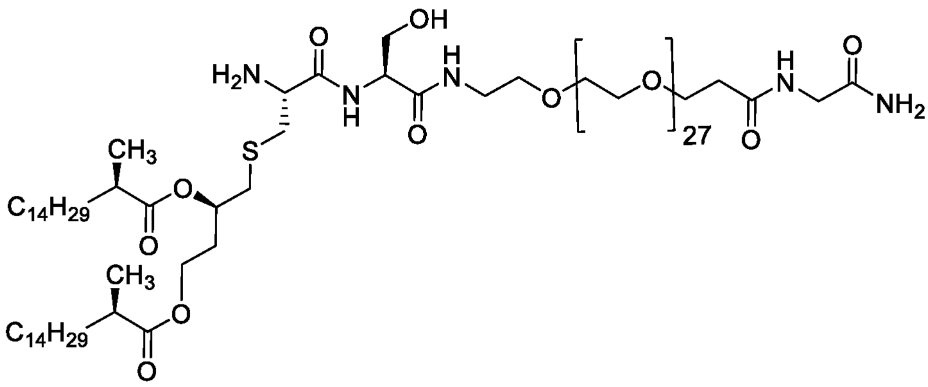 | B14 |
| 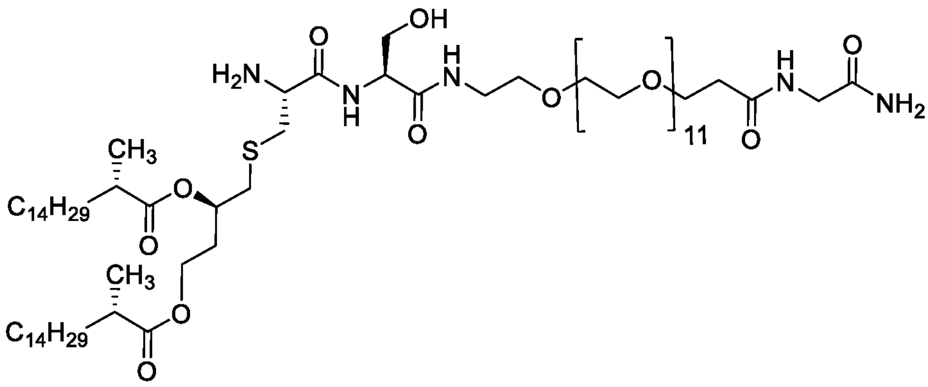 | B15 |
| 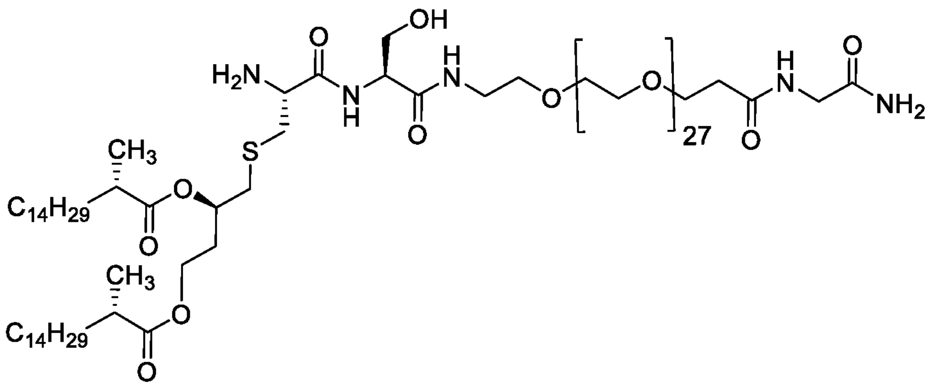 | B16 |

oder ein pharmazeutisch annehmbares Salz, Solvat oder Prodrug davon.

**14.** Zusammensetzung, umfassend eine Verbindung nach einem beliebigen der vorhergehenden Ansprüche oder ein pharmazeutisch annehmbares Salz, Solvat oder Prodrug davon, und gegebenenfalls einen pharmazeutisch annehmbaren Träger, Verdünnungsmittel oder Hilfsstoff.

**15.** Verbindung nach einem beliebigen der Ansprüche 1 - 13 oder ein pharmazeutisch annehmbares Salz, Solvat oder Prodrug davon zur Verwendung bei einem beliebigen von:

- Auslösen einer angeborenen Immunreaktion; und/oder
- Behandeln und/oder Vorbeugen einer durch ein infektiöses Agens verursachten Krankheit; und/oder
- Behandeln und/oder Vorbeugen einer Atemwegserkrankung oder eines Zustands, der mit einer viralen oder bakteriellen Infektion assoziiert ist; und/oder
- Behandeln und/oder Vorbeugen einer Atemwegsinfektion; und/oder

• Verringern von Entzündung der Atemwege; und/oder
• Verbessern der Fähigkeit eines Subjekts, eine Erkrankung oder einen Zustand der Atemwege während einer Virusinfektion der Atemwege zu kontrollieren; und/oder
• Behandeln und/oder Vorbeugen einer Krankheit oder eines Zustands, der mit dem TLR2-Rezeptor assoziiert ist.

## Revendications

**1.** Composé de formule (I) :

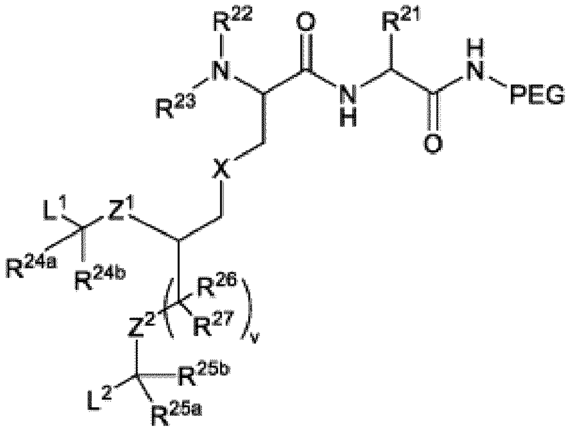

(I)

dans laquelle :

$R^{21}$ est choisi dans le groupe constitué par H, $-CH_2OH$, $-CH_2CH_2OH$, - $CH(CH_3)QH$, $-CH_2OPO(OH)_2$, $-CH_2C(=O)NH_2$, $-CH_2CH_2C(=O)OH$ et - $CH_2CH_2C(=O)OR_8$, dans lequel n'importe lequel des hydrogènes d'alkyle peut être remplacé par un halogène ;
$R^{22}$ est H, un radical aliphatique en $C_1$-$C_6$, un groupe amino protecteur, $L^3$-C(=O)- ou $A^2$ ;
$L^1$ et $L^2$ sont chacun indépendamment un radical aliphatique en $C_6$-$C_{21}$ ou hétéroaliphatique en $C_5$-$C_{20}$ ;
$L^3$ est un radical aliphatique en $C_1$-$C_{21}$ ou hétéroaliphatique en $C_2$-$C_{20}$ ;
$A^2$ est un acide aminé ou un peptide ;
$R^{23}$ est H ou un radical aliphatique en $C_1$-$C_6$ ;
$R^{24a}$ et $R^{25a}$ sont chacun indépendamment choisis parmi les radicaux aliphatiques en $C_1$-$C_6$ et hétéroaliphatiques en $C_1$-$C_6$, et
$R^{24b}$ et $R^{25b}$ sont chacun indépendamment choisis parmi H, les radicaux aliphatiques en $C_1$-$C_6$ et hétéroaliphatiques en $C_1$-$C_6$, ou
$R^{24a}$ et $R^{24b}$ forment conjointement avec l'atome de carbone auquel ils sont liés, un groupe cycloalkyle en $C_{3-8}$ ou un groupe hétérocyclyle de 3 à 8 chaînons, et/ou
$R^{25a}$ et $R^{25b}$ forment conjointement avec l'atome de carbone auquel ils sont liés, un groupe cycloalkyle en $C_{3-8}$ ou un groupe hétérocyclyle de 3 à 8 chaînons ;
X est choisi parmi -S-, -S(=O)- et $-S(=O)_2-$ ;
v est un nombre entier de 1 à 3.
$R^{26}$ et $R^{27}$ sont chacun indépendamment choisis parmi H et un radical aliphatique en $C_1$-$C_6$ ;
$Z^1$ et $Z^2$ sont indépendamment choisis dans le groupe constitué par - C(=O)O-, -OC(=O)-, -C(=O)NR-, -NRC(=O)-, -C(=O)S-, -SC(=O)-, -OC(=O)O-, - NRC(=O)O-, -OC(=O)NR- et -NRC(=O)NR- ;
PEG est un polyéthylène glycol ;
dans laquelle tout radical aliphatique, hétéroaliphatique, cycloalkyle et hétérocyclyle présent dans l'un quelconque de $R^{21}$, $R^{22}$, $R^{23}$, $R^{24a}$, $R^{24b}$, $R^{25a}$, $R^{25b}$, $R^{26}$, $R^{27}$, $L^1$, $L^2$ et $L^3$ est éventuellement substitué
ou un sel, un solvate ou un promédicament pharmaceutiquement acceptable de celui-ci.

**2.** Composé selon la revendication 1, ou sel, solvate ou promédicament pharmaceutiquement acceptable de celui-ci, dans lequel le PEG est un polyéthylène glycol substitué de formule B-I : :

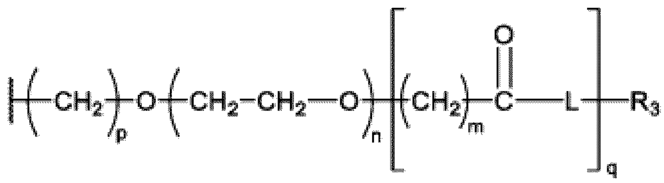

(B-I)

dans laquelle :

n est 3 à 100 ;
m est 1, 2, 3 ou 4 ;
p est 2, 3 ou 4 ;
q est nul ou 1 ;
$R_3$ est H, $-NH_2$ ou -OH, dans lequel lorsque q est nul, $R_3$ est H et lorsque q est 1, $R_3$ est $-NH_2$ ou -OH ;
L est nul ou est constitué par 1 à 10 unités, dans lequel chaque unité est un acide alpha-aminé naturel ou est dérivé d'un acide alpha-aminé naturel, et a pour formule :

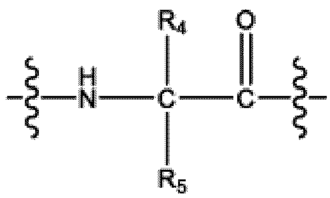

dans laquelle $R_4$ est H ; et
$R_5$ est la chaîne latérale, ou le second hydrogène de l'acide aminé.

**3.** Composé selon la revendication 1 ou 2, ou sel, solvate ou promédicament pharmaceutiquement acceptable de celui-ci, dans lequel $R^{24b}$ et $R^{25b}$ sont H.

**4.** Composé selon l'une quelconque des revendications 1 à 3, ou sel, solvate ou promédicament pharmaceutiquement acceptable de celui-ci, dans lequel $R^{24a}$ et $R^{25a}$ sont indépendamment un radical aliphatique en $C_1$-$C_6$, de préférence dans lequel $R^{24a}$ et $R^{25a}$ sont indépendamment un alkyle en $C_1$-$C_6$, plus préférablement dans lequel $R^{24a}$ et $R^{25a}$ sont chacun le méthyle.

**5.** Composé selon l'une quelconque des revendications 1 à 4, ou sel, solvate ou promédicament pharmaceutiquement acceptable de celui-ci, dans lequel v est 1 ou 2.

**6.** Composé selon l'une quelconque des revendications 1 à 5, ou sel, solvate ou promédicament pharmaceutiquement acceptable de celui-ci, dans lequel X est -S-.

**7.** Composé selon l'une quelconque des revendications 1 à 6, ou sel, solvate ou promédicament pharmaceutiquement acceptable de celui-ci, dans lequel $R^{22}$ est H.

**8.** Composé selon l'une quelconque des revendications 1 à 7, ou sel, solvate ou promédicament pharmaceutiquement acceptable de celui-ci, dans lequel $R^{23}$ est H.

**9.** Composé selon l'une quelconque des revendications 1 à 8, ou sel, solvate ou promédicament pharmaceutiquement acceptable de celui-ci, dans lequel $R^{26}$ est H.

**10.** Composé selon l'une quelconque des revendications 1 à 9, ou sel, solvate ou promédicament pharmaceutiquement acceptable de celui-ci, dans lequel $R^{27}$ est H.

**11.** Composé selon l'une quelconque des revendications 1 à 10, ou sel, solvate ou promédicament pharmaceutiquement acceptable de celui-ci, dans lequel $R^{21}$ est $-CH_2OH$.

12. Composé selon l'une quelconque des revendications 1 à 11, ou sel, solvate ou promédicament pharmaceutiquement acceptable de celui-ci, dans lequel $Z^1$ et $Z^2$ sont chacun -C(=O)-O-.

13. Composé selon l'une quelconque des revendications précédentes, choisi parmi l'un quelconque des composés suivants :

| Structure du composé | ID du composé |
|---|---|
| 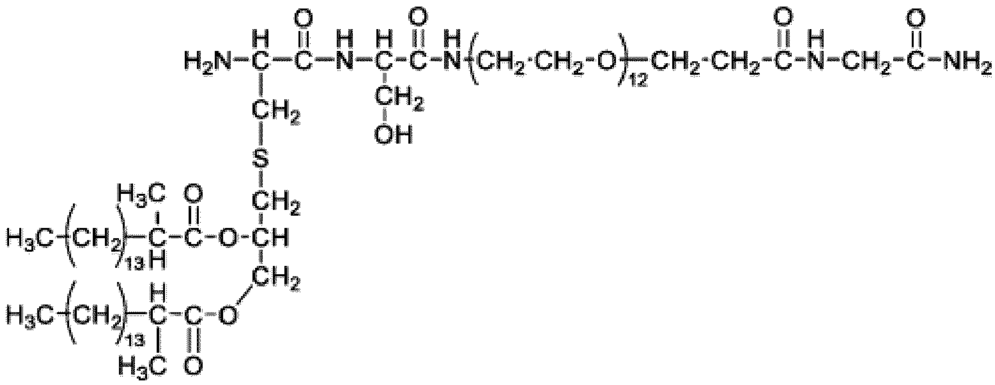 | B1 |
| 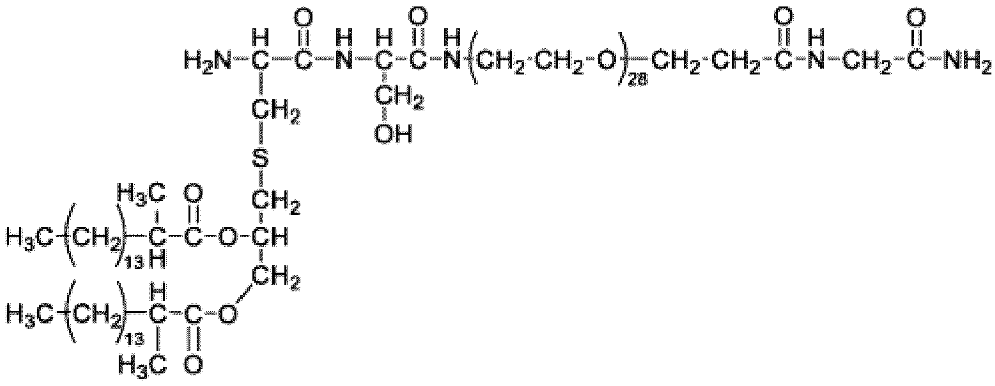 | B2 |
| 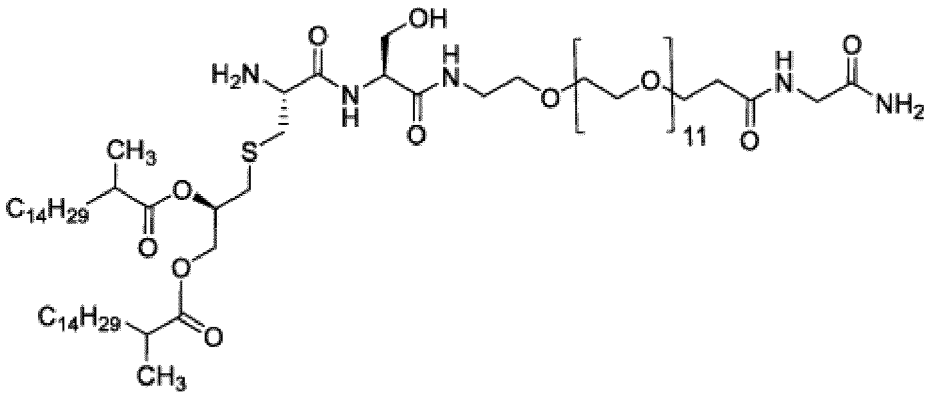 | B3 |

(continued)

| Structure du composé | ID du composé |
|---|---|
| 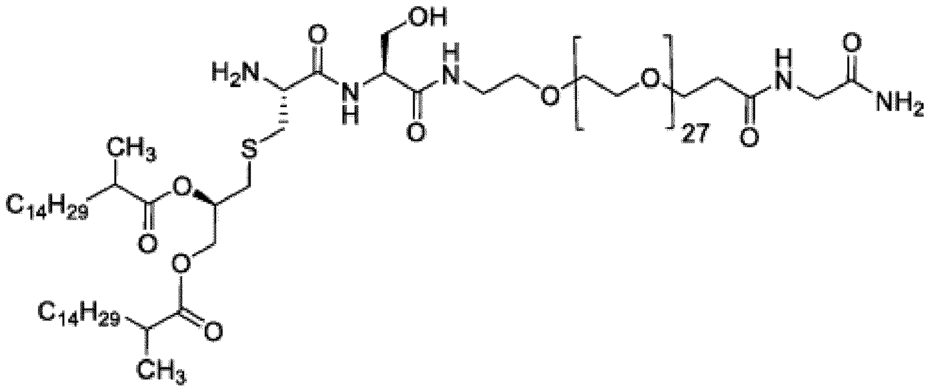 | B4 |
| 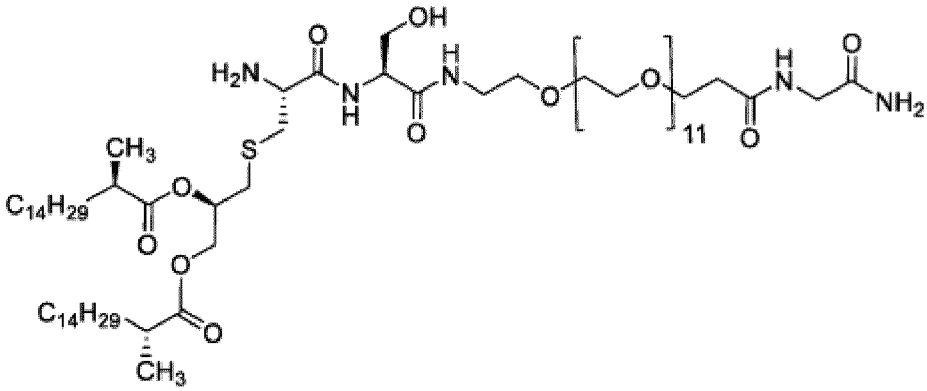 | B5 |
| 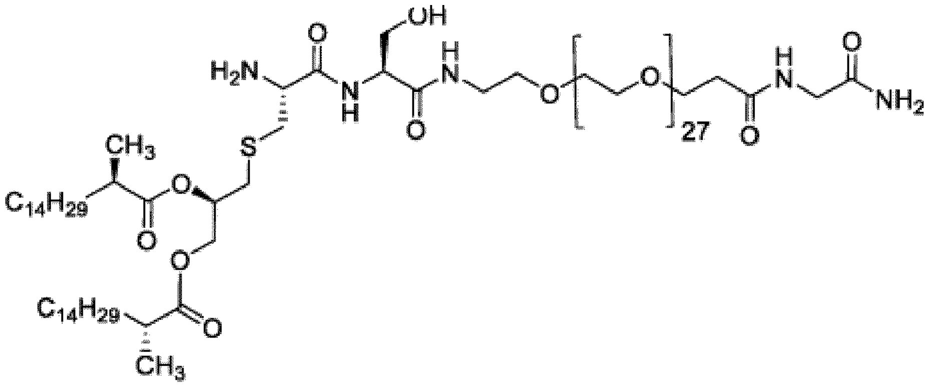 | B6 |
| 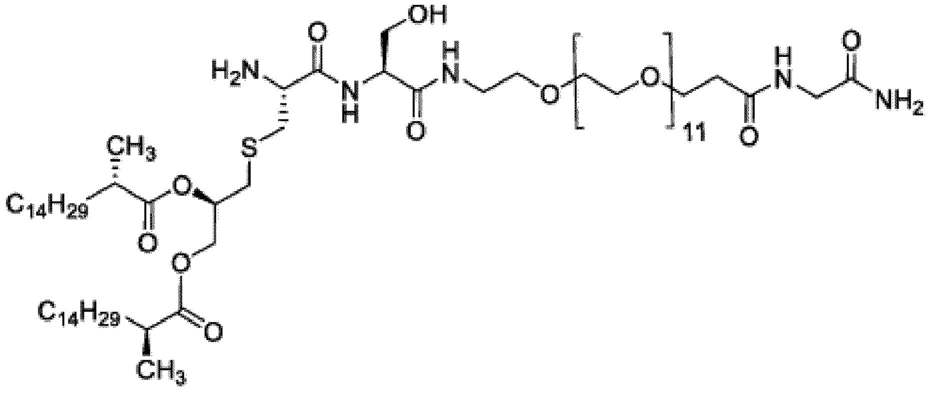 | B7 |

(continued)

| Structure du composé | ID du composé |
|---|---|
| 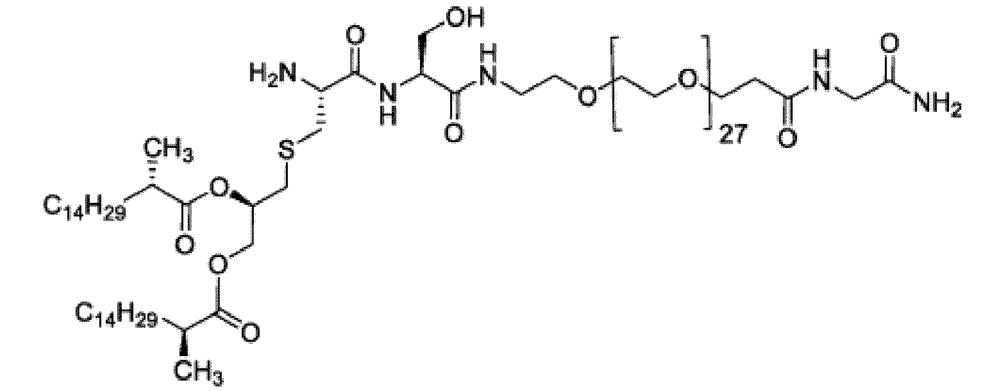 | B8 |
| 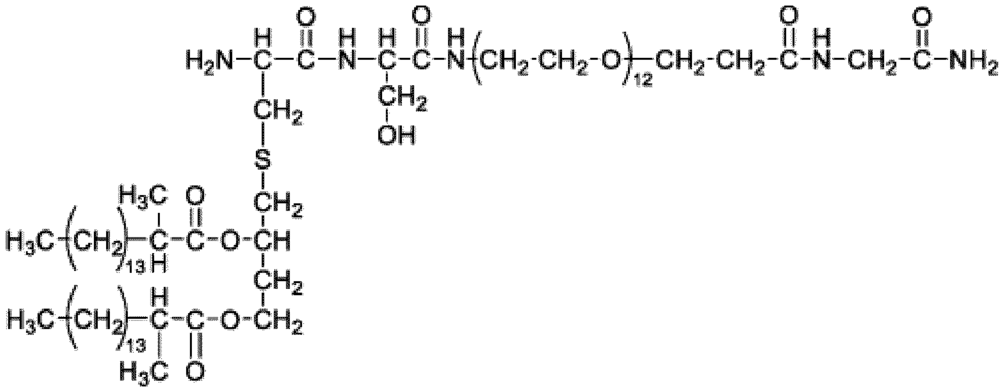 | B9 |
| 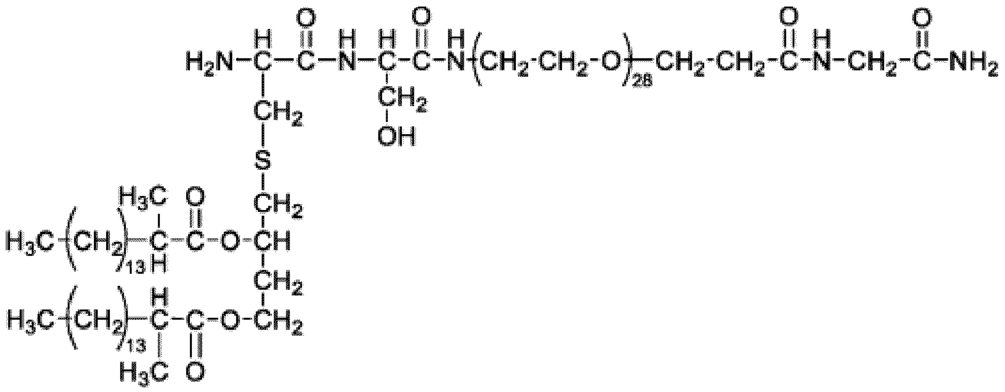 | B10 |
| 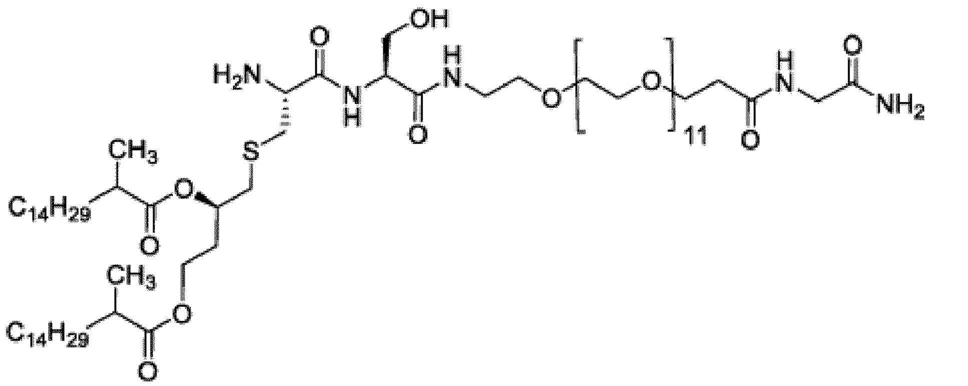 | B11 |

(continued)

| Structure du composé | ID du composé |
|---|---|
| 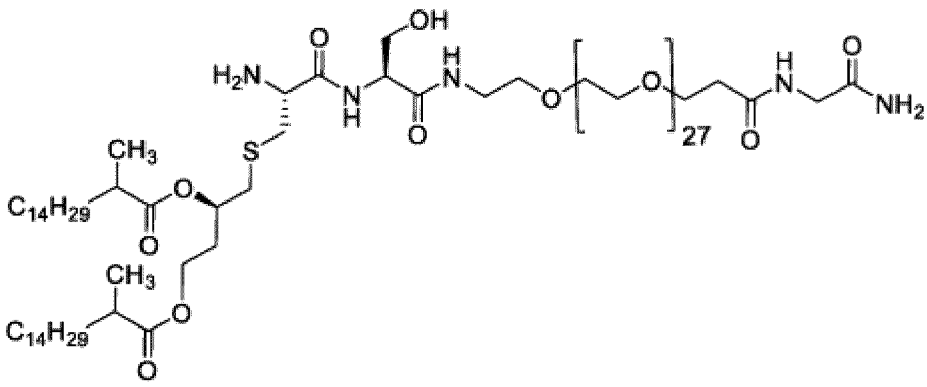 | B12 |
| 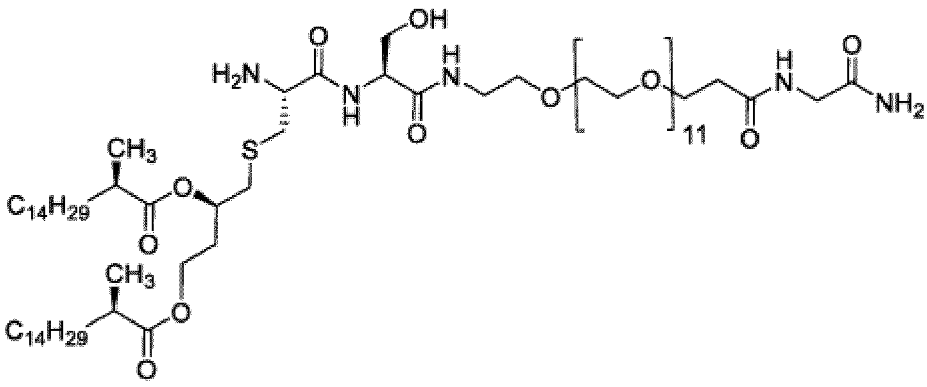 | B13 |
| 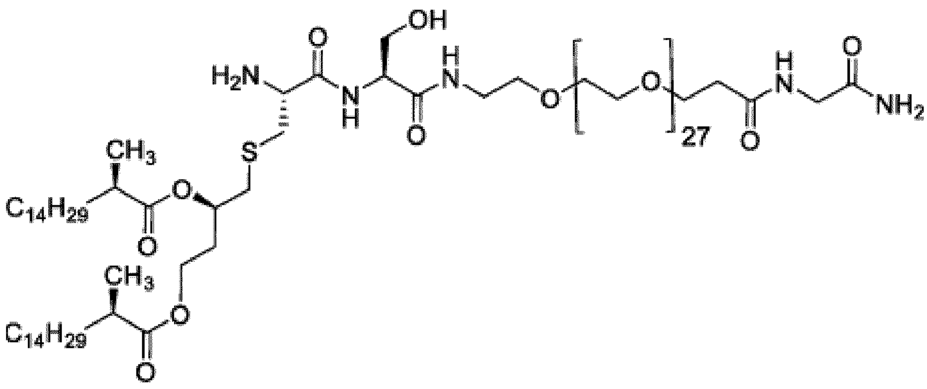 | B14 |
| 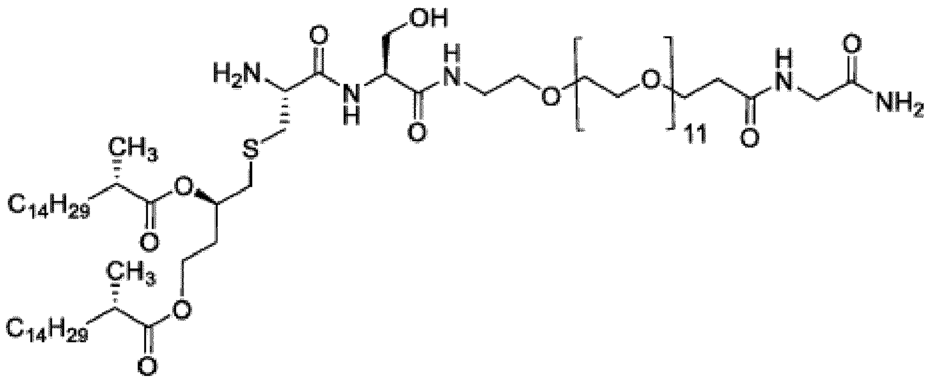 | B15 |

(continued)

| Structure du composé | ID du composé |
|---|---|
| 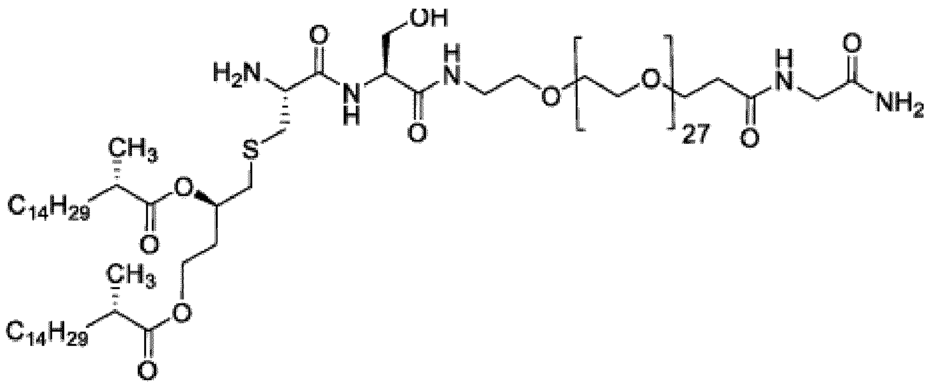 | B16 |

ou sel, solvate ou promédicament pharmaceutiquement acceptable de celui-ci.

**14.** Composition comprenant un composé selon l'une quelconque des revendications précédentes, ou un sel, un solvate ou un promédicament pharmaceutiquement acceptable de celui-ci, et éventuellement un support, un diluant ou un excipient pharmaceutiquement acceptable.

**15.** Composé selon l'une quelconque des revendications 1 à 13, ou sel, solvate ou promédicament pharmaceutiquement acceptable de celui-ci, pour une utilisation dans l'un quelconque de :

- l'augmentation d'une réponse immunitaire innée ; et/ou
- le traitement et/ou la prévention d'une maladie causée par un agent infectieux ; et/ou
- le traitement et/ou la prévention d'une maladie ou d'un état respiratoire associé à une infection virale ou bactérienne ; et/ou
- le traitement et/ou la prévention d'une infection respiratoire ; et/ou
- la réduction de l'inflammation des voies respiratoires ; et/ou
- l'amélioration de la capacité d'un sujet à lutter contre une maladie ou un état respiratoire lors d'une infection virale respiratoire ; et/ou
- le traitement et/ou la prévention d'une maladie ou d'un état associé au récepteur TLR2.

Figure 1

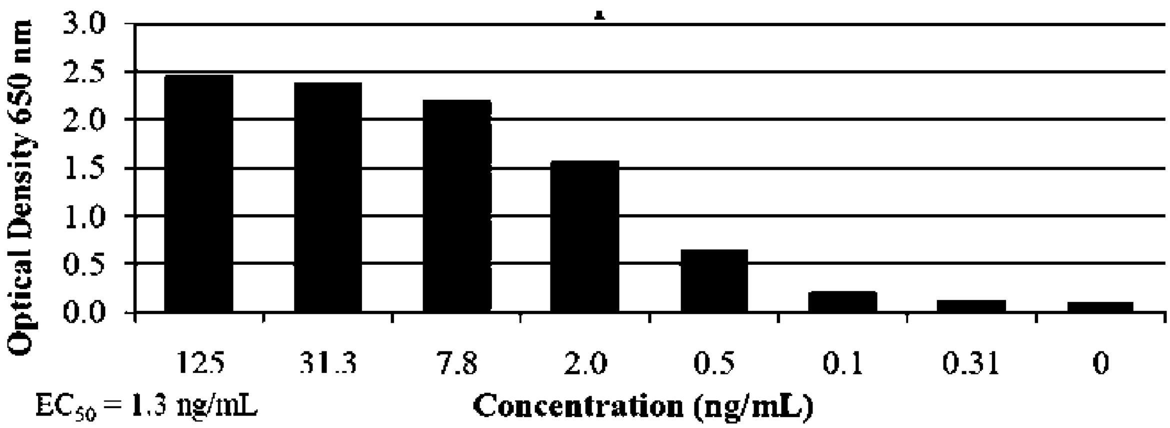


Figure 2

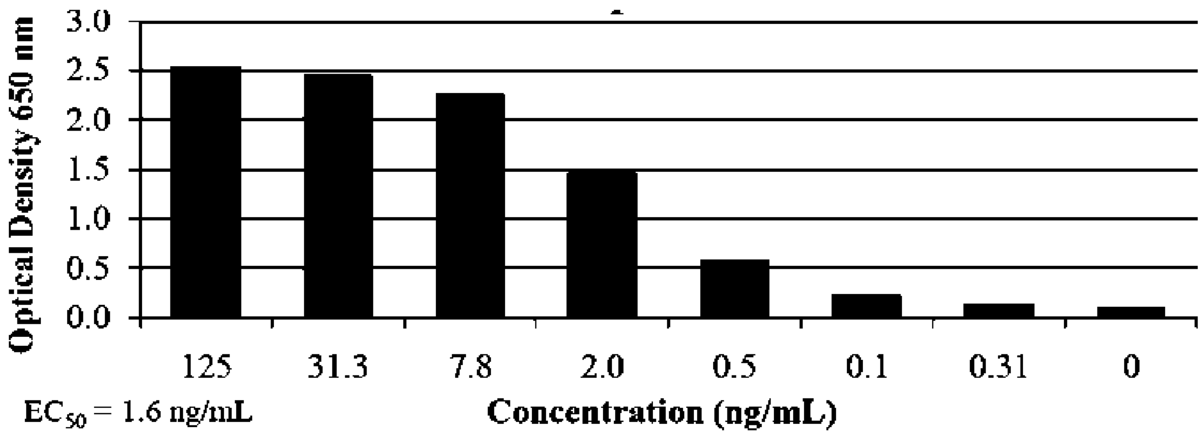

Figure 3

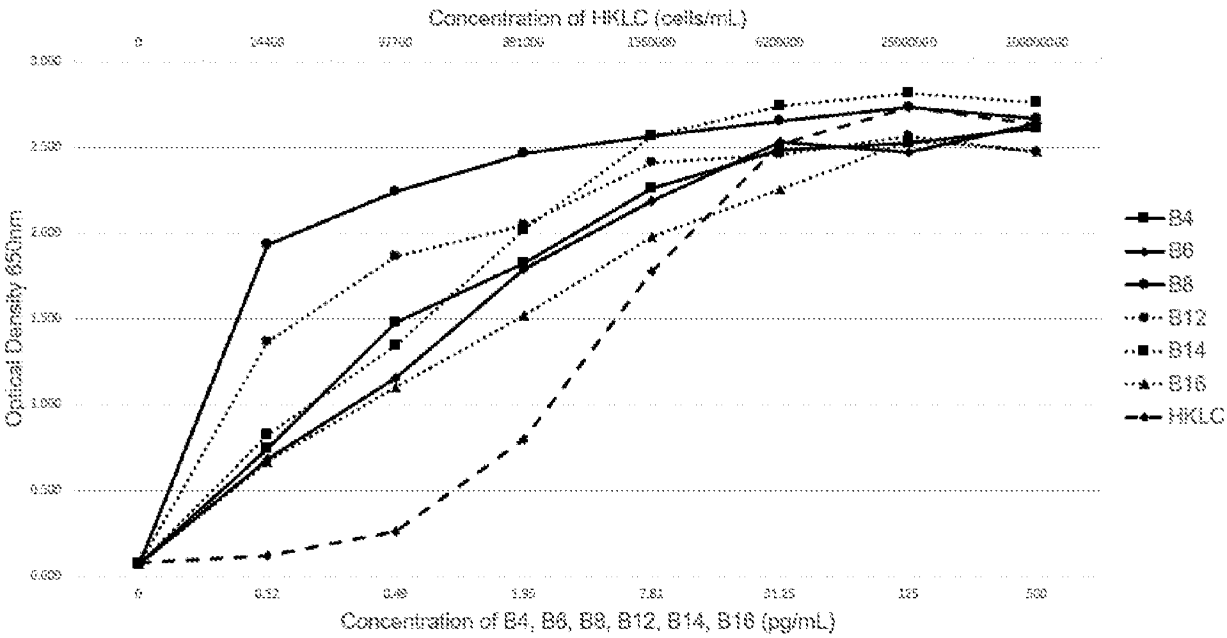


Figure 4

23 hr, HEK-Blue hTLR2 assay on Compounds 4 of WO2020/257870 and B12

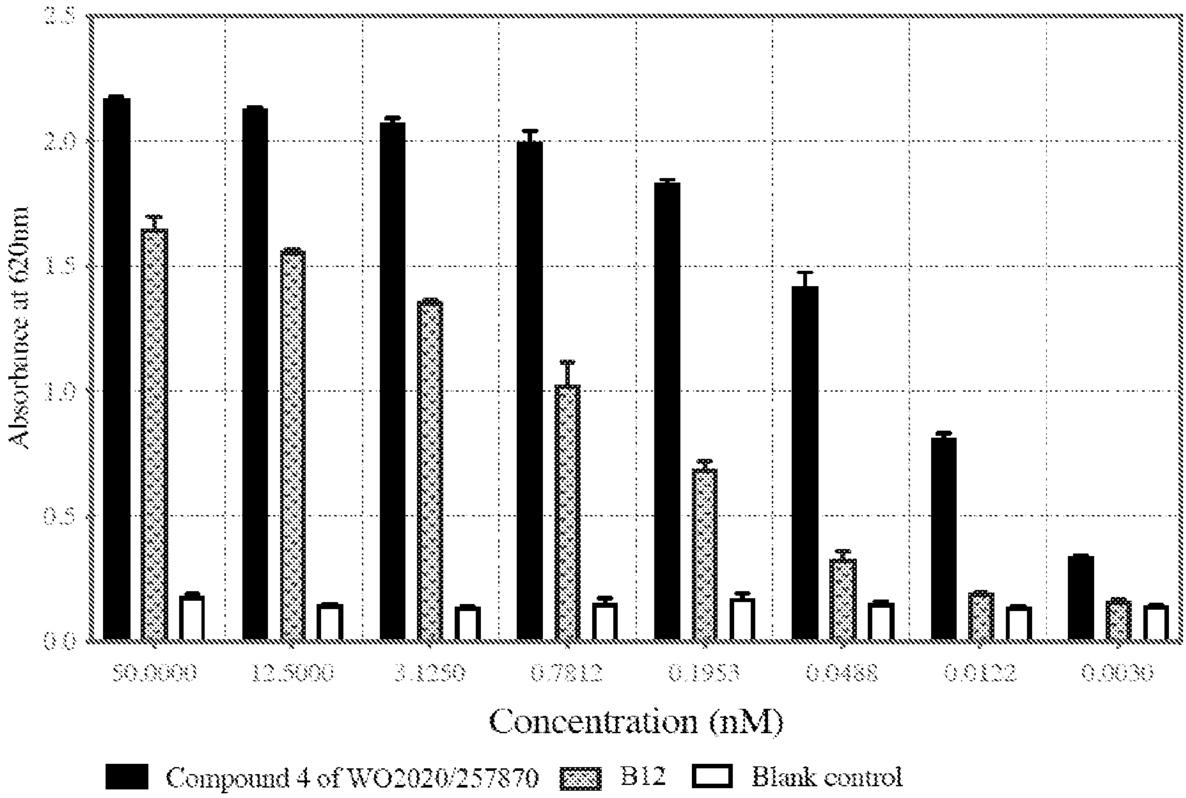

**Figure 5**

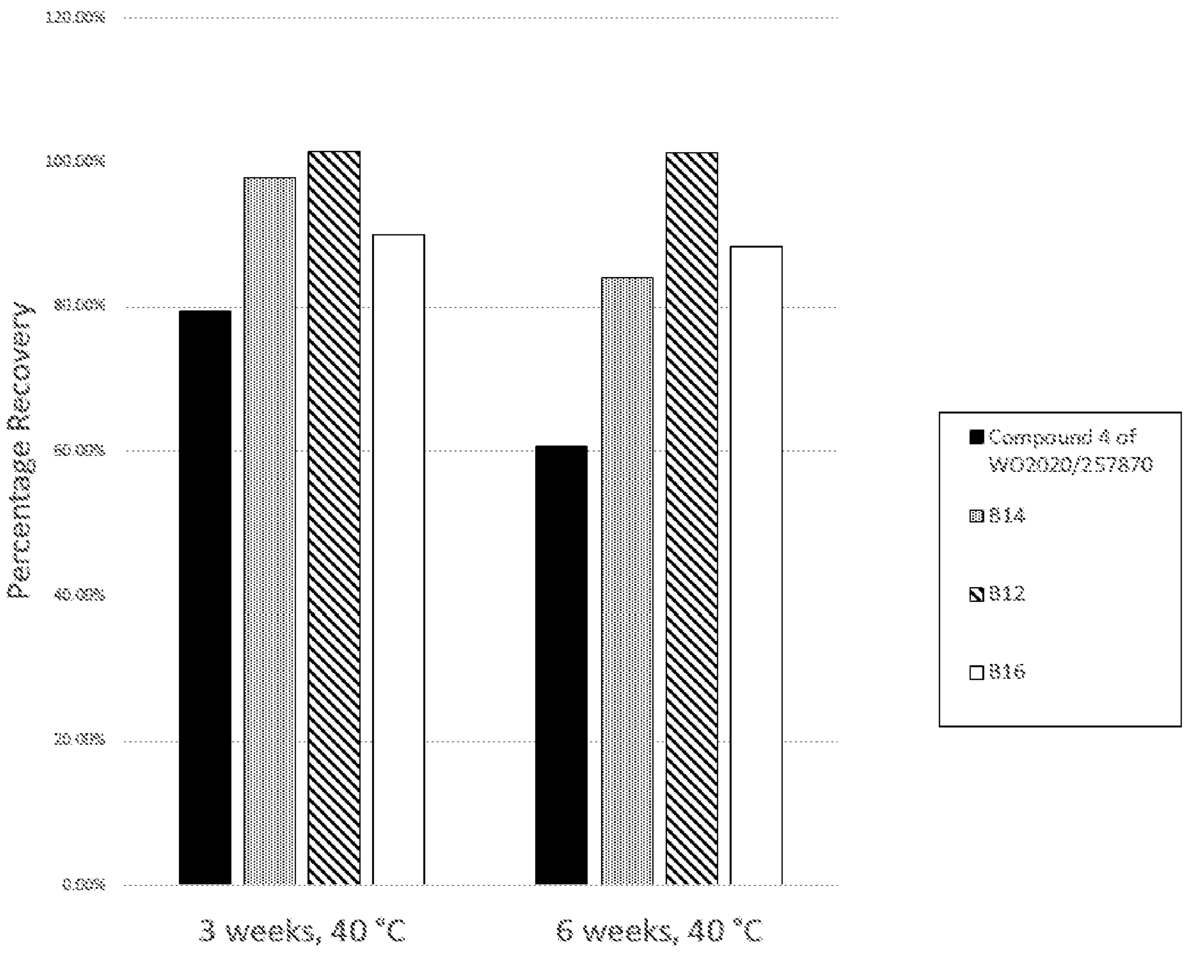
120.00%
100.00%
80.00%
60.00%
40.00%
20.00%
0.00%
Percentage Recovery
3 weeks, 40 °C
6 weeks, 40 °C
Compound 4 of WO2020/257870
B14
B12
B16

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020257870 A
- WO 2019119067 A


### Non-patent literature cited in the description


- Protective Groups in Organic Synthesis. John Wiley & Sons, 1999
- **ALBERT ISIDRO-LLOBET** ; **MERCEDES ALVAREZ**. Amino Acid-Protecting Groups' by Fernando Albericio. *Chemical Reviews*, 2009 (109), 2455-2504
- **FERNANDO ALBERICIO** ; **ALBERT ISIDRO-LLOBET** ; **MERCEDES ALVAREZ**. Amino Acid-Protecting Groups. *Chemical Reviews*, 2009 (109), 2455-2504
- **P.H.STAHL** ; **C.G.WERMUTH**. Handbook of Pharmaceutical salts. Wiley-VCH, 2002
- **MARTINDALE**. The Extra Pharmacopoeia. Pharmaceutical Press, 1993
- Remington's Pharmaceutical Sciences